# EUROPEAN PATENT SPECIFICATION

(11) **EP 2 091 560 B1**
(45) Date of publication and mention of the grant of the patent: **19.02.2014**
(21) Application number: 07844876.8
(22) Date of filing: 05.11.2007
(51) Int. Cl.: A61K 39/145, C12N 15/863

(54) **FELINE VACCINES AGAINST AVIAN INFLUENZA**
KATZENIMPFSTOFFE GEGEN VOGELGRIPPE
VACCINS CONTRE LA GRIPPE AVIAIRE POUR DES FELINS

(30) Priority: 06.11.2006 US 557040; 22.06.2007 US 767276
(43) Date of publication of application: 26.08.2009
(73) Proprietor: Merial Limited, Duluth, GA 30096-4640 (US)
(72) Inventor: KARACA, Kemal, Overland Park, KS 66221-2204 (US); MINKE, Jules, Maarten, 69906 Corbas (FR)
(74) Representative: Harding, Charles Thomas
(86) International application number: PCT/US2007/083648
(87) International publication number: WO 2008/058081

(56) References cited:
- WO-A1-92/15672
- US-A- 5 756 103
- US-A1- 2002 115 062
- US-A1- 2003 082 204
- US-A1- 2004 037 848
- US-A1- 2005 079 185
- BUBLOT MICHEL ET AL: "Development and use of fowlpox vectored vaccines for avian influenza", NEW YORK ACADEMY OF SCIENCES. ANNALS, WILEY-BLACKWELL PUBLISHING, INC, US, vol. 1081, 1 October 2006 (2006-10-01), pages 193-201, XP009089259, ISSN: 0077-8923, DOI: 10.1196/ANNALS.1373.023
- TAYLOR J ET AL: "Protective immunity against avian influenza induced by a fowlpox virus recombinant", VACCINE, ELSEVIER LTD, GB, vol. 6, no. 6, 1 December 1988 (1988-12-01), pages 504-508, XP023710103, ISSN: 0264-410X, DOI: 10.1016/0264-410X(88)90101-6 [retrieved on 1988-12-01]
- TRIPATHY D N ET AL: "EXPRESSION OF AVIAN INFLUENZA VIRUS HEMAGGLUTININ BY RECOMBINANT FOLPOX VIRUS", AVIAN DISEASES, AMERICAN ASSOCIATION OF AVIAN PATHOLOGISTS, KENNET SQ., PA, US, vol. 35, 1 January 1991 (1991-01-01), pages 186-191, XP002921341, ISSN: 0005-2086, DOI: 10.2307/1591312
- TAYLOR J ET AL: "Fowlpox virus as a vector in non-avian species", VACCINE, ELSEVIER LTD, GB, vol. 6, no. 6, 1 December 1988 (1988-12-01), pages 466-468, XP023710093, ISSN: 0264-410X, DOI: 10.1016/0264-410X(88)90091-6 [retrieved on 1988-12-01]
- PAOLETTI ENZO: "Applications of pox virus vectors to vaccination: An update", PROCEEDINGS OF THE NATIONAL ACADEMY OF SCIENCES, NATIONAL ACADEMY OF SCIENCES, vol. 93, no. 21, 1 October 1996 (1996-10-01), pages 11349-11353, XP002135943, ISSN: 0027-8424, DOI: 10.1073/PNAS.93.21.11349
- KARACA K.: 'Immunogenicity of Fowlpox Virus Expressing the Avian Influenza Virus H5 Gene (TROVAC AIV-H5) in Cats' CLINICAL AND DIAGNOSTIC LABORATORY IMMUNOLOGY vol. 12, no. 11, November 2005, pages 1340 - 1342, XP002434626
- MINKE J.M.: 'Use of DNA and Recombinant Canarypox Viral (ALVAC) Vectors for Equine Herpes Virus Vaccination' VETERINARY IMMUNOLOGY AND IMMUNOPATHOLOGY vol. 111, no. 1-2, 15 May 2006, pages 47 - 57, XP005387737
- FERRARI ET AL: 'Replication-defective canarypox (ALVAC) vectors effectively activate anti-human immunodeficiency virus-1 cytotoxic T lymphocytes present in infected patients: implications for antigen-specific immunotherapy.' BLOOD vol. 90, no. 6, 01 September 1997, pages 2406 - 2416, XP055024919 ISSN: 0006-4971
- TARTAGLIA J ET AL: "PROTECTION OF CATS AGAINST FELINE LEUKEMIA VIRUS BY VACCINATION WITH A CANARYPOX VIRUS RECOMBINANT, ALVAC-FL", JOURNAL OF VIROLOGY, THE AMERICAN SOCIETY FOR MICROBIOLOGY, US, vol. 67, no. 4, 1 April 1993 (1993-04-01), pages 2370-2375, XP002916084, ISSN: 0022-538X

## Description

### FIELD OF THE INVENTION

The present invention encompasses influenza vaccines, in particular avian influenza vaccines. The vaccine may be a recombinant poxvirus vaccine or an inactivated vaccine. The invention also encompasses recombinant poxvirus vectors encoding and expressing avian influenza antigens, epitopes or immunogens which can be used to protect animals, in particular cats, against avian influenza.

### BACKGROUND OF THE INVENTION

Upper respiratory tract diseases (UPTD) are common in felines, especially domestic cats. These diseases exhibit influenza-like symptoms, including fever, nasal discharge, sneezing, coughing, sinusitis and bronchitis, and can potentially be fatal. The most common agents for UPTD include chlamydia, feline herpesvirus-1, feline calicivirus, and Bordetella bronchiseptica. More recently, felines have displayed susceptibility of infection to influenza viruses, especially the avian influenza virus (AIV).

AIV is an RNA virus belonging to the family of Orthomyxoviridae, and is classified as a type A influenza virus, which relates to its nucleoprotein and membrane proteins. AIV has a lipid envelope that features two distinct glycoproteins: hemagglutinin (HA), which facilitates entry of the virus into the host cells, and neuraminidase (NA), which assists in the release of progeny virus from infected cells (de Jong et al., 2006). Thus far, 16 HA and 9 NA have been detected and can exist in varying combinations (Olsen et al., 2006), thereby forming subtypes of AIV that are based upon these antigenic differences. The H5N1 subtype (virus featuring HA 5 and NA 1) has specifically been associated with recent outbreaks in Asia, Russia, the Middle East, Europe and Africa, and is responsible for the growing concern of avian influenza infection of felids.

Reports regarding avian influenza infection of domestic cats and zoo felids emerged during the 2003 to 2004 avian influenza outbreak in Asia (Keawcharoen et al 2004). These reports described over 150 deaths of tigers and leopards as a result of feeding on raw chicken carcasses that were infected with the avian influenza. These felids developed lesions in the lungs, resulting in congestion and hemorrhaging, moderate meningoencephalitis, and mutifocal necrotizing hepatitis. Since then, scientists have confirmed that felines are susceptible to avian influenza infection via intratracheal injection, consumption of virus-infected chickens, and horizontal transmission through regular contact (Kuiken et al., 2004). The infected cats initially developed such symptoms as raised body temperature, conjunctivitis, and labored breathing, which eventually progressed to severe diffuse alveolar damage and death, although the virus can replicate in the respiratory tract without inducing any signs of the disease (Hinshaw et al. 1981). Further studies revealed that experimentally-infected cats displayed a presence of the virus in both respiratory and extra-respiratory organs, and excreted the virus through both the respiratory and digestive tracts (Rimmelzwaan et al., 2006). In addition, numerous new reports have described infection of domestic cats in Europe and Asia (Butler, 2006).

Considering the susceptibility of felines to AIV and their ability to excrete the virus into their surroundings, a method of preventing AIV infection and protecting felines is essential. The urgency is compounded given the possibility that feline infection may play a role in the epidemiology of AIV in poultry, humans, and other species (Influenza team, 2006; Kuiken et al., 2006). Accordingly, there is a need for an effective vaccine against influenza in felines.

Citation or identification of any document in this application is not an admission that such document is available as prior art to the present invention.

The present invention is defined in the appended claims.

### SUMMARY OF THE INVENTION

The present disclosure encompasses avian influenza immunological compositions, which may be a recombinant avian influenza immunological composition or an inactivated avian influenza immunological composition.

The present disclosure also encompasses avian influenza vaccines, which may be a recombinant avian influenza vaccine or an inactivated avian influenza vaccine.

Furthermore, the present disclosure encompasses influenza vaccines wherein the vaccine comprises one or more of an inactivated feline influenza isolate, an inactivated avian influenza isolate, or mixtures thereof.

In an embodiment of the disclosure wherein the avian influenza immunological composition or vaccine is a recombinant immunological composition or vaccine, advantageously, the composition or vaccine comprising a recombinant viral vector and a pharmaceutical or veterinary acceptable excipient, carrier or vehicle; the recombinant viral vector is an avipox expression vector which may comprise a polynucleotide encoding an influenza polypeptide, antigen, epitope or immunogen. The influenza polypeptide, antigen, epitope or immunogen may be a hemagglutinin, matrix protein, neuraminidase, nonstructural protein, nucleoprotein, polymerase or any fragment thereof.

In an embodiment of the disclosure, the avian influenza polypeptide, antigen, epitope or immunogen may be derived from a feline infected with influenza. For example, but not by limitation, influenza virus may be isolated from the broncho alveolar lavage and/or lung tissues of an affected felid. Isolation and characterization of the nucleotide sequence of the influenza infecting the felid may be done by routine experimentation by a person of ordinary skill in the art.

The avian influenza polypeptide, antigen, epitope or immunogen may be isolated from an avian influenza.

The avipox expression vector may be an attenuated avipox expression vector. In one embodiment of the disclosure, the avipox expression vector may be a fowlpox vector, advantageously TROVAC. In another embodiment of the disclosure, the avipox expression vector may be a canarypox vector, advantageously ALVAC. The influenza antigen, epitope, or immunogen may be a hemagglutinin, such as H5. The fowlpox vector may be vFP89 or vFP2211. The canarypox vector may be vCP2241

The present disclosure also encompasses an inactivated influenza immunological composition or vaccine. The inactivated influenza immunological composition or vaccine may be an inactivated influenza. In another embodiment of the disclosure, the inactivated influenza immunological composition or vaccine may be an avian influenza. The immunological composition or vaccine may be inactivated with formalin or beta-propiolactone.

The disclosure also relates to method of eliciting an immune response against influenza in a Felidae, in particular a cat, which may comprise administering a formulation comprising any one of the above recombinant influenza immunological composition or vaccine, or inactivated immunological composition or vaccine, and a pharmaceutically or veterinarily acceptable carrier, excipient or vehicle in an effective amount for eliciting an immune response. In an advantageous embodiment of the disclosure, an adjuvant may be added. The adjuvant may be aluminum hydroxide, aluminum phosphate, a carbomer or an oil-in-water-emulsion and optionally may comprise CpG. Advantageously, the administration may be subcutaneous intramuscular or transdermal with a needle-free injector.

The disclosure further relates to method of inducing a immune response against influenza in a Felidae, in particular in a cat, which may comprise administering a formulation comprising any one of the above recombinant influenza immunological compositions or vaccines, or inactivated immunological compositions or vaccines, and a pharmaceutically or veterinarily acceptable carrier, excipient or vehicle in an effective amount for inducing a immune response. In an advantageous embodiment, an adjuvant may be added. The adjuvant may be aluminum hydroxide, aluminum phosphate, a carbomer or an oil-in-water-emulsion and optionally may comprise CpG. Advantageously, the administration may be subcutaneous or intramuscular.

The invention further relates to the inducement or elicitation of an immune response against influenza in a Felidae wherein the immune response induced or elicited is a protective immune response.

The invention further encompasses a kit for performing a method of eliciting or inducing an immune response as defined in claim 11.

Accordingly, it is an object of the invention to not encompass within the invention any previously known product, process of making the product, or method of using the product such that Applicants reserve the right and hereby disclose a disclaimer of any previously known product, process, or method. It is further noted that the invention does not intend to encompass within the scope of the invention any product, process, or making of the product or method of using the product, which does not meet the written description and enablement requirements of the USPTO (35 U.S.C. §112, first paragraph) or the EPO (Article 83 of the EPC), such that Applicants reserve the right and hereby disclose a disclaimer of any previously described product, process of making the product, or method of using the product.

It is noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.

These and other embodiments are disclosed or are obvious from and encompassed by, the following Detailed Description.

### BRIEF DESCRIPTION OF THE DRAWINGS

The following detailed description, given by way of example, but not intended to limit the invention solely to the specific embodiments described, may best be understood in conjunction with the accompanying drawings, in which:

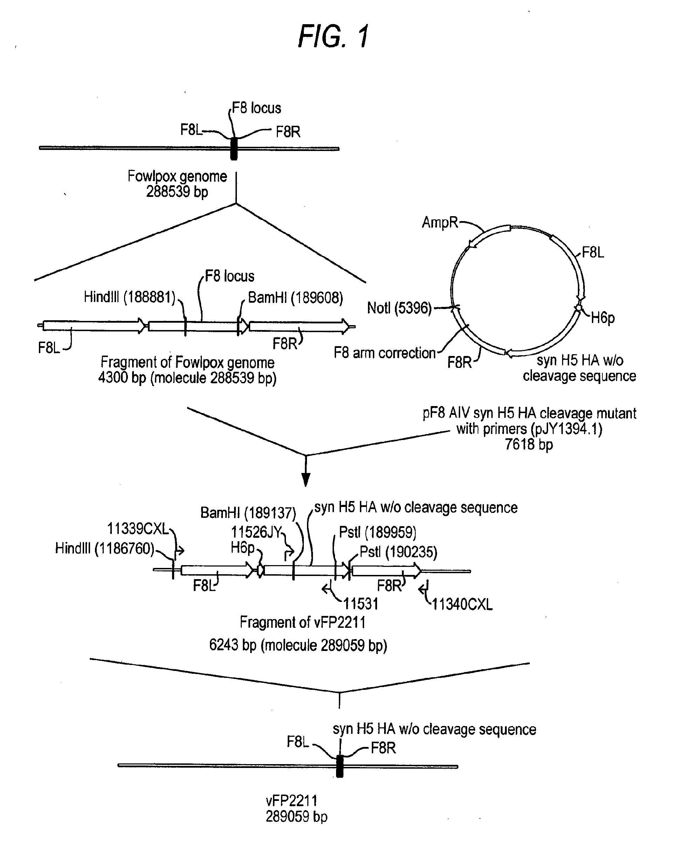

Figure 1 illustrates the generation of vFP2211, in which plasmid pJY1394.1, containing the synthetic AIV H5 HA insert, and a fragment of the Fowlpox genome at the F8 locus generates vFP2211 through in vitro recombination.
Figure 2 illustrates the fragment of vFP2211, indicating the positions of the primers for amplifying the AIV probe and for PCR amplification of the F8 arms and insert.
Figure 3 illustrates a theoretical restriction enzyme gel for the genomic DNA of vFP2211.
Figure 4 illustrates a gel from electrophoresis of genomic DNA extracted from vFP2211 and digested with BamHI, Hind III, and Pst I.
Figure 5 illustrates a gel from Western blot analysis indicating the correct insertion of synthetic AIV H5 HA into the C5 locus.
Figure 6 illustrates the results of an immunoplaque assay of vFP2211, indicating that the homogeneity of the population was 100%.
Figure 7 illustrates a comparison of nucleotide sequences between the wild type H5 HA without cleavage site (CK/Indonesia/2003) and the synthetic AIV H5 HA without cleavage site from plasmid pCR-Script/HA-CK/Indonesia/03-(modified)-avipox.
Figure 8 illustrates a comparison of amino acid sequences between the wild type H5 HA without cleavage site (CK/Indonesia/2003) and the synthetic AIV H5 HA without cleavage site from plasmid pCR-Script/HA-CK/Indonesia/03-(modified)-avipox.
Figure 9 illustrates the construction of the ALVAC plasmid pLH1852.5, in which the expression cassette of H6p-AIV synthetic H5 HA is isolated by digestion of the plasmid pJY 1394.1 and ligated to Eco DNA digested pALVAC C5 H6p donor (pCXL148.2).
Figure 10 illustrates the ALVAC plasmid pLH1852.5.
Figure 11 illustrates the generation of vCP2241, in which ALVAC plasmid pLH1852.5, containing the AIV synthetic H5 HA insert, and a fragment of the ALVAC genome at the C5 locus generates vCP2241 through in vitro recombination.
Figure 12 illustrates a theoretical restriction enzyme gel for the genomic DNA of vCP2241.4.1.1.1.
Figure 13 illustrates a gel from electrophoresis of genomic DNA extracted from vCP2241.4.1.1.1 and digested with BamHI, Hind III, and Pst I.
Figure 14 illustrates a gel from Southern blot analysis indicating the expression of the H5 HA protein.
Figure 15 illustrates a gel from Western blot analysis indicating the correct insertion of AIV synthetic H5 HA into the C5 locus.
Figure 16 illustrates the results of an immunoplaque assay of vCP2241.4.1.1.1, indicating that the homogeneity of the population was 100%.
Figure 17 illustrates the fragment of vCP2241, indicating the positions of the primers for amplifying the AIV probe and for PCR amplification of the C5 arms and insert.
Figure 18 illustrates the HI antibody responses to H5N1 AIV (A/Vietnam/1194/04 antigens in cats vaccinated with either vFP89 (TROVAC fowlpox virus expressing the H5 gene from A/Turkey/Ireland/1378/83), vFP2211 (TROVAC fowlpox virus expressing the H5 gene from A/Chicken/Indonesia/03), or vCP2241 (ALVAC canarypox virus expressing the H5 gene from A/Chicken/Indonesia/03).
Figure 19 illustrates the HI antibody responses to H5N8 AIV (A/Turkey/Ireland/1378/83, NIBRG14 strain) antigens in cats vaccinated with either vFP89 (TROVAC fowlpox virus expressing the H5 gene from A/Turkey/Ireland/1378/83), vFP2211 (TROVAC fowlpox virus expressing the H5 gene from A/Chicken/Indonesia/03), or vCP2241 (ALVAC canarypox virus expressing the H5 gene from A/Chicken/Indonesia/03).
Figures 20A-20C illustrate the body weights of the animals in this study. Figure 20A shows the body weights of the animals vaccinated with vCP2241 (ALVAC canarypox virus expressing the H5 gene from A/Chicken/Indonesia/03), while Figure 20B displays the body weights of the animals vaccinated with vFP89 (TROVAC fowlpox virus expressing the H5 gene from A/Turkey/Ireland/1378/83). Figure 20C shows the body weights of the animals in the control group.

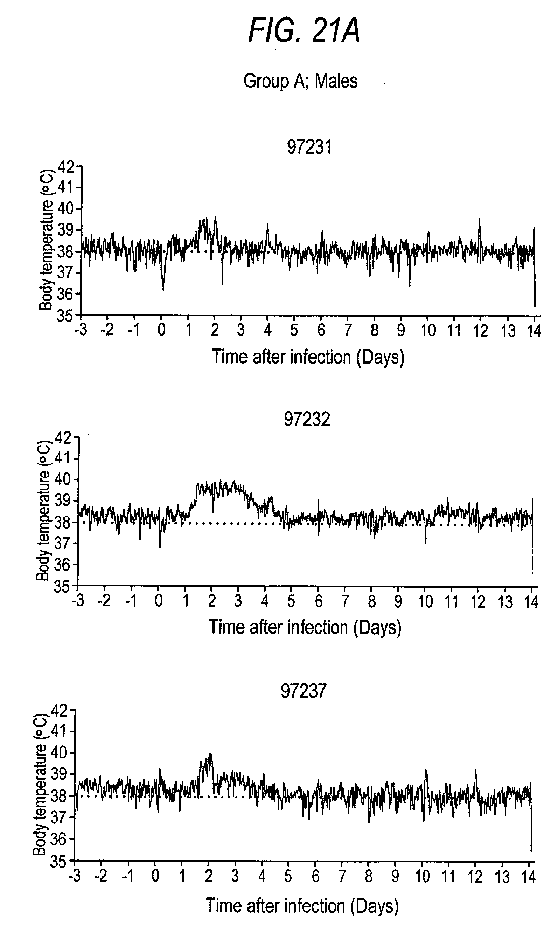

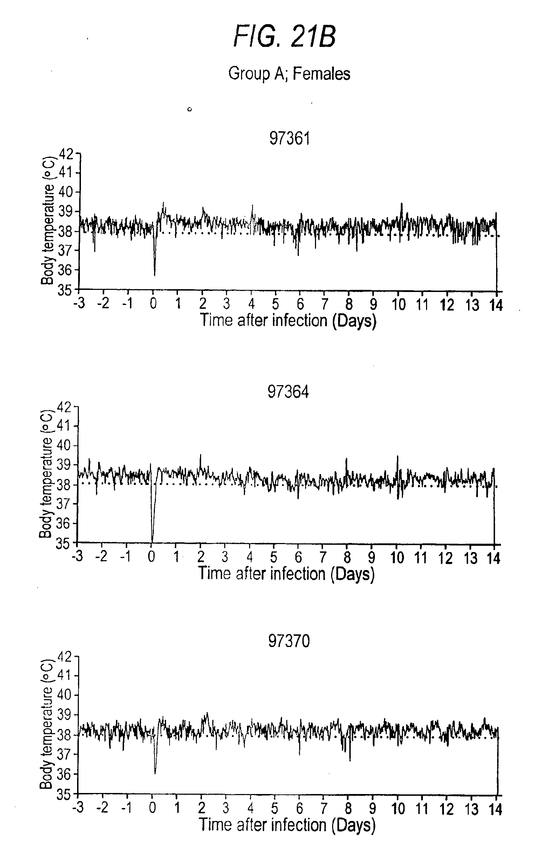

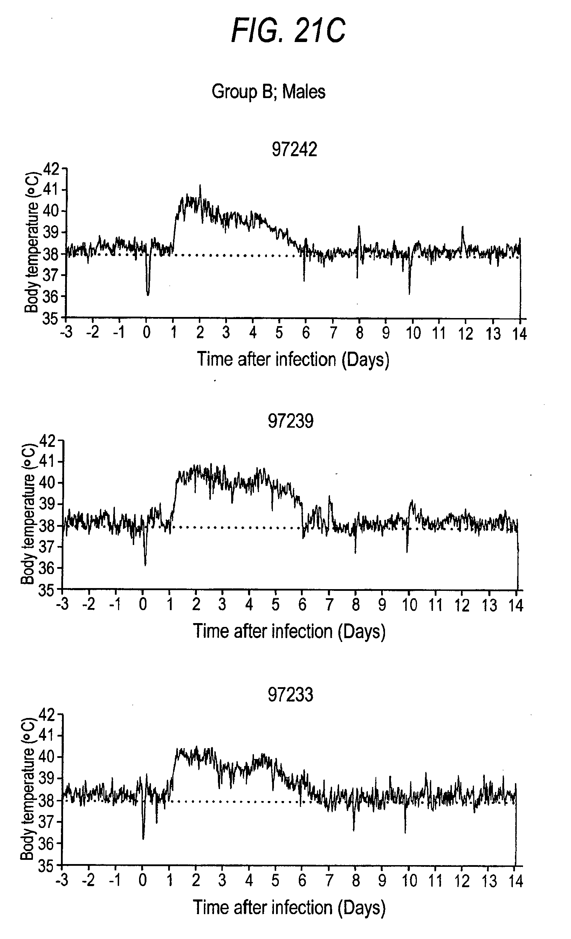

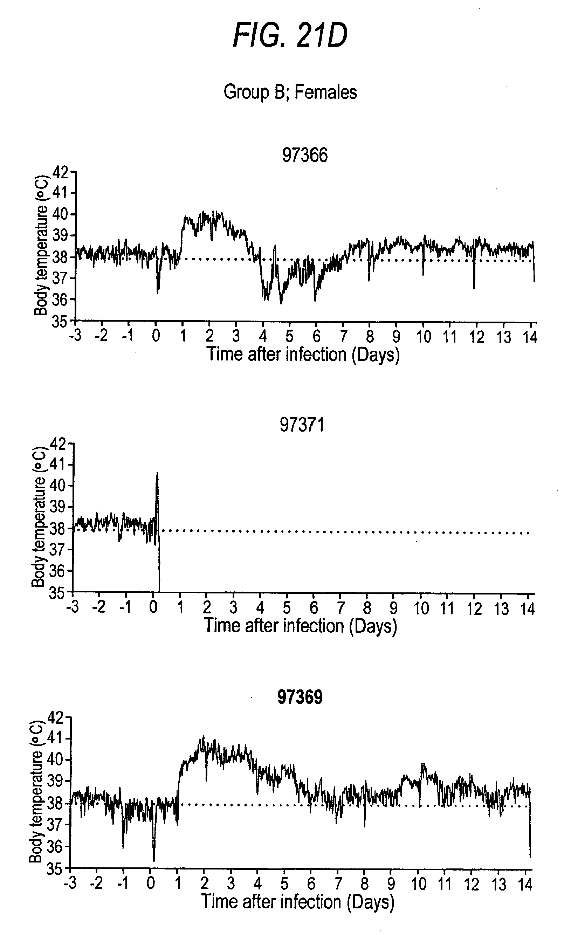

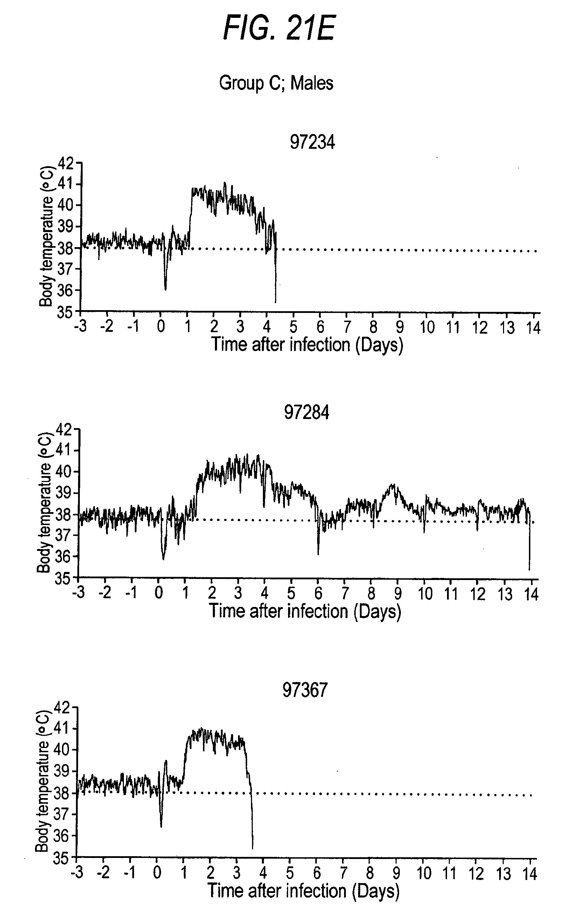

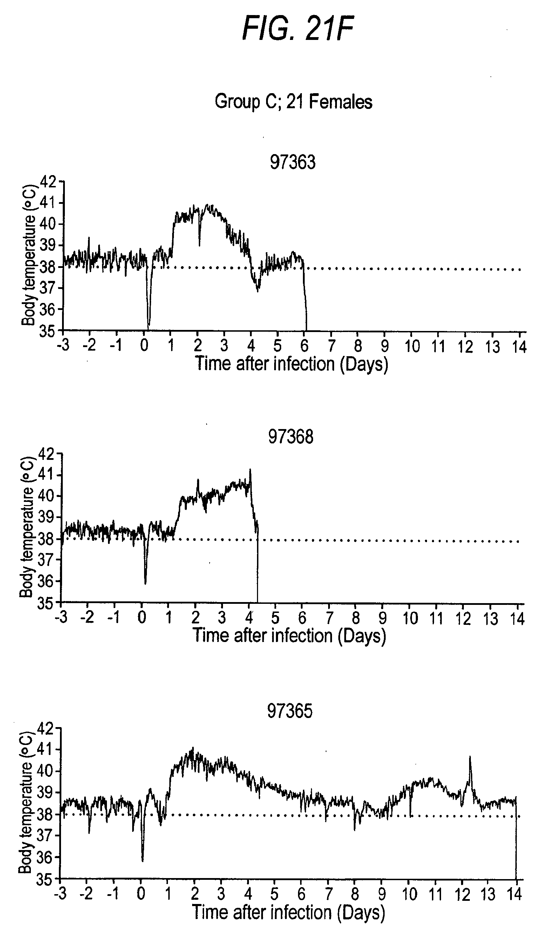

Figures 21A-21E illustrate the body temperatures of the animals in this study. Figures 21A and 21B show the body temperature of the male and female animals, respectively, that were vaccinated with vCP2241 (ALVAC canarypox virus expressing the H5 gene from A/Chicken/Indonesia/03). Figures 21C and 21D display the body temperature of the male and female animals, respectively, that were vaccinated with vFP89 (TROVAC fowlpox virus expressing the H5 gene from A/Turkey/Ireland/1378/83). Figures 21E and 21F shows the body temperature of the male and female animals, respectively, that were in the control group.
Figure 22 illustrates the Kaplain-Meier Survival Analysis, which shows the mortality results of the study. Group A refers to the animals vaccinated with vCP2241 (ALVAC canarypox virus expressing the H5 gene from A/Chicken/Indonesia/03), while Group B refers to animals vaccinated with vFP89 (TROVAC fowlpox virus expressing the H5 gene from A/Turkey/Ireland/1378/83). Group C refers to animals in the control group.
Figure 23 illustrates the HI antibody responses to A/Vietnam/1194/04 and A/Indonesia/5/05 antigens in animals of Group A, which are vaccinated with vCP2241 (ALVAC canarypox virus expressing the H5 gene from A/Chicken/Indonesia/03); Group B, which are vaccinated with vFP89 (TROVAC fowlpox virus expressing the H5 gene from A/Turkey/Ireland/1378/83); or Group C, which are the control group. The white bars refer to the titer results of the HI antibody against A/Vietnam/1194/04, while the black bars refer to the titer results of the HI antibody against A/Indonesia/5/05.

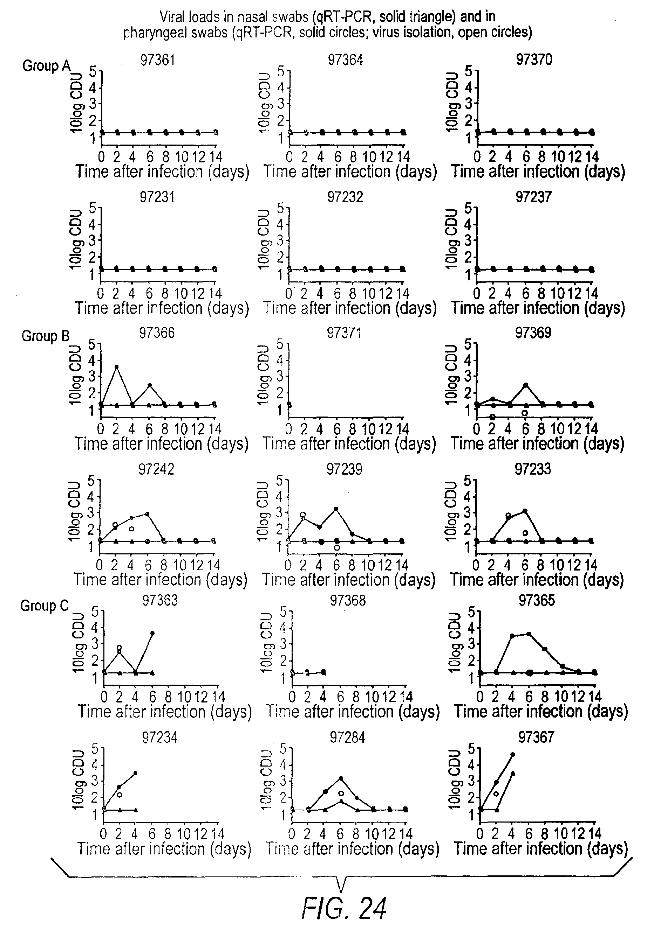

Figure 24 illustrates viral loads in the nasal and pharyngeal swabs in animals of Group A, which are vaccinated with vCP2241 (ALVAC canarypox virus expressing the H5 gene from A/Chicken/Indonesia/03); Group B, which are vaccinated with vFP89 (TROVAC fowlpox virus expressing the H5 gene from A/Turkey/Ireland/1378/83); Group C, which are in the control group. The solid triangles refer to viral load in nasal swabs as determined by qRT-PCR, while the solid circles refer to viral load in pharyngeal swabs as determined by qRT-PCR. The open circles refer to viral load in pharyngeal swabs as determined by virus isolation.
Figure 25 illustrates the Kaplain-Meier Survival Analysis, which shows the mortality results of the study. Group D refers to the animals vaccinated with vCP2241 (ALVAC canarypox virus expressing the H5 gene from A/Chicken/Indonesia/03), while Group E refers to animals vaccinated with vFP89 (TROVAC fowlpox virus expressing the H5 gene from A/Turkey/Ireland/1378/83). Group F refers to animals in the control group.
Figure 26 illustrates the HI antibody responses to A/Vietnam/1194/04 and A/Indonesia/5/05 antigens in animals of Group D, which are vaccinated with vCP2241 (ALVAC canarypox virus expressing the H5 gene from A/Chicken/Indonesia/03); Group E, which are vaccinated with vFP89 (TROVAC fowlpox virus expressing the H5 gene from A/Turkey/Ireland/1378/83); of Group F, which are in the control group. The white bars refer to the titer results of the HI antibody against A/Vietnam/1194/04, while the black bars refer to the titer results of the HI antibody against A/Indonesia/5/05.
Figure 27 illustrates viral loads in the nasal and pharyngeal swabs in animals of Group D, which are vaccinated with vCP2241 (ALVAC canarypox virus expressing the H5 gene from A/Chicken/Indonesia/03); or Group E, which are vaccinated with vFP89 (TROVAC fowlpox virus expressing the H5 gene from A/Turkey/Ireland/1378/83). The solid triangles refer to viral load in nasal swabs as determined by qRT-PCR, while the solid circles refer to viral load in pharyngeal swabs as determined by qRT-PCR. The open circles refer to viral load in pharyngeal swabs as determined by virus isolation.
Figure 28 illustrates the HI antibody responses to H5N1 AIV (A/Vietnam/1194/04, NIBRG14 strain) antigens in cats vaccinated with an inactivated H5N9 AIV (A/Chicken/Italy/22A/98) at dosages of 512UHA/dose and 1536UHA/dose.
Figure 29 illustrates the HI antibody responses to H5N9 AIV (A/Turkey/Wisconsin/68) antigens in cats vaccinated with an inactivated H5N9 AIV (A/Chicken/Italy/22A/98) at dosages of 512UHA/dose and 1536UHA/dose.

### DETAILED DESCRIPTION

The present invention is based, in part, on Applicants' studies demonstrating a recombinant canarypox expressing avian influenza HA is immunogenic in cats.

The present disclosure encompasses any influenza polypeptide, antige, epitope or immunogen that elicits an immunogenic response in an animal, advantageously a vertebrate, more advantageously a Felidae, even more advantageously a cat. The influenza polypeptide, antigen, epitope or immunogen may be any influenza polypeptide, antigen, epitope or immunogen, such as, but not limited to, a protein, peptide or fragment thereof, that elicits, induces or stimulates a response in an animal, advantageously a vertebrate, a Felidae more advantageously a cat.

In an embodiment of the disclosure, the influenza polypeptide, antigen, epitope or immunogen is derived from a feline infected with influenza. For example, but not by limitation, influenza virus may be isolated from the broncho alveolar lavage and/or lung tissues of an affected felid. Isolation and characterization of the nucleotide sequence of the influenza infecting the felid may be done by routine experimentation by a person of ordinary skill in the art.

In another embodiment of the disclosure, the influenza polypeptide, antigen, epitope or immunogen may be derived from an avian infected with influenza or an avian influenza strain. Advantageously, the avian influenza antigen, epitope or immunogen is a hemagglutinin (HA) (e.g., HA precursor, H1, H2), protein, matrix protein (e.g., matrix protein M1 or M2), neuraminidase, nonstructural (NS) protein (e.g., NS1 or NS2), nucleoprotein (NP) and polymerase (e.g., PA polymerase, PB1 polymerase 1 or PB2 polymerase 2).

Examples of avian influenza strains that may be used in methods of the present invention include, but are not limited to, turkey influenza virus strain A/Turkey/Ireland/1378/83 (H5N8) (see, e.g., Taylor et al., 1988b), turkey influenza virus strain A/Turkey/England/63 (H7N3) (see, e.g., Alexander et al., 1979; Rott et al., 1979; Horimoto et al., 2001), turkey influenza virus strain A/Turkey/England/66 (H6N2) (see, e.g., Alexander et al., 1979), A/Turkey/England/69 (H7N2) (see, e.g., Alexander et al., 1979; Horimoto et al., 2001), A/Turkey/Scotland/70 (H6N2) (see, e.g., Banks et al., 2000; Alexander et al., 1979), turkey influenza virus strain A/Turkey/England/N28/73 (H5N2) (see, e.g., Alexander et al., 1979), turkey influenza virus strain A/Turkey/England/110/77 (H6N2) (see, e.g., Alexander et al., 1979), turkey influenza virus strain A/Turkey/England/647/77 (H1N1) (see, e.g., Alexander et al., 1979; Karasin et al., 2002)), turkey influenza virus strain A/turkey/Ontario/7732/66 (H5N9) (see, e.g., Slemons et al., 1972; Philpott et al., 1989), turkey influenza virus strain A/Turkey/England/199/79 (H7N7) (see, e.g., Horimoto et al., 2001), turkey influenza virus strain A/Turkey/Ontario/7732/66 (H5N9) (see, e.g., Horimoto et al., 2001; Panigrahy et al., 1996), turkey influenza virus strain A/Turkey/Ireland/1378/85 (H5N8) (see, e.g., Horimoto et al., 2001; Walker et al., 1993), turkey influenza virus strain A/Turkey/England/50-92/91 (H5N1) (see, e.g., Horimoto et al., 2001; Howard et al., 2006), turkey influenza virus strain A/Turkey/Wisconsin/68 (H5N9), turkey influenza virus strain A/Turkey/Massachusetts/65 (H6N2), turkey influenza virus strain A/Turkey/Oregon/71 (H7N3), (see, e.g., Orlich et al., 1990), turkey influenza virus strain A/Turkey/Ontario/6228/67 (H8N4), turkey influenza virus strain A/Turkey/Wisconsin/66 (H9N2), (see, e.g., Zakstel'skaia et al., 1977), turkey influenza virus strain A/Turkey/England/647/77 (H1N1) (see, e.g., Karasin et al., 2002; Alexander et al., 1979), turkey influenza virus strain A/Turkey/Ontario/6118/68 (H8N4) (see, e.g., Blok et al., 1982), turkey influenza virus strain A/Tur/Ger 3/91 (see, e.g., Zakay-Rones et al., 1995), turkey influenza virus strain A/Turkey/Minnesota/833/80 (H4N2) (see, e.g., Gubareva et al., 1997) chicken influenza virus strain A/Chicken/Indonesia/03 (H5N1), chicken influenza virus strain A/Chicken/FPV/Rostock/1934 (see, e.g., Ohuchi et al., 1994), chicken influenza virus strain A/Chicken/Texas/298313/04 (see, e.g., Lee et al., 2005), chicken influenza virus strain A/Chicken/Texas/167280-4-/02 (see, e.g., Lee et al., 2005), chicken influenza virus strain A/Chicken/Hong Kong/220/97 (see, e.g., Perkins et al., 2001), chicken influenza virus strain A/Chicken/Italy/8/98 (see, e.g., Capua et al., 1999), chicken influenza virus strain A/Chicken/Victoria/76 (H7N7) (see, e.g., Zambon, 2001; Nestorowicz et al., 1987), chicken influenza virus strain A/Chicken/Germany/79 (H7N7) (see, e.g., Rohm et al., 1996), chicken influenza virus strain A/Chicken/Scotland/59 (H5N1) (see, e.g., Horimoto et al., 2001; De et al., 1988; Wood et al., 1993), chicken influenza virus strain A/Chicken/Pennsylvania/1370/83 (H5N2) (see, e.g., Bean et al., 1985; van der Goot et al., 2002), chicken influenza virus strain A/Chicken/Queretaro-19/95 (H5N2) (see, e.g., Horimoto et al., 2001; Garcia et al., 1998), chicken influenza virus strain A/Chicken/Queretaro-20/95 (H5N2) (see, e.g., Horimoto et al., 2001), chicken influenza virus strain A/Chicken/Hong Kong/258/97 (H5N1) (see, e.g., Horimoto et al., 2001; Webster, 1998), chicken influenza virus strain A/Chicken/Italy/1487/97 (H5N2) (see, e.g., Horimoto et al., 2001), chicken influenza virus strain A/Chicken/Leipzig/79 (H7N7) (see, e.g., Horimoto et al., 2001; Rohm et al., 1996), chicken influenza virus strain A/Chicken/Victoria/85 (H7N7) (see, e.g., Horimoto et al., 2001), chicken influenza virus strain A/Chicken/Victoria/92 (H7N3) (see, e.g., Horimoto et al., 2001), chicken influenza virus strain A/Chicken/Queensland/95 (H7N3) (see, e.g., Horimoto et al., 2001), chicken influenza virus strain A/Chicken/Pakistan/1369/95 (H7N2) (see, e.g., Horimoto et al., 2001), chicken influenza virus strain A/Chicken/Pakistan/447-4/95 (H7N3) (see, e.g., Horimoto et al., 2001), chicken influenza virus strain A/Chicken/HK/G9/97 (H9N2) (see, e.g., Leneva et al., 2001), chicken influenza virus strain A/Chicken/Nakorn-Patom/Thailand/CU-K2/2004(H5N1) (see, e.g., Anwar et al., 2006; Viseshakul et al., 2004), chicken influenza virus strain A/Chicken/Hong Kong/31.2/2002 (H5N1), (see, e.g., Anwar et al., 2006), chicken influenza virus strain A/Chicken/Vietnam/C58/04 (H5N1), (see, e.g., Anwar et al., 2006;), chicken influenza virus strain A/Chicken/Vietnam/38/2004(H5N1). (see, e.g., Anwar et al., 2006), chicken influenza virus strain A/Chicken/Alabama/7395/75 (H4N8), (see, e.g., Swayne et al., 1994), chicken influenza virus strain A/Chicken/Germany/N/49 (H10N7), (see, e.g., Yamane et al., 1981), chicken influenza virus strain A/Chicken/Beijing/1/94 (H9N2) (see, e.g., Karasin et al., 2002), chicken influenza virus strain A/Chicken/Hong Kong/G23/97 (H9N2) (see, e.g., Karasin et al., 2002), chicken influenza virus strain A/Chicken/Pennsylvania/8125/83 (H5N2) (see, e.g., Karasin et al., 2002; Shortridge et al., 1998), chicken influenza virus strain A/Chicken/Hong Kong/97 (H5N1) (see, e.g., Chen et al., 2003), duck influenza virus strain A/Duck/Anyang/AVL-1/01 (see, e.g., Tumpey et al., 2002), duck influenza virus strain A/Duck/New York/17542-4/86 (H9N1) (see, e.g., Banks et al., 2000), duck influenza virus strain A/Duck/Alberta/28/76 (H4N6) (see, e.g., Blok et al., 1982), duck influenza virus strain A/Duck/Nanchang/4-165/2000 (H4N6) (see, e.g., Liu et al., 2003), duck influenza virus strain A/Duck/Germany/49 (H10N7) (see, e.g., Blok et al., 1982), duck influenza virus strain A/Black Duck/Australia/702/78 (H3N8) (see, e.g., Blok et al., 1982), duck influenza virus strain A/Duck/Vietnam/11/2004 (H5N1), (see, e.g., Anwar et al., 2006), duck influenza virus strain A/Duck/Alberta/60/76 (H12N5), (see, e.g., Baez et al., 1981), duck influenza virus strain A/Duck/Hong Kong/196/77 (H1) (see, e.g., Karasin et al., 2002; Kanegae et al., 1994), duck influenza virus strain A/Duck/Wisconsin/1938/80 (H1N1) (see, e.g., Karasin et al., 2002), duck influenza virus strain A/DuckBavaria/2/77 (H1N1) (see, e.g., Karasin et al., 2002; Ottis et al., 1980), duck influenza virus strain A/DuckBavaria/1/77 (H1N1) (see, e.g., Ottis et al., 1980), duck influenza virus strain A/Duck/Australia/749/80 (H1N1) (see, e.g., Karasin et al., 2002), duck influenza virus strain A/Duck/Hong Kong/Y280/97 (H9N2) (see, e.g., Karasin et al., 2002; Guan et al., 2000), duck influenza virus strain A/Duck/Alberta/35/76 H1N1) (see, e.g., Austin et al., 1990), avian influenza virus strain A/Mallard duck/Gurjev/263/82 (H14N5), (see, e.g., Kawaoka et al., 1990), avian influenza virus strain A/Mallard duck/PA/10218/84 (H5N2) (see, e.g., Smirnov et al., 2000), avian influenza virus strain A/Mallard duck/Astrakhan/244/82 (H14N6) (see, e.g., Karasin et al., 2002), goose influenza virus strain A/Goose/Guangdong/1/96 (see, e.g., Xu et al., 1999), goose influenza virus strain A/Goose/Leipzig/137-8/79 (H7N7) (see, e.g., Horimoto et al., 2001), goose influenza virus strain A/Goose/Hong Kong/W222/97 (H6N7) (see, e.g., Chin et al., 2002), goose influenza virus strain A/Goose/Leipzig/187-7/79 (H7N7) (see, e.g., Horimoto et al., 2001), goose influenza virus strain A/Goose/Leipzig/192-7/79 (H7N7) (see, e.g., Horimoto et al., 2001), avian influenza virus strain A/Env/HK/437-4/99 (see, e.g., Cauthen et al., 2000), avian influenza virus strain A/Env/HK/437-6/99 (see, e.g., Cauthen et al., 2000), avian influenza virus strain A/Env/HK/437-8/99 (see, e.g., Cauthen et al., 2000), avian influenza virus strain A/Env/HK/437-10/99, (see, e.g., Cauthen et al., 2000), avian influenza virus strain A/Fowl plague virus strain/Dutch/27 (H7N7) (see, e.g., Horimoto et al., 2001; Carter et al., 1982), avian influenza virus strain A/Fowl plague virus strain/Dobson/27 (H7N7) (see, e.g., Horimoto et al., 2001), avian influenza virus strain A/Fowl plague virus strain/Rostock/34 (H7N1) (see, e.g., Horimoto et al., 2001; Takeuchi et al., 1994), avian influenza virus strain A/Fowl plague virus strain/Egypt/45 (H7N1) (see, e.g., Horimoto et al., 2001), avian influenza virus strain A/Fowl plague virus strain/Weybridge (H7N7) (see, e.g., Tonew et al., 1982), avian influenza virus strain A/Tern/South Africa/61 (H5N3) (see, e.g., Horimoto et al., 2001; Perkins et al., 2002; Walker et al., 1992), avian influenza virus strain A/Tern/Australia/G70C/75 (H11N9) (see, e.g., Pruett et al., 1998), avian influenza virus strain A/Quail/Vietnam/36/04(H5N1). (see, e.g., Anwar et al., 2006), avian influenza virus strain A/Gull/Maryland/704/77 (H13N6), (see, e.g., Iamnikova et al., 1989), avian influenza virus strain A/Black-headed gull/Sweden/5/99 (H16N3) (see, e.g., Fouchier et al., 2005), avian influenza virus strain A/Herring gull/DE/677/88 (H2N8) (see, e.g., Saito et al., 1993), avian influenza virus strain A/Swan/Italy/179/06 (H5N1) (see, e.g., Terregino et al., 2006), avian influenza virus strain A/Hong Kong/156/97 (A/HK/156/97) (see, e.g., Leneva et al., 2001; Claas et al., 1998; Cauthen et al., 2000), avian influenza virus strain A/Quail/HK/G1/97 (H9N2) (see, e.g., Leneva et al., 2001), avian influenza virus strain A/Quail/Hong Kong/AF157/93 (H9N2) (see, e.g., Karasin et al., 2002), avian influenza virus strain A/Teal/HK/W312/97 (H6N1) (see, e.g., Leneva et al., 2001), avian influenza virus strain A/Shearwater/West Australia/2576/79 (H15N9) (see, e.g., Rohm et al., 1996), avian influenza virus strain A/Shearwater/Australia/72 (H6N5) (see, e.g., Harley et al., 1990), avian influenza virus strain A/Hong Kong/212/03 (see, e.g., Shinya et al., 2005), avian influenza virus strain A/England/321/77 (H3N2) (see, e.g., Hauptmann et al., 1983), avian pandemic influenza A viruses of avian origin (see, e.g., Audsley et al., 2004) avian H5N1 influenza virus, , avian H7N1 influenza strain (see, e.g., Foni et al., 2005), avian H9N2 influenza virus (see, e.g., Leneva et al., 2001), and avian influenza virus, cold-adapted (ca) and temperature sensitive (ts) master donor strain, A/Leningrad/134/17/57 (H2N2) (see, e.g., Youil et al., 2004).

Other influenza strains that may be used in methods of the present invention include, but are not limited to, equine influenza virus (A/Equi 2 (H3N8), Newmarket 1/93) (see, e.g., Mohler et al., 2005; Nayak et al., 2005), equine-2 influenza virus (EIV; subtype H3N8) (see, e.g., Lin et al., 2001), equine-2 influenza virus, A/Equine/Kentucky/1/91 (H3N8) (see, e.g., Youngner et al., 2001), equine influenza virus strain A/Equine/Berlin/2/91 (H3N8) (see, e.g., Ilobi et al., 1998), equine influenza virus strain A/Equine/Cambridge/1/63 (H7N7) (see, e.g., Gibson et al., 1992), equine influenza virus strain A/Equine/Prague/1/56 (H7N7) (see, e.g., Karasin et al., 2002; Appleton et al., 1989), equine influenza virus strain A/Eq/Kentucky/98 (see, e.g., Crouch et al., 2004), equine influenza virus strain A/Equi 2 (Kentucky 81) (see, e.g., Short et al., 1986; Homer et al., 1988), equine influenza virus strain A/Equine/Kentucky/1/81 (Eq/Ky) (see, e.g., Breathnach et al., 2004), equine influenza virus strain A/Equine/Kentucky/1/81 (H3N8) (see, e.g., Olsen et al., 1997; Morley et al., 1995; Ozaki et al., 2001; Sugiura et al., 2001; Goto et al., 1993), equine influenza virus strain A/Equine/Kentucky/1/91 (H3N8) (see, e.g., Youngner et al., 2001), equine influenza virus strain A/Equine/Kentucky/1277/90 (Eq/Kentucky) (see, e.g., Webster et al., 1993), equine influenza virus strain A/Equine/Kentucky/2/91 (H3N8) (see, e.g., Donofrio et al., 1994), equine influenza virus strain A/Equine/Kentucky/79 (H3N8) (see, e.g., Donofrio et al., 1994), equine influenza virus strain A/Equine/Kentucky/81 (see, e.g., Sugiura et al., 2001), equine influenza virus strain A/Equine/Kentucky/91 (H3N8) (see, e.g., Gross et al., 1998), equine influenza virus strain A/Equine-2/Kentucky/95 (H3N8) (see, e.g., Heldens et al., 2004) and equine influenza virus strain A/Equine-2/Kentucky/98 (see, e.g., Chambers et al., 2001), equine influenza virus strain A/Eq/Newmarket/1/77 (see, e.g., Lindstrom et al., 1998), equine influenza virus strain A/Eq/Newmarket/5/03 (see, e.g., Edlund Toulemonde et al., 2005), equine influenza virus strain A/Equi 2 (H3N8), Newmarket 1/93 (see, e.g., Mohler et al., 2005; Nayak et al., 2005), equine influenza virus strain A/Equi-2/Newmarket-1/93 (see, e.g., Heldens et al., 2002), equine influenza virus strain A/Equine/Newmarket/2/93 (see, e.g., Wattrang et al., 2003), equine influenza virus strain A/Equine/Newmarket/79 (H3N8) (see, e.g., Duhaut et al., 2000 ; Noble et al., 1994; Duhaut et al., 1998; Hannant et al., 1989; Hannant et al., 1989; Hannant et al., 1988; Richards et al., 1992; Heldens et al., 2004), equine influenza virus strain A/Equine/Newmarket/1/77 (H7N7) (see, e.g., Goto et al., 1993; Sugiura et al., 2001) and equine influenza virus strain A/Equine-2/Newmarket-2/93 (see, e.g., Heldens et al., 2004), equine influenza virus strain A/Eq/Miami/63 (H3N8) (see, e.g., van Maanen et al., 2003), A/Equi 1 (Prague strain) (see, e.g., Homer et al., 1988; Short et al., 1986), equine influenza virus strain A/Equi 2 (Miami) (see, e.g., Short et al., 1986), equine influenza virus strain A/Equi-1/Prague/56 (Pr/56) (see, e.g., Heldens et al., 2002), equine influenza virus strain A/Equi-2/Suffolk/89 (Suf/89) (see, e.g., Heldens et al., 2002), equine influenza virus strain A/Equine 2/Sussex/89 (H3N8) (see, e.g., Mumford et al., 1994), equine influenza virus strain A/Equine/Sussex/89 (see, e.g., Wattrang et al., 2003), equine influenza virus strain A/Equine-2/Saskatoon/90 (see, e.g., Chambers et al., 2001), equine influenza virus strain A/Equine/Prague/1/56 (H7N7) (see, e.g., Donofrio et al., 1994; Morley et al., 1995), equine influenza virus strain A/Equine/Miami/1/63 (H3N8) (see, e.g., Morley et al., 1995; Ozaki et al., 2001; Thomson et al., 1977; Mumford et al., 1988; Donofrio et al., 1994; Mumford et al., 1983), A/Aichi/2/68 (H3N2) (see, e.g., Ozaki et al., 2001), equine influenza virus strain A/Equine/Tokyo/2/71 (H3N8) (see, e.g., Goto et al., 1993), equine influenza virus strain A/Eq/LaPlata/1/88 (see, e.g., Lindstrom et al., 1998), equine influenza virus strain A/Equine/Jilin/1/89 (Eq/Jilin) (see, e.g., Webster et al., 1993), equine influenza virus strain A/Equine/Alaska/1/91 (H3N8) (see, e.g., Webster et al., 1993), equine influenza virus strain A/Equine/Saskatoon/1/91 (H3N8) (see, e.g., Morley et al., 1995), equine influenza virus strain A/Equine/Rome/5/91 (H3N8) (see, e.g., Sugiura et al., 2001), equine influenza virus strain A/Equine/La Plata/1/93 (H3N8) (see, e.g., Ozaki et al., 2001), equine influenza virus strain A/Equine/La Plata/1/93 (LP/93) (see, e.g., Sugiura et al., 2001), equine influenza virus strain A/Eq/Holland/1/95 (H3N8) (see, e.g., van Maanen et al., 2003) and equine influenza virus strain A/Eq/Holland/2/95 (H3N8) (see, e.g., van Maanen et al., 2003), human influenza virus A(H3N2) isolates (see, e.g., Abed et al., 2002), human influenza virus A/Memphis/1/71 (H3N2) (see, e.g., Suzuki et al., 1996), human influenza virus A/Nanchang/933/95 (H3N2) virus (see, e.g., Scholtissek et al., 2002), human influenza virus A/PR/8/34 (H1N1) virus (see, e.g., Scholtissek et al., 2002), human influenza virus A/Singapore/57 (H2N2) virus (see, e.g., Scholtissek et al., 2002), influenza virus A (see, e.g., Chare et al., 2003), influenza virus A/HK/213/03 (see, e.g., Guan et al., 2004; Anwar et al., 2006), influenza virus strain A/HK/483/97 (see, e.g., Cheung et al., 2002), influenza virus strain A/HK/486/97 (see, e.g., Cheung et al., 2002), influenza virus strain A/Thailand/5(KK-494)/2004 (H5N1).(see, e.g., Anwar et al., 2006), influenza virus strain A PR/8/34 (PR8) virus strain (H1N1 subtype) (see, e.g., Mantani et al., 2001), influenza virus strain A/Aichi/2/68(H3N2) (see, e.g., Miyamoto et al., 1998), influenza virus strain A/Ann Arbor/6/60 cold-adapted virus strain (see, e.g., Treanor et al., 1994), influenza virus strain A/Beijing 32/92 (H3N2) (see, e.g., Zakay-Rones et al., 1995), influenza virus strain A/Charlottesville/31/95 (H1N1) (see, e.g., Gubareva et al., 2002), influenza virus strain A/Kawasaki/86 (H1N1) virus strain (see, e.g., Staschke et al., 1998), influenza virus strain A/Korea/82 (H3N2) (see, e.g., Treanor et al., 1994), influenza virus strain A/Leningrad/134/57 (see, e.g., Egorov et al., 1998), influenza virus strain A/NWS/33 (H1N1) (see, e.g., Sidwell et al., 1998), influenza virus strain A/PR/8/34(H1N1) (see, e.g., Miyamoto et al., 1998), influenza virus strain A/PR8/34 (see, e.g., Nunes-Correia et al., 1999; Tree et al., 2001), influenza virus strain A/Puerto Rico (PR)/8/34 (see, e.g., Egorov et al., 1998), influenza virus strain A/Puerto Rico/8-Mount Sinai (see, e.g., Mazanec et al., 1995), influenza virus strain A/Shangdong 9/93 (H3N2) (see, e.g., Zakay-Rones et al., 1995; Sidwell et al., 1998), influenza virus strain A/Shingapol/1/57(H2N2) (see, e.g., Miyamoto et al., 1998), influenza virus strain A/Singapore 6/86 (H1N1) (see, e.g., Zakay-Rones et al., 1995), influenza virus strain A/Singapore/1/57 (H2N2) (see, e.g., Bantia et al., 1998), influenza virus strain A/Texas 36/91 (H1N1) (see, e.g., Zakay-Rones et al., 1995), influenza virus strain A/Texas/36/91 (H1N1) virus strain (see, e.g., Gubareva et al., 2001; Halperin et al., 1998), influenza virus strain A/Texas/36/91(H1N1) (see, e.g., Hayden et al., 1994), influenza virus strain A/Udorn/72 virus infection (see, e.g., Shimizu et al., 1999), influenza virus A/Victoria/3/75 (H3N2) (see, e.g., Sidwell et al., 1998), influenza virus A/Virginia/88(H3N2) (see, e.g., Hayden et al., 1994), influenza virus A/WSN/33 (H1N1) (see, e.g., Lu et al., 2002), influenza virus A/WSN/33 (see, e.g., Gujuluva et al., 1994), influenza virus B (see, e.g., Chare et al., 2003), influenza virus B/Ann Arbor 1/86 (see, e.g., Zakay-Rones et al., 1995), influenza virus B/Harbin/7/94 (see, e.g., Halperin et al., 1998), influenza virus B/Hong Kong/5/72 (see, e.g., Sidwell et al., 1998), influenza virus B/Lee/40 (see, e.g., Miyamoto et al., 1998), influenza virus B/Victoria group (see, e.g., Nakagawa et al., 1999), influenza virus B/Yamagata 16/88 (see, e.g., Zakay-Rones et al., 1995), influenza virus B/Yamagata group (see, e.g., Nakagawa et al., 1999), influenza virus B/Yamanashi/166/98 (see, e.g., Hoffmann et al., 2002), influenza virus C (see, e.g., Chare et al., 2003), influenza virus strain A/Equi/2/Kildare/89 (see, e.g., Quinlivan et al., 2004), influenza virus type B/Panama 45/90 (see, e.g., Zakay-Rones et al., 1995), live, cold-adapted, temperature-sensitive (ca/ts) Russian influenza A vaccines (see, e.g., Palker et al., 2004), swine H1 and H3 influenza viruses (see, e.g., Gambaryan et al., 2005), swine influenza A viruses (see, e.g., Landolt et al., 2005), swine influenza virus (SIV) (see, e.g., Clavijo et al., 2002), swine influenza virus A/Sw/Ger 2/81 (see, e.g., Zakay-Rones et al., 1995), swine influenza virus A/Sw/Ger 8533/91 (see, e.g., Zakay-Rones et al., 1995), swine influenza virus strain A/Swine/Wisconsin/125/97 (H1N1) (see, e.g., Karasin et al., 2002; Karasin et al., 2006), swine influenza virus strain A/Swine/Wisconsin/136/97 (H1N1) (see, e.g., Karasin et al., 2002), swine influenza virus strain A/Swine/Wisconsin/163/97 (H1N1) (see, e.g., Karasin et al., 2002), swine influenza virus strain A/Swine/Wisconsin/164/97 (H1N1) (see, e.g., Karasin et al., 2002), swine influenza virus strain A/Swine/Wisconsin/166/97 (H1N1) (see, e.g., Karasin et al., 2002), swine influenza virus strain A/Swine/Wisconsin/168/97 (H1N1) (see, e.g., Karasin et al., 2002), swine influenza virus strain A/Swine/Wisconsin/235/97 (H1N1) (see, e.g., Karasin et al., 2002; Olsen et al., 2000), swine influenza virus strain A/Swine/Wisconsin/238/97 (H1N1) (see, e.g., Karasin et al., 2002; Ayora-Talavera et al., 2005), swine influenza virus strain A/Swine/Wisconsin/457/98 (H1N1) (see, e.g., Karasin et al., 2002), swine influenza virus strain A/Swine/Wisconsin/458/98 (H1N1) (see, e.g., Karasin et al., 2002; Karasin et al., 2006), swine influenza virus strain A/Swine/Wisconsin/464/98 (H1N1) (see, e.g., Karasin et al., 2002; Karasin et al., 2006), swine influenza virus strain A/Swine/Indiana/1726/88 (H1N1) (see, e.g., Karasin et al., 2002; Macklin et al., 1998), swine influenza virus strain A/Swine/Indiana/9K035/99 (H1N2) (see, e.g., Karasin et al., 2002; Karasin et al., 2000), swine influenza virus strain A/Swine/Nebraska/1/92 (H1N1) (see, e.g., Karasin et al., 2002), swine influenza virus strain A/Swine/Quebec/91 (H1N1) (see, e.g., Karasin et al., 2002), swine influenza virus strain A/Swine/Quebec/81 (H1N1) (see, e.g., Karasin et al., 2002), swine influenza virus strain A/Swine/New Jersey/11/76 (H1N1) (see, e.g., Karasin et al., 2002), swine influenza virus strain A/Swine/Ehime/1/80 (H1N2) (see, e.g., Karasin et al., 2002; Nerome et al., 1985), swine influenza virus strain A/Swine/England/283902/93 (H1N1) (see, e.g., Karasin et al., 2002), swine influenza virus strain A/Swine/England/195852/92 (H1N1) (see, e.g., Karasin et al., 2002; Brown et al., 1993), swine influenza virus strain A/Swine/Germany/8533/91 (H1N1) (see, e.g., Karasin et al., 2002), swine influenza virus strain A/Swine/Germany/2/81 (H1N1) (see, e.g., Karasin et al., 2002), swine influenza virus strain A/Swine/Nebraska/209/98 (H3N2) (see, e.g., Karasin et al., 2002), A/Swine/Iowa/533/99 (H3N2) (see, e.g., Karasin et al., 2002), swine influenza virus strain A/Swine/Iowa/569/99 (H3N2) (see, e.g., Karasin et al., 2002), swine influenza virus strain A/Swine/Minnesota/593/99 (H3N2) (see, e.g., Karasin et al., 2002; Ayora-Talavera et al., 2005), swine influenza virus strain A/Swine/Iowa/8548-1/98 (H3N2) (see, e.g., Karasin et al., 2002), swine influenza virus strain A/Swine/Minnesota/9088-2/98 (H3N2) (see, e.g., Karasin et al., 2002), swine influenza virus strain A/Swine/Texas/4199-2/98 (H3N2) (see, e.g., Karasin et al., 2002), swine influenza virus strain A/Swine/Ontario/41848/97 (H3N2) (see, e.g., Karasin et al., 2002), swine influenza virus strain A/Swine/North Carolina/35922/98 (H3N2) (see, e.g., Karasin et al., 2002), /Swine/Colorado/1/77 (H3N2) (see, e.g., Karasin et al., 2002), swine influenza virus strain A/Swine/Hong Kong/3/76 (H3N2) (see, e.g., Karasin et al., 2002), swine influenza virus strain A/Swine/Hong Kong/13/77 (H3N2) (see, e.g., Karasin et al., 2002), swine influenza virus strain A/Swine/Nagasaki/1/90 (H1N2) (see, e.g., Karasin et al., 2002), swine influenza virus strain A/Swine/Nagasaki/1/89 (H1N2) (see, e.g., Karasin et al., 2002), swine influenza virus strain A/Swine/Wisconsin/1915/88 (H1N1) (see, e.g., Karasin et al., 2002), swine influenza virus strain A/Swine/Iowa/17672/88 (H1N1) (see, e.g., Karasin et al., 2002), swine influenza virus strain A/Swine/Tennessee/24/77 (H1N1) (see, e.g., Karasin et al., 2002), swine influenza virus strain A/Swine/Ontario/2/81 (H1N1) (see, e.g., Karasin et al., 2002), swine influenza virus strain A/Swine/Wisconsin/1/67 (H1N1) (see, e.g., Karasin et al., 2002), swine influenza virus strain A/Swine/Italy/1521/98 (H1N2) (see, e.g., Marozin et al., 2002), swine influenza virus strain A/Swine/Italy/839/89 (H1N1) (see, e.g., Karasin et al., 2002), swine influenza virus strain A/Swine/Hong Kong/126/82 (H3N2) (see, e.g., Karasin et al., 2002), influenza virus strain A/Idaho/4/95 (H3N2) (see, e.g., Karasin et al., 2002), influenza virus strain A/Johannesburg/33/94 (H3N2) (see, e.g., Karasin et al., 2002; Johansson et al., 1998), influenza virus strain A/Bangkok/1/79 (H3N2) (see, e.g., Karasin et al., 2002; Nelson et al., 2001), influenza virus strain A/Udorn/72 (H3N2) (see, e.g., Karasin et al., 2002; Markoff et al., 1982), influenza virus strain A/Hokkaido/2/92 (H1N1) (see, e.g., Karasin et al., 2002), influenza virus strain A/Thailand/KAN-1/04 (see, e.g., Puthavathana et al., 2005; Amonsin et al., 2006), influenza virus strain A/England/1/53 (see, e.g., Govorkova EA, et al.,, 1995), influenza virus strain A/Vietnam/3046/2004 (H5N1), (see, e.g., Anwar et al., 2006), influenza virus strain A/Vietnam/1203/2004 (H5N1), (see, e.g., Anwar et al., 2006; Gao et al., 2006), influenza virus strain A/tiger/Thailand/SPB-1(H5N1), (see, e.g., Anwar et al., 2006), influenza virus strain A/Japan/305/57 (H2N2) (see, e.g., Naeve et al., 1990; Brown et al., 1982), influenza virus strain A/Adachi/2/57 (H2N2) (see, e.g., Gething et al., 1980), influenza virus strain A/Camel/Mongolia/82 (H1N1) (see, e.g., Yamnikova et al., 1993), influenza virus strain A/RI/5/57 (H2N2) (see, e.g., Elleman et al., 1982), influenza virus strain A/Whale/Maine/1/84 (H13N9) (see, e.g., Air et al., ,1987), influenza virus strain A/Taiwan/1/86 (H1N1) (see, e.g., Karasin et al., 2002; Brown, 1988), influenza virus strain A/Bayern/7/95 (H1N1) (see, e.g., Karasin et al., 2002), influenza virus strain A/USSR/90/77 (H1N1) (see, e.g., Karasin et al., 2002; Iftimovici et al., 1980), influenza virus strain A/Wuhan/359/95 (H3N2) (see, e.g., Karasin et al., 2002; Hardy et al., 2001), influenza virus strain A/Hong Kong/5/83 (H3N2) (see, e.g., Karasin et al., 2002), influenza virus strain A/Memphis/8/88 (H3N2) (see, e.g., Karasin et al., 2002; Hatta et al., 2002), influenza virus strain A/Beijing/337/89 (H3N2) (see, e.g., Karasin et al., 2002), influenza virus strain A/Shanghai/6/90 (H3N2) (see, e.g., Karasin et al., 2002), influenza virus strain A/Akita/1/94 (H3N2) (see, e.g., Karasin et al., 2002), influenza virus strain A/Akita/1/95 (H3N2) (see, e.g., Karasin et al., 2002), influenza virus strain A/Memphis/6/90 (H3N2) (see, e.g., Karasin et al., 2002), influenza virus strain A/Udorn/307/72 (H3N2) (see, e.g., Karasin et al., 2002; Iuferov et al., 1984), influenza virus strain A/Singapore/1/57 (H2N2) (see, e.g., Karasin et al., 2002; Zhukova et al., 1975), influenza virus strain A/Ohio/4/83 (H1N1) (see, e.g., Karasin et al., 2002), influenza virus strain Madin Darby Canine Kidney (MDCK)-derived cell line (see, e.g., Halperin et al., 2002), mouse-adapted influenza virus strain A/Guizhou/54/89 (H3N2 subtype) (see, e.g., Nagai et al., 1995), mouse-adapted influenza virus A/PR/8/34 (A/PR8) (see, e.g., Nagai et al., 1995), mouse-adapted influenza virus B/Ibaraki/2/85 (see, e.g., Nagai et al., 1995), Russian live attenuated influenza vaccine donor strains A/Leningrad/134/17/57, A/Leningrad/134/47/57 and B/USSR/60/69 (see, e.g., Audsley et al. 2005).

In another embodiment of the disclosure, the avian influenza polypeptide, antigen, epitope or immunogen may be derived from an avian infected with influenza or an avian influenza strain derived from a recent isolate.

In one embodiment of the disclosure, the influenza vaccine comprises one or more influenza polypeptide, antigen, epitope or immunogen, wherein the influenza isolate is selected from one or more strain of influenza. In one embodiment of the disclosure, the influenza vaccine comprises one or more influenza isolates chosen from the group consisting of a feline influenza isolate, an avian influenza isolate, or mixtures thereof. In yet another embodiment of the disclosure, the influenza vaccine comprises one or more influenza isolates chosen from the group consisting of an inactivated feline influenza isolate, an inactivated avian influenza isolate, or mixtures thereof.

As used herein, the term "antigen" or "immunogen" means a substance that induces a specific immune response in a host animal. The antigen may comprise a whole organism, killed, attenuated or live; a subunit or portion of an organism; a recombinant vector containing an insert with immunogenic properties; a piece or fragment of DNA capable of inducing an immune response upon presentation to a host animal; a protein, a polypeptide, a peptide, an epitope, a hapten, or any combination thereof. Alternately, the immunogen or antigen may comprise a toxin or antitoxin.

The term "immunogenic protein or peptide" as used herein refers to peptides and polypeptides that are immunologically active in the sense that once administered to the host, it is able to evoke an immune response of the humoral and/or cellular type directed against the protein. Preferably the protein fragment is such that it has substantially the same immunological activity as the total protein. Thus, a protein fragment according to the disclosure comprises or consists essentially of or consists of at least one epitope or antigenic determinant. The term epitope relates to a protein site able to induce an immune reaction of the humoral type (B cells) and/or cellular type (T cells).

The term "immunogenic protein or peptide" further contemplates deletions, additions and substitutions to the sequence, so long as the polypeptide functions to produce an immunological response as defmed herein. In this regard, particularly preferred substitutions will generally be conservative in nature, i.e., those substitutions that take place within a family of amino acids. For example, amino acids are generally divided into four families: (1) acidic--aspartate and glutamate; (2) basic--lysine, arginine, histidine; (3) non-polar--alanine, valine, leucine, isoleucine, proline, phenylalanine, methionine, tryptophan; and (4) uncharged polar--glycine, asparagine, glutamine, cystine, serine threonine, tyrosine. Phenylalanine, tryptophan, and tyrosine are sometimes classified as aromatic amino acids. It is reasonably predictable that an isolated replacement of leucine with isoleucine or valine, or vice versa; an aspartate with a glutamate or vice versa; a threonine with a serine or vice versa; or a similar conservative replacement of an amino acid with.a structurally related amino acid, will.not have a major effect on the biological activity. Proteins having substantially the same amino acid sequence as the reference molecule but possessing minor amino acid substitutions that do not substantially affect the immunogenicity of the protein are, therefore, within the definition of the reference polypeptide.

The term "epitope" refers to the site on an antigen or hapten to which specific B cells and/or T cells respond. The term is also used interchangeably with "antigenic determinant" or "antigenic determinant site". Antibodies that recognize the same epitope can be identified in a simple immunoassay showing the ability of one antibody to block the binding of another antibody to a target antigen.

An "immunological response" to a composition or vaccine is the development in the host of a cellular and/or antibody-mediated immune response to a composition or vaccine of interest. Usually, an "immunological response" includes but is not limited to one or more of the following effects: the production of antibodies, B cells, helper T cells, and/or cytotoxic T cells, directed specifically to an antigen or antigens included in the composition or vaccine of interest. Preferably, the host will display either a therapeutic or protective immunological response such that resistance to new infection will be enhanced and/or the clinical severity of the disease reduced. Such protection will be demonstrated by either a reduction or lack of symptoms normally displayed by an infected host, a quicker recovery time and/or a lowered viral titer in the infected host.

The terms "immunogenic" protein or polypeptide as used herein also refers to an amino acid sequence which elicits an immunological response as described above. An "immunogenic" protein or polypeptide, as used herein, includes the full-length sequence of the protein, analogs thereof, or immunogenic fragments thereof. By "immunogenic fragment" is meant a fragment of a protein which includes one or more epitopes and thus elicits the immunological response described above. Such fragments can be identified using any number of epitope mapping techniques, well known in the art. See, e.g., Epitope Mapping Protocols in Methods in Molecular Biology, Vol. 66 (Glenn E. Morris, Ed., 1996). For example, linear epitopes may be determined by e.g., concurrently synthesizing large numbers of peptides on solid supports, the peptides corresponding to-portions of the protein-molecule, and reacting the peptides with antibodies while the peptides are still attached to the supports. Such techniques are known in the art and described in, e.g., U.S. Pat. No. 4,708,871; Geysen et al., 1984; Geysen et al., 1986. Similarly, conformational epitopes are readily identified by determining spatial conformation of amino acids such as by, e.g., x-ray crystallography and 2-dimensional nuclear magnetic resonance. See, e.g., Epitope Mapping Protocols, supra. Methods especially applicable to the proteins of T. parva are fully described in the PCT Application WO 2005/007691.

Synthetic antigens are also included within the definition, for example, polyepitopes, flanking epitopes, and other recombinant or synthetically derived antigens. See, e.g., Bergmann et al., 1993; Bergmann et al., 1996; Suhrbier 1997; Gardner et al., 1998. Immunogenic fragments, for purposes of the present disclosure, will usually include at least about 3 amino acids, preferably at least about 5 amino acids, more preferably at least about 10-15 amino acids, and most preferably about 15-25 amino acids or more amino acids, of the molecule. There is no critical upper limit to the length of the fragment, which could comprise nearly the full-length of the protein sequence, or even a fusion protein comprising at least one epitope of the protein.

Accordingly, a minimum structure of a polynucleotide expressing an epitope is that it comprises or consists essentially of or consists of nucleotides to encode an epitope or antigenic determinant of an influenza protein or polyprotein. A polynucleotide encoding a fragment of the total protein or polyprotein, more advantageously, comprises or consists essentially of or consists of a minimum of 15 nucleotides, at least 15-30, advantageously about 30-45 nucleotides, and preferably about 45-75, at least 57, 87 or 150 consecutive or contiguous nucleotides of the sequence encoding the total protein or polyprotein. Epitope determination procedures, such as, generating overlapping peptide libraries (Hemmer et al., 1998), Pepscan (Geysen et al., 1984; Geysen et al., 1985; Van der Zee R. et al., 1989; Geysen, 1990; Multipin.RTM. Peptide Synthesis Kits de Chiron) and algorithms (De Groot et al., 1999), and in PCT Application WO 2005/007691, can be used in the practice of the disclosure, without undue experimentation. Other documents cited and incorporated herein may also be consulted for methods for determining epitopes of an immunogen or antigen and thus nucleic acid molecules that encode such epitopes.

A "polynucleotide" is a polymeric form of nucleotides of any length, which contain deoxyribonucleotides, ribonucleotides, and analogs in any combination. Polynucleotides may have three-dimensional structure, and may perform any function, known or unknown. The term "polynucleotide" includes double-, single-stranded, and triple-helical molecules. Unless otherwise specified or required, any embodiment of the disclosure described herein that is a polynucleotide encompasses both the double stranded form and each of two complementary forms known or predicted to make up the double stranded form of either the DNA, RNA or hybrid molecule.

The following are non-limiting examples of polynucleotides: a gene or gene fragment, exons, introns, mRNA, tRNA, rRNA, ribozymes, cDNA, recombinant polynucleotides, branched polynucleotides, plasmids, vectors, isolated DNA of any sequence, isolated RNA of any sequence, nucleic acid probes and primers. A polynucleotide may comprise modified nucleotides, such as methylated nucleotides and nucleotide analogs, uracyl, other sugars and linking groups such as fluororibose and thiolate, and nucleotide branches. The sequence of nucleotides may be further modified after polymerization, such as by conjugation, with a labeling component. Other types of modifications included in this definition are caps, substitution of one or more of the naturally occurring nucleotides with an analog, and introduction of means for attaching the polynucleotide to proteins, metal ions, labeling components, other polynucleotides or solid support. The polynucleotides can be obtained by chemical synthesis or derived from a microorganism.

The invention further comprises a complementary strand to a polynucleotide encoding an influenza antigen, epitope or immunogen. The complementary strand can be polymeric and of any length, and can contain deoxyribonucleotides, ribonucleotides, and analogs in any combination.

The terms "protein", "peptide", "polypeptide" and "polypeptide fragment" are used interchangeably herein to refer to polymers of amino acid residues of any length. The polymer can be linear or branched; it may comprise modified amino acids or-amino acid analogs, and it may-be-interrupted by chemical moieties other than amino acids. The terms also.encompass an amino acid polymer that has been modified naturally or by intervention; for example disulfide bond formation, glycosylation, lipidation, acetylation, phosphorylation, or any other manipulation or modification, such as conjugation with a labeling or bioactive component.

An "isolated" polynucleotide or polypeptide is one that is substantially free of the materials with which it is associated in its native environment. By substantially free, is meant at least 50%, advantageously at least 70%, more advantageously at least 80%, and even more advantageously at least 90% or at least 95% free of these materials.

Hybridization reactions can be performed under conditions of different "stringency." Conditions that increase stringency of a hybridization reaction are well known. See for example, "Molecular Cloning: A Laboratory Manual", second edition (Sambrook et al., 1989). Examples of relevant conditions include (in order of increasing stringency): incubation temperatures of 25°C, 37°C, 50°C, and 68°C; buffer concentrations of 10 x SSC, 6 x SSC, 1 x SSC, 0.1 x SSC (where SSC is 0.15 M NaCl and 15 mM citrate buffer) and their equivalent using other buffer systems; formamide concentrations of 0%, 25%, 50%, and 75%; incubation times from 5 minutes to 24 hours; 1, 2 or more washing steps; wash incubation times of 1, 2, or 15 minutes; and wash solutions of 6 x SSC, 1 x SSC, 0.1 x SSC, or deionized water.

The invention further encompasses polynucleotides encoding functionally equivalent variants and derivatives of the influenza polypeptides and functionally equivalent fragments thereof which may enhance, decrease or not significantly affect properties of the polypeptides encoded thereby. These functionally equivalent variants, derivatives, and fragments display the ability to retain influenza activity. For instance, changes in a DNA sequence that do not change the encoded amino acid sequence, as well as those that result in conservative substitutions of amino acid residues, one or a few amino acid deletions or additions, and substitution of amino acid residues by amino acid analogs are those which will not significantly affect properties of the encoded polypeptide. Conservative amino acid substitutions are glycine/alanine; valine/isoleucine/leucine; asparagine/glutamine; aspartic acid/glutamic acid; serine/threonine/methionine; lysine/arginine; and phenylalanine/tyrosine/tryptophan. In one embodiment, the variants have at least 50%, at least 55%, at least 60%, at least 65%, at least 70%, at least 75%, at least 80%, at least 85%, at least 86%, at least 87%, at least 88%, at least 89%, at least 90%, at least 91%, at least 92%, at least 93%, at least 94%, at least 95%, at least 96%, at least 97%, at least 98% or at least 99% homology or identity to the influenza polynucleotide or polypeptide of interest.

For the purposes of the present invention, sequence identity or homology is determined by comparing the sequences when aligned so as to maximize overlap and identity while minimizing sequence gaps. In particular, sequence identity may be determined using any of a number of mathematical algorithms. A nonlimiting example of a mathematical algorithm used for comparison of two sequences is the algorithm of Karlin et al., 1990 modified as in Karlin et al., 1993.

Another example of a mathematical algorithm used for comparison of sequences is the algorithm of Myers et al., 1988. Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used. Yet another useful algorithm for identifying regions of local sequence similarity and alignment is the FASTA algorithm as described in Pearson et al., 1988.

Advantageous for use according to the present disclosure is the WU-BLAST (Washington University BLAST) version 2.0 software. WU-BLAST version 2.0 executable programs for several UNIX platforms can be downloaded from ftp ://blast.wustl.edu/blast/executables. This program is based on WU-BLAST version 1.4, which in turn is based on the public domain NCBI-BLAST version 1.4 (Altschul et al., 1996; Altschul et al., 1990; Gish et al., 1993; Karlin et al., 1993).

In general, comparison of amino acid sequences is accomplished by aligning an amino acid sequence of a polypeptide of a known structure with the amino acid sequence of a the polypeptide of unknown structure. Amino acids in the sequences are then compared and groups of amino acids that are homologous are grouped together. This method detects conserved regions of the polypeptides and accounts for amino acid insertions and deletions. Homology between amino acid sequences can be determined by using commercially available algorithms (see also the description of homology above). In addition to those otherwise mentioned herein, mention is made too of the programs BLAST, gapped BLAST, BLASTN, BLASTP, and PSI-BLAST, provided by the National Center for Biotechnology Information. These programs are widely used in the art for this purpose and can align homologous regions of two amino acid sequences.

In all search programs in the suite the gapped alignment routines are integral to the database search itself. Gapping can be turned off if desired. The default penalty (Q) for a gap of length one is Q=9 for proteins and BLASTP, and Q=10 for BLASTN, but may be changed to any integer. The default per-residue penalty for extending a gap (R) is R=2 for proteins and BLASTP, and R=10 for BLASTN, but may be changed to any integer. Any combination of values for Q and R can be used in order to align sequences so as to maximize overlap and identity while minimizing sequence gaps. The default amino acid comparison matrix is BLOSUM62, but other amino acid comparison matrices such as PAM can be utilized.

Alternatively or additionally, the term "homology" or "identity", for instance, with respect to a nucleotide or amino acid sequence, can indicate a quantitative measure of homology between two sequences. The percent sequence homology can be calculated as
(N*_{ref} -* N*_{dif}*)* 100/N*_{ref}*, wherein N*_{dif}* is the total number of non-identical residues in the two sequences when aligned and wherein N*_{ref}* is the number of residues in one of the sequences. Hence, the DNA sequence AGTCAGTC will have a sequence identity of 75% with the sequence AATCAATC (N*_{ref}* = 8; N*_{dif}*=2).

Alternatively or additionally, "homology" or "identity" with respect to sequences can refer to the number of positions with identical nucleotides or amino acids divided by the number of nucleotides or amino acids in the shorter of the two sequences wherein alignment of the two sequences can be determined in accordance with the Wilbur and Lipman algorithm (Wilbur et al., 1983), for instance, using a window size of 20 nucleotides, a word length of 4 nucleotides, and a gap penalty of 4, and computer-assisted analysis and interpretation of the sequence data including alignment can be conveniently performed using commercially available programs (e.g., Intelligenetics ™ Suite, Intelligenetics Inc. CA). When RNA sequences are said to be similar, or have a degree of sequence identity or homology with DNA sequences, thymidine (T) in the DNA sequence is considered equal to uracil (U) in the RNA sequence. Thus, RNA sequences are within the scope of the invention and can be derived from DNA sequences, by thymidine (T) in the DNA sequence being considered equal to uracil (U) in RNA sequences.

And, without undue experimentation, the skilled artisan can consult with many other programs or references for determining percent homology.

The invention further encompasses the influenza polynucleotides contained in a vector molecule or an expression vector and operably linked to a promoter element and optionally to an enhancer.

A "vector" refers to a recombinant DNA or RNA plasmid or virus that comprises a heterologous polynucleotide to be delivered to a target cell, either *in vitro* or *in vivo*. The heterologous polynucleotide may comprise a sequence of interest for purposes of prevention or therapy, and may optionally be in the form of an expression cassette. As used herein, a vector needs not be capable of replication in the ultimate target cell or subject. The term includes cloning vectors also included are viral vectors.

The vector of the disclosure is advantageously a poxvirus, particularly a vaccinia virus or an avipox virus, such as fowlpox virus. Advantageously, the virus is a fowlpox virus. An advantageous fowlpox strains may be an attenuated strain. The vector can express at least one epitope from avian strains. Advantageous fowlpox constructs include, but are not limited to, vFP89 and vFP2211. The virus used in the invention is the canarypox virus ALVAC. Advantageous canarypox strains may be an attenuated strain. The disclosed vector can express at least one epitope of an avian strain. An advantageous canarypox construct includes, but is not limited to, vCP2241. Recombinant avipox viruses (see, e.g., U.S. Patent Nos. 5,505,941 and 5,756,103), such as an attenuated recombinant fowlpox virus, for instance TROVAC. In one embodiment of the disclosure, the recombinant TROVAC vaccine described by Karaca et al., 2005, may be used as a feline influenza immunological composition or vaccine. Other viruses that may be used in methods of the disclosure include, but are not limited to, vaccinia viruses, such as an attenuated vaccinia virus, for instance NYVAC, adenoviruses, such as canine adenoviruses (CAV), and herpesviruses, such as canine herpesvirus (CHV) or a feline herpesvirus (FHV).

The term "recombinant" means a polynucleotide semisynthetic, or synthetic origin which either does not occur in nature or is linked to another polynucleotide in an arrangement not found in nature.

"Heterologous" means derived from a genetically distinct entity from the rest of the entity to which it is being compared. For example, a polynucleotide, may be placed by genetic engineering techniques into a plasmid or vector derived from a different source, and is a heterologous polynucleotide. A promoter removed from its native coding sequence and operatively linked to a coding sequence other than the native sequence is a heterologous promoter.

The polynucleotides of the invention may comprise additional sequences, such as additional encoding sequences within the same transcription unit, controlling elements such as promoters, ribosome binding sites, 5'UTR, 3'UTR, transcription terminators, polyadenylation sites, additional transcription units under control of the same or a different promoter, sequences that permit cloning, expression, homologous recombination, and transformation of a host cell, and any such construct as may be desirable to provide embodiments of this invention.

Elements for the expression of an influenza polypeptide, antigen, epitope or immunogen are advantageously present in the vector used in the invention. In minimum manner, this comprises, consists essentially of, or consists of an initiation codon (ATG), a stop codon and a promoter, and optionally also a polyadenylation sequence for certain vectors such as plasmid and certain viral vectors, e.g., viral vectors other than poxviruses. When the polynucleotide encodes a polyprotein fragment, e.g. an influenza peptide, advantageously, in the vector, an ATG is placed at 5' of the reading frame and a stop codon is placed at 3'. Other elements for controlling expression may be present, such as enhancer sequences, stabilizing sequences, such as intron and signal sequences permitting the secretion of the protein.

Methods for making and/or administering a vector or recombinants or plasmid for expression of gene products of genes of the disclosure either *in vivo* or *in vitro* can be any desired method, e.g., a method which is by or analogous to the methods disclosed in, or disclosed in documents cited in: U.S. Patent Nos. 4,603,112; 4,769,330; 4,394,448; 4,722,848; 4,745,051; 4,769,331; 4,945,050; 5,494,807; 5,514,375; 5,744,140; 5,744,141; 5,756,103; 5,762,938; 5,766,599; 5,990,091; 5,174,993; 5,505,941; 5,338,683; 5,494,807; 5,591,639; 5,589,466; 5,677,178; 5,591,439; 5,552,143; 5,580,859; 6,130,066; 6,004,777; 6,130,066; 6,497,883; 6,464,984; 6,451,770; 6,391,314; 6,387,376; 6,376,473; 6,368,603; 6,348,196; 6,306,400; 6,228,846; 6,221,362; 6,217,883; 6,207,166; 6,207,165; 6,159,477; 6,153,199; 6,090,393; 6,074,649; 6,045,803; 6,033,670; 6,485,729; 6,103,526; 6,224,882; 6,312,682; 6,348,450, 6; 312,683, and 6,596,279; EP 0 265 785; WO 90/01543; WO91/11525; WO 94/16716; WO 96/39491; WO 98/33510; EP 265785; EP 0 370 573; Andreansky et al., 1996; Ballay et al., 1993; Felgner et al., 1994; Frolov et al., 1996; Graham, 1990; Grunhaus et al., 1992; Ju et al., 1998; Kitson et al., 1991; McClements et al., 1996; Moss, 1996; Paoletti, 1996; Pennock et al., 1984; Richardson (Ed), 1995; Smith et al., 1983; Robertson et al., 1996; Robinson et al., 1997; and Roizman, 1996. Thus, the vector in the invention can be any suitable recombinant virus or virus vector, such as a poxvirus (e.g., vaccinia virus, avipox virus, canarypox virus, fowlpox virus, raccoonpox virus, swinepox virus, etc.), adenovirus (e.g., human adenovirus, canine adenovirus), herpesvirus (e.g. canine herpesvirus), baculovirus, retrovirus, etc.; or the vector can be a plasmid. The herein cited documents, in addition to providing examples of vectors useful in the practice of the dislcosure, can also provide sources for non-influenza peptides or fragments thereof to be expressed by vector or vectors in, or included in, the compositions of the disclosure.

The present disclosure also relates to preparations comprising vectors, such as expression vectors, e.g., therapeutic compositions. The preparations can comprise, consist essentially of, or consist of one or more vectors, e.g., expression vectors, such as *in vivo* expression vectors, comprising, consisting essentially or consisting of (and advantageously expressing) one or more of influenza polypeptides, antigens, epitopes or immunogens. Advantageously, the vector contains and expresses a polynucleotide that includes, consists essentially of, or consists of a polynucleotide coding for (and advantageously expressing) an influenza antigen, epitope or immunogen, in a pharmaceutically or veterinarily acceptable carrier, excipient or vehicle. Thus, according to an embodiment of the disclosure, the other vector or vectors in the preparation comprises, consists essentially of or consists of a polynucleotide that encodes, and under appropriate circumstances the vector expresses one or more other proteins of an influenza polypeptide, antigen, epitope or immunogen (e.g., hemagglutinin, neuraminidase, nucleoprotein) or a fragment thereof.

According to another embodiment of the disclosure, the vector or vectors in the preparation comprise, or consist essentially of, or consist of polynucleotide(s) encoding one or more proteins or fragment(s) thereof of an influenza polypeptide, antigen, epitope or immunogen, the vector or vectors expressing the polynucleotide(s). The disclosed preparation advantageously comprises, consists essentially of, or consists of, at least two vectors comprising, consisting essentially of, or consisting of, and advantageously also expressing, advantageously *in vivo* under appropriate conditions or suitable conditions or in a suitable host cell, polynucleotides from different feline influenza isolates encoding the same proteins and/or for different proteins, but advantageously the same proteins. Preparations containing one or more vectors containing, consisting essentially of or consisting of polynucleotides encoding, and advantageously expressing, advantageously *in vivo,* an influenza polypeptide, antigen, fusion protein or an epitope thereof. The disclosure is also directed at mixtures of vectors that contain, consist essentially of, or consist of coding for, and express, different influenza polypeptides, antigens, epitopes or immunogens, e.g., an influenza polypeptide, antigen, epitope or immunogen from different species such as, but not limited to, humans, horses, pigs, in addition to avian species including chicken, ducks and geese.

According to one embodiment of the disclosure, the expression vector is a viral vector, in particular an *in vivo* expression vector. In an advantageous embodiment, the expression vector is an adenovirus vector. Advantageously, the adenovirus is a human Ad5 vector, an E1-deleted and/ or an E3-deleted adenovirus.

In one particular embodiment of the disclosure the viral vector is a poxvirus, e.g. a vaccinia virus or an attenuated vaccinia virus, (for instance, MVA, a modified Ankara strain obtained after more than 570 passages of the Ankara vaccine strain on chicken embryo fibroblasts; see Stickl et al., 1971; Sutter et al., 1992; available as ATCC VR-1508; or NYVAC, available under VR-2259, see U.S. Patent No. 5,494,807, for instance, Examples 1 to 6 and *et seq* of U.S. Patent No. 5,494,807 which discuss the construction of NYVAC, as well as variations of NYVAC with additional ORFs deleted from the Copenhagen strain vaccinia virus genome, as well as the insertion of heterologous coding nucleic acid molecules into sites of this recombinant, and also, the use of matched promoters; see also WO96/40241), an avipox virus or an attenuated avipox virus (e.g., canarypox, fowlpox, dovepox, pigeonpox, quailpox, ALVAC, available under VR-2547, or TROVAC; available under VR-2553, see, e.g., U.S. Patent No. 5,505,941, 5,494,807), swinepox, raccoonpox, camelpox, or myxomatosis virus.

According to another embodiment of the disclosure, the poxvirus vector is a fowlpox virus, advantageously an attenuated fowlpox virus. In this regard, attenuated fowlpox viruses are available, such asDIFTOSEC CT strain marketed by MERIAL and the NOBILIS VARIOLE vaccine marketed by INTERVET. Attenuated fowlpox viruses are described in U.S. Patent No. 5,766,599 which pertains to the attenuated fowlpox strain TROVAC. Canarypox viruses are also available from the ATCC under access number VR-111, and are described in U.S. Patent No. 5,756,103 (ALVAC) and WO01/05934.

For information on the method to generate recombinants thereof and how to administer recombinants thereof, the skilled artisan can refer documents cited herein and to WO90/12882, e.g., as to vaccinia virus mention is made of U.S. Patents Nos. 4,769,330, 4,722,848, 4,603,112, 5,110,587, 5,494,807, and 5,762,938 *inter alia;* as to fowlpox, mention is made of U.S. Patents Nos. 5,174,993, 5,505,941 and US-5,766,599 *inter alia;* as to canarypox mentionis made of U.S. Patent No. 5,756,103 *inter alia;* as to swinepox mention is made of U.S. Patent No. 5,382,425 *inter alia;* and, as to raccoonpox, mention is made of WO00/03030 *inter alia.*

When the expression vector is a vaccinia virus, insertion site or sites for the polynucleotide or polynucleotides to be expressed are advantageously at the thymidine kinase (TK) gene or insertion site, the hemagglutinin (HA) gene or insertion site, the region encoding the inclusion body of the A type (ATI); see also documents cited herein, especially those pertaining to vaccinia virus. In the case of fowlpox, advantageously the insertion site or sites are ORFs F7 and/or F8; see also documents cited herein, especially those pertaining to fowlpox virus. In the case of canarypox, advantageously the insertion site or sites are ORF(s) C3, C5 and/or C6; see also documents cited herein, especially those pertaining to canarypox virus. The insertion site or sites for MVA virus area advantageously as in various publications, including Carroll et al., 1997; Stittelaar et al., 2000; Sutter et al., 1994; and, in this regard it is also noted that the complete MVA genome is described in Antoine, 1998, which enables the skilled artisan to use other insertion sites or other promoters.

Advantageously, the polynucleotide to be expressed is inserted under the control of a specific poxvirus promoter, e.g., the vaccinia promoter 7.5 kDa (Cochran et al., 1985), the vaccinia promoter I3L (Riviere et al., 1992), the vaccinia promoter HA (Shida, 1986), the cowpox promoter ATI (Funahashi et al., 1988), the vaccinia promoter H6 (Taylor et al., 1988b; Guo et al., 1989; Perkus et al., 1989), *inter alia.*

In a particular embodiment of the disclosure the viral vector is an adenovirus, such as a human adenovirus (HAV) or a canine adenovirus (CAV).

In one embodiment of the disclosure the viral vector is a human adenovirus, in particular a serotype 5 adenovirus, rendered incompetent for replication by a deletion in the E1 region of the viral genome, in particular from about nucleotide 459 to about nucleotide 3510 by reference to the sequence of the hAd5 disclosed in Genbank under the accession number M73260 and in the referenced publication Chroboczek et al, 1992. The deleted adenovirus is propagated in E1-expressing 293 (Graham et al., 1977) or PER cells, in particular PER.C6 (Falloux et al., 1998). The human adenovirus can be deleted in the E3 region, in particular from about nucleotide 28592 to about nucleotide 30470. The deletion in the E1 region can be done in combination with a deletion in the E3 region (see, e.g. Shriver et al., 2002; Graham et al., 1991; Ilan et al., 1997; U.S. Patent Nos. 6,133,028 and 6,692,956; Tripathy et al., 1994; Tapnell, 1993; Danthinne et al., 2000; Berkner, 1988; Berkner et al., 1983; Chavier et al., 1996). The insertion sites can be the E1 and/or E3 loci (region) eventually after a partial or complete deletion of the E1 and/or E3 regions. Advantageously, when the expression vector is an adenovirus, the polynucleotide to be expressed is inserted under the control of a promoter functional in eukaryotic cells, such as a strong promoter, preferably a cytomegalovirus immediate-early gene promoter (CMV-IE promoter), in particular the enhancer / promoter region from about nucleotide -734 to about nucleotide +7 in Boshart et al., 1985 or the enhancer / promoter region from the pCI vector from Promega Corp. The CMV-IE promoter is advantageously of murine or human origin. The promoter of the elongation factor 1α can also be used. A muscle specific promoter can also be used (Li et al., 1999). Strong promoters are also discussed herein in relation to plasmid vectors. In one embodiment, a splicing sequence can be located downstream of the enhancer / promoter region. For example, the intron 1 isolated from the CMV-IE gene (Stenberg et al., 1984), the intron isolated from the rabbit or human β-globin gene, in particular the intron 2 from the b-globin gene, the intron isolated from the immunoglobulin gene, a splicing sequence from the SV40 early gene or the chimeric intron sequence isolated from the pCI vector from Promege Corp. comprising the human β-globin gene donor sequence fused to the mouse immunoglobulin acceptor sequence (from about nucleotide 890 to about nucleotide 1022 in Genbank under the accession number CVU47120). A poly(A) sequence and terminator sequence can be inserted downstream the polynucleotide to be expressed, e.g. a bovine growth hormone gene, in particular from about nucleotide 2339 to about nucleotide 2550 in Genbank under the accession number BOVGHRH, a rabbit β-globin gene or a SV40 late gene polyadenylation signal.

In another embodiment of the disclosure the viral vector is a canine adenovirus, in particular a CAV-2 (see, e.g. Fischer et al., 2002; U.S. Patent Nos. 5,529,780 and 5,688,920; PCT Application No. WO95/14102). For CAV, the insertion sites can be in the E3 region and /or in the region located between the E4 region and the right ITR region (see U.S. Patent Nos. 6,090,393 and 6,156,567). In one embodiment the insert is under the control of a promoter, such as a cytomegalovirus immediate-early gene promoter (CMV-IE promoter) or a promoter already described for a human adenovirus vector. A poly(A) sequence and terminator sequence can be inserted downstream the polynucleotide to be expressed, e.g. a bovine growth hormone gene or a rabbit β-globin gene polyadenylation signal.

In another particular embodiment of the disclosure the viral vector is a herpesvirus such as a canine herpesvirus (CHV) or a feline herpesvirus (FHV). For CHV, the insertion sites may be in particular in the thymidine kinase gene, in the ORF3, or in the UL43 ORF (see U.S. Patent No. 6,159,477). In one embodiment of the disclosure the polynucleotide to be expressed is inserted under the control of a promoter functional in eukaryotic cells, advantageously a CMV-IE promoter (murine or human).. A poly(A) sequence and terminator sequence can be inserted downstream the polynucleotide to be expressed, e.g. bovine growth hormone or a rabbit β-globin gene polyadenylation signal.

According to a yet further embodiment of the disclosure, the expression vector is a plasmid vector or a DNA plasmid vector, in particular *an in vivo* expression vector. In a specific, non-limiting example, the pVR1020 or 1012 plasmid (VICAL Inc.; Luke et al., 1997; Hartikka et al., 1996, see, e.g., U.S. Patent Nos. 5,846,946 and 6,451,769) can be utilized as a vector for the insertion of a polynucleotide sequence. The pVR1020 plasmid is derived from pVR1012 and contains the human tPA signal sequence. In one embodiment of the disclosure the human tPA signal comprises from amino acid M(1) to amino acid S(23) in Genbank under the accession number HUMTPA14. In another specific, non-limiting example, the plasmid utilized as a vector for the insertion of a polynucleotide sequence can contain the signal peptide sequence of equine IGF1 from amino acid M(24) to amino acid A(48) in Genbank under the accession number U28070. Additional information on DNA plasmids which may be consulted or employed in the practice are found, for example, in U.S. Patent Nos. 6,852,705; 6,818,628; 6,586,412; 6,576,243; 6,558,674; 6,464,984; 6,451,770; 6,376,473 and 6,221,362.

The term plasmid covers any DNA transcription unit comprising a polynucleotide according to the disclosure and the elements necessary for its *in vivo* expression in a cell or cells of the desired host or target; and, in this regard, it is noted that a supercoiled or non-supercoiled, circular plasmid, as well as a linear form, are intended to be within the scope of the invention.

Each plasmid comprises or contains or consists essentially of, in addition to the polynucleotide encoding an influenza antigen, epitope or immunogen, optionally fused with a heterologous peptide sequence, variant, analog or fragment, operably linked to a promoter or under the control of a promoter or dependent upon a promoter. In general, it is advantageous to employ a strong promoter functional in eukaryotic cells. The preferred strong promoter is the immediate early cytomegalovirus promoter (CMV-IE) of human or murine origin, or optionally having another origin such as the rat or guinea pig. The CMV-IE promoter can comprise the actual promoter part, which may or may not be associated with the enhancer part. Reference can be made to EP-A-260 148, EP-A-323 597, U.S. Patents Nos. 5,168,062, 5,385,839, and 4,968,615, as well as to PCT Application No WO87/03905. The CMV-IE promoter is advantageously a human CMV-IE (Boshart et al., 1985) or murine CMV-IE.

In more general terms, the promoter has either a viral or a cellular origin. A strong viral promoter other than CMV-IE that may be usefully employed in the practice of the invention is the early/late promoter of the SV40 virus or the LTR promoter of the Rous sarcoma virus. A strong cellular promoter that may be usefully employed in the practice of the invention is the promoter of a gene of the cytoskeleton, such as e.g. the desmin promoter (Kwissa et al., 2000), or the actin promoter (Miyazaki et al., 1989).

Functional sub fragments of these promoters, i.e., portions of these promoters that maintain an adequate promoting activity, are included within the present invention, e.g. truncated CMV-IE promoters according to PCT Application No. WO98/00166 or U.S. Patent No. 6,156,567 can be used. A promoter consequently includes derivatives and sub fragments of a full-length promoter that maintain an adequate promoting activity and hence function as a promoter, preferably promoting activity substantially similar to that of the actual or full-length promoter from which the derivative or sub fragment is derived, e.g., akin to the activity of the truncated CMV-IE promoters of U.S. Patent No. 6,156,567 to the activity of full-length CMV-IE promoters. Thus, a CMV-IE promoter can comprise or consist essentially of or consist of the promoter portion of the full-length promoter and/or the enhancer portion of the full-length promoter, as well as derivatives and sub fragments.

Preferably, the plasmids comprise or consist essentially of other expression control elements. It is particularly advantageous to incorporate stabilizing sequence(s), e.g., intron sequence(s), preferably the first intron of the hCMV-IE (PCT Application No. WO89/01036), the intron II of the rabbit β-globin gene (van Ooyen et al., 1979).

As to the polyadenylation signal (polyA) for the plasmids and viral vectors other than poxviruses, use can more be made of the poly(A) signal of the bovine growth hormone (bGH) gene (see U.S. Patent No. 5,122,458), or the poly(A) signal of the rabbit β-globin gene or the poly(A) signal of the SV40 virus.

According to_another.embodiment of the disclosure, the expression vectors are expression vectors used for the *in vitro* expression of proteins in an appropriate cell system. The expressed proteins can be harvested in or from the culture supernatant after, or not after secretion (if there is no secretion a cell lysis typically occurs or is performed), optionally concentrated by concentration methods such as ultrafiltration and/or purified by purification means, such as affinity, ion exchange or gel filtration-type chromatography methods.

A "host cell" denotes a prokaryotic or eukaryotic cell that has been genetically altered, or is capable of being genetically altered by administration of an exogenous polynucleotide, such as a recombinant plasmid or vector. When referring to genetically altered cells, the term refers both to the originally altered cell and to the progeny thereof. Advantageous host cells include, but are not limited to, baby hamster kidney (BHK) cells, colon carcinoma (Caco-2) cells, COS7 cells, MCF-7 cells, MCF-10A cells, Madin-Darby canine kidney (MDCK) lines, mink lung (Mv1Lu) cells, MRC-5 cells, U937 cells, CHO cells, and VERO cells. Polynucleotides comprising a desired sequence can be inserted into a suitable cloning or expression vector, and the vector in turn can be introduced into a suitable host cell for replication and amplification. Polynucleotides can be introduced into host cells by any means known in the art. The vectors containing the polynucleotides of interest can be introduced into the host cell by any of a number of appropriate means, including direct uptake, endocytosis, transfection, f-mating, electroporation, transfection employing calcium chloride, rubidium chloride, calcium phosphate, DEAE-dextran, or other substances; microprojectile bombardment; lipofection; and infection (where the vector is infectious, for instance, a retroviral vector). The choice of introducing vectors or polynucleotides will often depend on features of the host cell.

In an embodiment, the disclosure provides for the administration of a therapeutically effective amount of a formulation for the delivery and expression of an influenza antigen, epitope or immunogen in a target cell. Determination of the therapeutically effective amount is routine experimentation for one of ordinary skill in the art. In one embodiment of the disclosure, the formulation comprises an expression vector comprising a polynucleotide that expresses an influenza antigen, epitope or immunogen and a pharmaceutically or veterinarily acceptable carrier, vehicle or excipient. In an embodiment of the disclosure, the pharmaceutically or veterinarily acceptable carrier, vehicle or excipient facilitates transfection and/or improves preservation of the vector or protein.

The pharmaceutically or veterinarily acceptable carriers or vehicles or excipients are well known to the one skilled in the art. For example, a pharmaceutically or veterinarily acceptable carrier or vehicle or excipient can be a 0.9% NaCl (e.g., saline) solution or a phosphate buffer. Other pharmaceutically or veterinarily acceptable carrier or vehicle or excipients that can be used for methods of this invention include, but are not limited to, poly-(L-glutamate) or polyvinylpyrrolidone. The pharmaceutically or veterinarily acceptable carrier or vehicle or excipients may be any compound or combination of compounds facilitating the administration of the vector (or protein expressed from an inventive vector *in vitro);* advantageously, the carrier, vehicle or excipient may facilitate transfection and/or improve preservation of the vector (or protein). Doses and dose volumes are herein discussed in the general description and can also be determined by the skilled artisan from this disclosure read in conjunction with the knowledge in the art, without any undue experimentation.

The cationic lipids containing a quaternary ammonium salt which are advantageously but not exclusively suitable for plasmids, are advantageously those having the following formula: 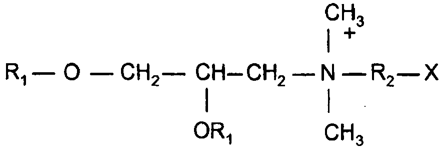 in which R1 is a saturated or unsaturated straight-chain aliphatic radical having 12 to 18 carbon atoms, R2 is another aliphatic radical containing 2 or 3 carbon atoms and X is an amine or hydroxyl group, e.g. the DMRIE. In another embodiment of the disclosure the cationic lipid can be associated with a neutral lipid, e.g. the DOPE.

Among these cationic lipids, preference is given to DMRIE (N-(2-hydroxyethyl)-N,N-dimethyl-2,3-bis(tetradecyloxy)-1-propane ammonium; WO96/34109), advantageously associated with a neutral lipid, advantageously DOPE (dioleoyl-phosphatidyl-ethanol amine; Behr, 1994), to form DMRIE-DOPE.

Advantageously, the plasmid mixture with the adjuvant is formed extemporaneously and advantageously contemporaneously with administration of the preparation or shortly before administration of the preparation; for instance, shortly before or prior to administration, the plasmid-adjuvant mixture is formed, advantageously so as to give enough time prior to administration for the mixture to form a complex, e.g. between about 10 and about 60 minutes prior to administration, such as approximately 30 minutes prior to administration.

When DOPE is present, the DMRIE:DOPE molar ratio is advantageously about 95: about 5 to about 5:about 95, more advantageously about 1: about 1, e.g., 1:1.

The DMRIE or DMRIE-DOPE adjuvant:plasmid weight ratio can be between about 50: about 1 and about 1: about 10, such as about 10: about 1 and about 1:about 5, and advantageously about 1: about 1 and about 1: about 2, e.g., 1:1 and 1:2.

The invention also provides for inactivated feline influenza immunological compositions or vaccines. As used herein, the term "inactivated immunological composition" or "inactivated vaccine" means an immunological composition or vaccine containing an infectious organism or pathogen that is no longer capable of replication or growth. Inactivation may be accomplished by a variety of methods sufficient to prevent replication or growth of the organism while maintaining its immunogenicity.

The inactivated immunological composition or vaccine may be an inactivated form of an isolate of an influenza virus from an affected cat. The virus may be isolated from the alveoli or lung of an affected cat. In another embodiment of the disclosure, the inactivated immunological composition or vaccine may be an inactivated avian influenza. The inactivated immunological composition or vaccine may be an inactivated version of any one of the influenza strains described above.

An inactivated immunological composition or vaccine may be prepared as well from the harvested culture fluid. The virus may be produced either by inoculation of 10-11-day embryonated eggs (U.S. Patent No. 6,048,537) or by inoculation of BHK-21 cell culture (Ross et al., 1970; Tolstova et al., 1966; Merten et al., 1996), of MDCK cell culture (Tree et al., 2001; Ghendon et al., 2005; Brands et al., 1999; Youil et al., 2004), of Vero cell culture (Kistner et al., 1998; Govorkova et al., 1996). The allantoic fluid or the cell culture supernatant can be clarified by low centrifugation and/or filtration. The virus can be concentrated by ultrafiltration and can be purified by zonal centrifugation on sucrose gradient (U.S. Patent No. 6,048,537; O. Kistner et al. idem), by gel filtration (Nayak et al., 2005; Tomita et al., 1971).

Inactivation may be achieved by treating the viruses by any of the methods commonly employed to make inactivated immunological compositions or vaccines. These methods include but are not limited to formaldehyde treatment (O. Kistner et al. idem; Garcia et al., 1998), betapropriolactone treatment (Budowsky et al., 1991; Budowsky et al., 1993; Keverin et al., 2000), ethylene-imine treatment (Swayne et al., 2001), treatment with organic solvents, treatment with detergents, treatment with Tween-ether or treatment with Triton X-100 (J. Vilay et al. idem) for allantoic fluid. For the inactivation the concentration can be about 0.01-0.2 % w/v for the formaldehyde; about 0.03-0.2 % w/v for the betapropiolactone; about 0.5-20 mM for ethyleneimine. The methods recited herein serve as art-known examples for inactivating virus. Inactivated virus immunological compositions or vaccines are usually administered mixed with an adjuvant. The inactivated immunological composition or vaccine can be administered to the animal by any of a plurality of methods which include but are not limited to inoculation intramuscularly or subcutaneously, spraying, ocularly, nasally, orally, or in ovo.

The immunological compositions and vaccines according to the disclosure may comprise or consist essentially of one or more adjuvants. Suitable adjuvants for use in the practice of the present invention are (1) polymers of acrylic or methacrylic acid, maleic anhydride and alkenyl derivative polymers, (2) immunostimulating sequences (ISS), such as oligodeoxyribonucleotide sequences having one ore more non-methylated CpG units (Klinman et al., 1996; WO98/16247), (3) an oil in water emulsion, such as the SPT emulsion described on p 147 of "Vaccine Design, The Subunit and Adjuvant Approach" published by M. Powell, M. Newman, Plenum Press 1995, and the emulsion MF59 described on p 183 of the same work, (4) cation lipids containing a quaternary ammonium salt, e.g., DDA (5) cytokines, (6) aluminum hydroxide or aluminum phosphate, (7) saponin or (8) other adjuvants discussed in any document cited, or (9) any combinations or mixtures thereof.

The oil in water emulsion (3), which is especially appropriate for viral vectors, can be based on: light liquid paraffin oil (European pharmacopoeia type), isoprenoid oil such as squalane, squalene, oil resulting from the oligomerization of alkenes, e.g. isobutene or decene, esters of acids or alcohols having a straight-chain alkyl group, such as vegetable oils, ethyl oleate, propylene glycol, di(caprylate/caprate), glycerol tri(caprylate/caprate) and propylene glycol dioleate, or esters of branched, fatty alcohols or acids, especially isostearic acid esters.

The oil is used in combination with emulsifiers to form an emulsion. The emulsifiers may be nonionic surfactants, such as: esters of on the one hand sorbitan, mannide (e.g. anhydromannitol oleate), glycerol, polyglycerol or propylene glycol and on the other hand oleic, isostearic, ricinoleic or hydroxystearic acids, said esters being optionally ethoxylated, or polyoxypropylene-polyoxyethylene copolymer blocks, such as Pluronic, e.g., L121.

Among the type (1) adjuvant polymers, preference is given to polymers of crosslinked acrylic or methacrylic acid, especially crosslinked by polyalkenyl ethers of sugars or polyalcohols. These compounds are known under the name carbomer (Pharmeuropa, vol. 8, no. 2, June 1996). One skilled in the art can also refer to U.S. Patent No. 2,909,462, which provides such acrylic polymers crosslinked by a polyhydroxyl compound having at least three hydroxyl groups, preferably no more than eight such groups, the hydrogen atoms of at least three hydroxyl groups being replaced by unsaturated, aliphatic radicals having at least two carbon atoms. The preferred radicals are those containing 2 to 4 carbon atoms, e.g. vinyls, allyls and other ethylenically unsaturated groups. The unsaturated radicals can also contain other substituents, such as methyl. Products sold under the name Carbopol (BF Goodrich, Ohio, USA) are especially suitable. They are crosslinked by allyl saccharose or by allyl pentaerythritol. Among them, reference is made to Carbopol 974P, 934P and 971P.

As to the maleic anhydride-alkenyl derivative copolymers, preference is given to EMA (Monsanto), which are straight-chain or crosslinked ethylene-maleic anhydride copolymers and they are, for example, crosslinked by divinyl ether. Reference is also made to J. Fields et al., 1960.

With regard to structure, the acrylic or methacrylic acid polymers and EMA are preferably formed by basic units having the following formula: 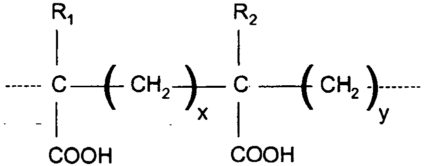 in which:
- R1 and R2, which can be the same or different, represent H or CH3
- x = 0 or 1, preferably x = 1
- y= 1 or 2, with x + y = 2.

For EMA, x = 0 and y = 2 and for carbomers x = y = 1.

These polymers are soluble in water or physiological salt solution (20 g/l NaCl) and the pH can be adjusted to 7.3 to 7.4, e.g., by soda (NaOH), to provide the adjuvant solution in which the expression vector(s) can be incorporated. The polymer concentration in the final immunological or vaccine composition can range between 0.01 and 1.5% w/v, advantageously 0.05 to 1% w/v and preferably 0.1 to 0.4% w/v.

The cytokine or cytokines (5) can be in protein form in the immunological or vaccine composition, or can be co-expressed in the host with the immunogen or immunogens or epitope(s) thereof. Preference is given to the co-expression of the cytokine or cytokines, either by the same vector as that expressing the immunogen or immunogens or epitope(s) thereof, or by a separate vector therefor.

The disclosure comprehends preparing such combination compositions; for instance by admixing the active components, advantageously together and with an adjuvant, carrier, cytokine, and/or diluent.

Cytokines that may be used in the present invention include, but are not limited to, granulocyte colony stimulating factor (G-CSF), granulocyte/macrophage colony stimulating factor (GM-CSF), interferon α (IFNα), interferon β (IFNβ), interferon γ, (IFNγ), interleukin-1α(IL-1α), interleukin-1β (IL-1β), interleukin-2 (IL-2), interleukin-3 (IL-3), interleukin-4 (IL-4), interleukin-5 (IL-5), interleukin-6 (IL-6), interleukin-7 (IL-7), interleukin-8 (IL-8), interleukin-9 (IL-9), interleukin-10 (IL-10), interleukin-11 (IL-11), interleukin-12 (IL-12), tumor necrosis factor α (TNFα), tumor necrosis factor β (TNFβ), and transforming growth factor β (TGFβ). It is understood that cytokines can be co-administered and/or sequentially administered with the immunological or vaccine composition of the present invention. Thus, for instance, the vaccine of the instant disclosure can also contain an exogenous nucleic acid molecule that expresses *in vivo* a suitable cytokine, e.g., a cytokine matched to this host to be vaccinated or in which an immunological response is to be elicited (for instance, a feline cytokine for preparations to be administered to felids).

Advantageously, the immunological composition and/or vaccine according to the invention comprise or consist essentially of or consist of an effective quantity to elicit a therapeutic response of one or more expression vectors and/or polypeptides as discussed herein; and, an effective quantity can be determined from this disclosure, including the documents cited herein, and the knowledge in the art, without undue experimentation.

In the case of immunological composition and/or vaccine based on a plasmid vector, a dose can comprise, consist essentially of or consist of, in general terms, about in 1 µg to about 2000 µg, advantageously about 50 µg to about 1000 µg and more advantageously from about 100 µg to about 800 µg of plasmid expressing the influenza antigen, epitope or immunogen. When immunological composition and/or vaccine based on a plasmid vector is administered with electroporation the dose of plasmid is generally between about 0.1 µg and 1mg, advantageously between about 1µg and 100µg, advantageously between about 2µg and 50µg. The dose volumes can be between about 0.1 and about 2 ml, advantageously between about 0.2 and about 1 ml. These doses and dose volumes are suitable for the treatment of felines.

The immunological composition and/or vaccine contains per dose from about 10⁴ to about 10¹¹, advantageously from about 10⁵ to about 10¹⁰ and more advantageously from about 10⁶ to about 10⁹ viral particles of recombinant adenovirus expressing an influenza antigen, epitope or immunogen. In the case of immunological composition and/or vaccine based on a poxvirus, a dose can be between about 10² pfu and about 10⁹ pfu. The immunological composition and/or vaccine contains per dose from about 10⁵ to 10⁹, advantageously from about 10⁶ to 10⁸ pfu of poxvirus or herpesvirus recombinant expressing the influenza antigen, epitope or immunogen.

The dose volume of compositions for target species that are mammals, e.g., the dose volume of feline compositions, based on viral vectors, e.g., non-poxvirus-viral-vector-based compositions, is generally between about 0.1 to about 2.0 ml, preferably between about 0.1 to about 1.0 ml, and more preferably between about 0.5 ml to about 1.0 ml.

In one embodiment of the disclosure, a prime-boost regimen can be employed, which is comprised of at least one primary administration and at least one booster administration using at least one common polypeptide, antigen, epitope or immunogen. The immunological composition or vaccine used in primary administration is different in nature from those used as a booster. This administration protocol is called "prime-boost". The prime-boost protocol according to the invention comprises a primary administration with an immunological composition or vaccine comprising, in a pharmaceutically acceptable vehicle or excipient, a plasmid containing a polynucleotide sequence for expressing, in vivo, an avian influenza polypeptide, antigen, epitope or immunogen, followed by a booster with an immunological composition or vaccine, or a recombined immunological composition or vaccine comprising, in a pharmaceutically acceptable vehicle or excipient, a viral vector containing a polynucleotide sequence for expressing, in vivo, an avian influenza polypeptide antigen, epitope or immunogen, with the condition according to which at least one of the polypeptides, antigens, epitopes or immunogens is encoded by both the plasmids and the viral vectors. Alternatively, the booster can be comprised of an inactivated immunological composition or vaccine. In another alternative embodiment of the disclosure, the avian influenza polypeptide antigen, epitope or immunogen of the primary administration may derive from a different avian influenza strain than the avian influenza polypeptide antigen, epitope or immunogen of the booster administration

The primary administration may comprise one or more administrations of the same plasmid-based immunological compositions of vaccines. Similarly, the booster administration may comprise one or more administrations of the same viral vector-based immunological composition of vaccine. According to a particular embodiment of the disclosure, the protocol comprises two successive administrations of the same plasmid-based immunological compositions of vaccines, and then one administration of a viral vector-based immunological composition of vaccine, as a booster. Alternatively, the primary administration may be a plasmid-based or viral vector-based immunological composition or vaccine, and the booster administration may be an inactivated immunological composition or vaccine.

The various administrations are preferably carried out 3 to 6 weeks apart, and more particularly about 4 weeks apart. According to a preferred mode, an annual booster, preferably using the viral vector-based immunological composition of vaccine, is also envisaged. The animals are preferably at least 6 to 8 weeks old at the time of the first administration.

With inactivated compositions of the virus or organism or pathogen produced on the new cell culture, the animal may be administered approximately 10⁴-10⁹ equivalent CCID50 (titer before inactivation), advantageously approximately 10⁵-10⁸ equivalent CCID50 in a single dosage unit. The volume of one single dosage unit can be between 0.2 ml and 5.0 ml and advantageously between 0.5 ml and 2.0 ml and more advantageously about 2.0 ml. One or more administrations can be done; e.g. with two injections at 2-4 weeks interval, and advantageously with a boost about 3 weeks after the first inj ection.

In an advantageous embodiment, a felid is vaccinated with two doses of inactivated vaccine at about 3 to 4 week intervals via the subcutaneous route, although an intramuscular route is also contemplated. Blood samples may be collected on the day of the first and/or second vaccination and about 2 to 10 weeks after the second vaccination to determine the levels of anti-influenza virus-specific antibodies by methods known to one of skill in the art, for example, virus neutralization, hemagglutination inhibition, ELISA, or single radial heamolysis (SRH) tests.

The efficacy of the inactivated vaccines may be tested about 2 to 4 weeks after the second immunization by challenging animals, advantageously felids, with a virulent strain of influenza, advantageously the influenza H5N1, H5N8 or H5N9 strains. The animal may be challenged by spray, intra-nasally, intra-tracheally, orally, and/or by contact. The challenge viral may be about 10⁵⁻⁸ EID50 in a volume depending upon the route of administration. For example, if the administration is by spray, a virus suspension is aerosolized to generate about 1 to 100 µm droplets, if the administration is intra-nasal, intra-tracheal or oral, the volume of the challenge virus is about 0.5 ml, 1-2 ml, and 5-10 ml, respectively. Animals may be observed daily for 14 days following challenge for clinical signs, for example, fever, cough, nasal, ocular discharge, respiratory distress, anorexia, and lethargy. In addition, the groups of animals may be euthanized and evaluated for pathological findings of pulmonary and pleural hemorrhage, tracheitis, bronchitis, broncolilitis, and bronchopneumonia. Tracheal swabs may be collected from all animals post challenge days 1-14 for virus isolation. The presence or absence of viral antigens in respiratory tissues may be evaluated by immunohistochemistry, for example, on days 3, 7, and 10 post-challenge. Blood samples may be collected post-challenge (e.g., on days 7 and 14 post-challenge) and may be analyzed for the presence of anti-influenza H5N1 virus-specific antibody.

It should be understood by one of skill in the art that the disclosure herein is provided by way of example and the present invention is not limited thereto. From the disclosure herein and the knowledge in the art, the skilled artisan can determine the number of administrations, the administration route, and the doses to be used for each injection protocol, without any undue experimentation.

The present invention contemplates at least one administration to a felid of an efficient amount of the therapeutic composition made according to the invention. The animal may be male, female, pregnant female and newborn. This administration may be via various routes including, but not limited to, intramuscular (IM), intradermal (ID) or subcutaneous (SC) injection or via intranasal or oral administration. The therapeutic composition according to the invention can also be administered by a needleless apparatus (as, for example with a Pigjet, Dermojet, Biojector, Vetjet or Vitajet apparatus (Bioject, Oregon, USA)). Another approach to administer plasmid compositions is to use electroporation (see, e.g. Tollefsen et al., 2002; Tollefsen et al., 2003; Babiuk et al., 2002; PCT Application No. WO99/01158). In another embodiment, the therapeutic composition is delivered to the felid by gene gun or gold particle bombardment.

One embodiment of the disclosure is a method of eliciting an immune response against avian influenza virus in an animal, comprising administering a formulation for delivery and expression of a recombinant poxvirus influenza immunological composition or vaccine or inactivated influenza immunological composition or vaccine in an effective amount for eliciting an immune response. Still another embodiment of the invention is a method of inducing an immunological or a protective immune response against avian influenza virus in an animal, comprising administering to the animal an effective amount of a formulation for delivery and expression of an influenza antigen, epitope or immunogen wherein the formulation comprises recombinant poxvirus influenza immunological composition or vaccine or inactivated influenza immunological composition or vaccine and a pharmaceutically or veterinarily acceptable carrier, vehicle or excipient.

The disclosure relates to a method to elicit, induce or stimulate the immune response of an animal, advantageously a felid.

In the invention, the immune response elicited to induced is a protective immune response. As is accepted by those of skill in the art, a protective immune response is one that successfully protects a subject from challenge.

Another embodiment of the invention is a kit for performing a method of inducing a protective response against influenza in a felid comprising a recombinant influenza ALVAC immunological composition as defined in claim 11.

The invention will now be further described by way of the following non-limiting examples which further illustrate the invention, and are not intended, nor should they be interpreted to, limit the scope of the invention.

### EXAMPLES

### Example 1: Construction and Development of TROVAC AIV H5 (vFP89)

The construction of vFP89 disclosed herein Example 1 can be found in the following U.S. Patent Nos. 5,494,807, 5,529,780, 5,688,920, 5,756,102, 5,756,103, 5,762,938, 5,766,599, 5,833,975, 5,863,542, 5,942,235, 6,017,542, 6,265,189, 6,309,647, 6,537,594, 6,596,279, and 6,780,407.

### Development of Attenuated Fowlpox Virus

Plasmids containing cDNA clones of the H5 hemagglutinin gene was obtained from Dr. Robert Webster, St. Jude Children's Research Hospital, Memphis, Tenn. The strain of FPV designated FP-1 has been described previously (Taylor et al., 1988a, b). It is a vaccine strain useful in vaccination of day old chickens. The parental virus strain Duvette was obtained in France as a fowlpox scab from a chicken. The virus was attenuated by approximately 50 serial passages in chicken embryonated eggs followed by 25 passages on chick embryo fibroblast (CEF) cells. This virus was obtained in September 1980 by Rhone Merieux, Lyon, France, and a master viral seed established. The virus was received by Virogenetics in September 1989, where it was subjected to four successive plaque purifications. One plaque isolate was further amplified in primary CEF cells and a stock virus, designated as TROVAC, was established. The stock virus used in the in vitro recombination test to produce TROVAC-AIV H5 (vFP89) and TROVAC-AIV H4 (vFP92) had been further amplified though 8 passages in primary CEF cells. The stock virus used to produce TROVAC-AIV H7 (vFP 100) had been further amplified through 12 passages in primary CEF cells.

### Construction of TROVAC Insertion Plasmid at F8 Locus.

Plasmid pRW731.15 contains a 10 kbp PvuII-PvuII fragment cloned from TROVAC genomic DNA. The nucleotide sequence was determined on both strands for a 3659 bp PvuII-EcoRV fragment. This sequence is shown in SEQ ID NO: 1. The limits of an open reading frame designated in this laboratory as F8 were determined within this sequence. The open reading frame is initiated at position 495 and terminates at position 1887. A deletion was made from position 779 to position 1926, as described below. With respect to plasmid pRW731.15, reference is made to U.S. Patent Nos. 5,494,807, 5,529,780, 5,756,102, 5,756,103, 5,766,599, 5,833,975, and 6,596,279.

Plasmid pRW761 is a sub-clone of pRW731.15 containing a 2430 bp EcoRV-EcORV fragment. Plasmid pRW761 was completely digested with XbaI and partially digested with SspI. A 3700 bp XbaI-SspI band was isolated and ligated with the annealed double-stranded oligonucleotides JCA017 (SEQ ID NO: 2) and JCA018 (SEQ ID NO: 3). With respect to plasmid pRW761, reference is made to U.S. Patent Nos. 5,494,807, 5,529,780, 5,756,102, 5,756,103, 5,766,599, 5,833,975, and 6,596,279.

The plasmid resulting from this ligation was designated pJCA002. Plasmid pJCA004 contains a non-pertinent gene linked to the vaccinia virus H6 promoter in plasmid pJCA002. The sequence of the vaccinia virus H6 promoter has been previously described (Taylor et al., 1988a, b; Guo et al., 1989; Perkus et al., 1989). Plasmid pJCA004 was digested with EcoRV and BamHI which deletes the non-pertinent gene and a portion of the 3' end of the H6 promoter. Annealed oligonucleotides RW178 (SEQ ID NO: 4) and RW179 (SEQ ID NO: 5) were cut with EcoRV and BamHI and inserted between the EcoRV and BamHI sites of JCA004 to form pRW846. With respect to plasmids pJCA002, pJCA004, and pRW846, references are made to U.S. Patent Nos. 5,494,807, 5,529,780, 5,756,102, 5,756,103, 5,766,599, 5,833,975, and 6,596,279.

Plasmid pRW846 therefore contains the H6 promoter 5' of EcoRV in the de-ORFed F8 locus. The HincII site 3' of the H6 promoter in pRW846 is followed by translation stop codons, a transcriptional stop sequence recognized by vaccinia virus early promoters (Yuen et al., 1987) and a SmaI site.

### Construction of Insertion Plasmid for H5 Hemagglutinin at the F8 Locus in TROVAC

A cDNA clone of avian influenza H5 derived from A/Turkey/Ireland/1378/83 was received in plasmid pTH29 from Dr. R. Webster. Synthetic oligonucleotides RW10 (SEQ ID NO: 6) through RW 13 (SEQ ID NO: 9) were designed to overlap the translation initiation codon of the previously described vaccinia virus H6 promoter with the ATG of the H5 gene. The sequence continues through the 5' SalI site of the H5 gene and begins again at the 3' H5 DraI site containing the H5 stop codon.

The oligonucleotides were annealed at 95°C for three minutes followed by slow cooling at room temperature. This results in the following double strand structure.

Cloning of oligonucleotides between the EcoRV and PstI sites of pRW742B resulted in pRW744. Plasmid pRW742B contains the vaccinia virus H6 promoter linked to a non-pertinent gene inserted at the HincII site of pRW731.15 described previously. Digestion with PstI and EcoRV eliminates the non-pertinent gene and the 3'-end of the H6 promoter. Plasmid pRW744 now contains the 3' portion of the H6 promoter overlapping the ATG of avian influenza H5. The plasmid also contains the H5 sequence through the 5' SalI site and the 3' sequence from the H5 stop codon (containing a DraI site). Use of the DraI site removes the H5 3' non-coding end. The oligonucleotides add a transcription termination signal recognized by early vaccinia virus RNA polymerase (Yuen et al., 1987). To complete the H6 promoted H5 construct, the H5 coding region was isolated as a 1.6 kpb SalI-DraI fragment from pTH29. Plasmid pRW744 was partially digested with DraI, the linear fragment isolated, recut with SalI and the plasmid now with eight bases deleted between SalI and DraI was used as a vector for the 1.6 kpb pTH29 SalI and DraI fragment. The resulting plasmid pRW759 was cut with EcoRV and DraI. The 1.7 kbp PRW759 EcoRV-DraI fragment containing the 3' H6 promoter and the H5 gene was inserted between the EcoRV and HincII sites of pRW846 (previously described). The resulting plasmid pRW849 contains the H6 promoted avian influenza virus H5 gene in the de-ORFed F8 locus. With respect to plasmids pRW742B, pRW744, pRW759, and pRW849, references are made to U.S. Patent Nos. 5,494,807, 5,529,780, 5,756,102, 5,756,103, 5,766,599, 5,833,975, and 6,596,279.

### Development of TROVAC-Avian Influenza Virus Recombinants.

Insertion plasmids containing the avian influenza virus HA coding sequences were individually transfected into TROVAC infected primary CEF cells by using the calcium phosphate precipitation method previously described (Panicali et al., 1982; Piccini et al., 1987). Positive plaques were selected on the basis of hybridization to HA specific radiolabeled probes and subjected to sequential rounds of plaque purification until a pure population was achieved. One representative plaque was then amplified to produce a stock virus. Plasmid pRW849 was used in an in vitro recombination test to produce recombinant TROVAC-AIV H5 (vFP89) expressing the H5 hemagglutinin.

### Determination of H5 Expression Using Immunofluorescence.

In influenza virus infected cells, the HA molecule is synthesized and glycosylated as a precursor molecule at the rough endoplasmic reticulum. During passage to the plasma membrane it undergoes extensive post-translational modification culminating in proteolytic cleavage into the disulphide linked HA.sub. 1 and HA.sub.2 subunits and insertion into the host cell membrane where it is subsequently incorporated into mature viral envelopes. To determine whether the HA molecules produced in cells infected with the TROVAC-AIV recombinant viruses were expressed on the cell surface, immunofluorescence studies were performed. Indirect immunofluorescence was performed as described (Taylor et al., 1990). Surface expression of the H5 hemagglutinin in TROVAC-AIV H5 was confirmed by indirect immunofluorescence. Expression of the H5 hemagglutinin was detected using a pool of monoclonal antibodies specific for the H5 HA.

### Characterization of H5 Using Immunoprecipitation.

It has been determined that the sequence at and around the cleavage site of the hemagglutinin molecule plays an important role in determining viral virulence since cleavage of the hemagglutinin polypeptide is necessary for virus particles to be infectious. The hemagglutinin protein of the virulent H5 possesses more than one basic amino acid at the carboxy terminus of HA1. It is thought that this allows cellular proteases which recognize a series of basic amino acids to cleave the hemagglutinin and allow the infectious virus to spread both in vitro and in vivo.

In order to determine that the hemagglutinin molecules expressed by the TROVAC recombinant was authentically processed, immunoprecipitation experiments were performed as described (Taylor et al., 1990) using the specific reagents described above.

Immunoprecipitation analysis of the H5 hemagglutinin showed that the glycoprotein is evident as the two cleavage products HA.sub.1 and HA.sub.2 with approximate molecular weights of 44 and 23 kDa, respectively. No such proteins were precipitated from uninfected cells or cells infected with parental TROVAC. Generation of recombinant virus by recombination, in situ hybridization of nitrocellulose filters and screening for B-galactosidase activity are as previously described (Panicali et al., 1982; Perkus et al., 1989).

### Example 2: Construction and Development of TROVAC AIV H5 (vFP2211)

### AIV H5 HA Gene

The nucleotide sequence used in the construction of vFP2211 was derived from AIV A/Chicken/Indonesia/03 H5 HA gene supplied by GeneArt GmbH (Regensburg, Germany). The sequence is synthetic with codon optimization for expression in avian cells and with a modification of HA cleavage site (SEQ ID NO: 15, SEQ ID NO: 16).

### Plasmid Construction of pJY1394.1

To construct the donor plasmid pF8 AIV synthetic H5 HA without cleavage site (pJY1394.1), plasmid pRW744 (see Example 1) was partially digested with DraI. The linear fragment was isolated, recut with SalI, and the plasmid now with 8 bases deleted between SalI and DraI was used as a vector for the insertion of the double stranded SalI and DraI fragment comprising the synthetic AIV H5 HA without cleavage site. The resulting plasmid was cut with EcoRV and DraI, providing a 1.8 kbp EcoRV-DraI fragment containing the 3' H6 promoter and the H5 HA gene. This fragment was inserted between the EcoRV and HincII sites of a donor plasmid based on pRW846 (see Example 1). The resulting plasmid pJY1394.1 contains the vaccinia H6 promoter followed by the synthetic codon-optimized/cleavage site deleted avian influenza virus A/chicken/Indonesia/03 H5 HA gene in the de-ORFed F8 locus. With respect to plasmids pRW744 and pRW846, references are made to U.S. Patent Nos. 5,494,807, 5,529,780, 5,756,102, 5,756,103, 5,766,599, 5,833,975, and 6,596,279.

### Generation of TROVAC AIV H5 Recombinants vFP2211

To generate vFP2211, plasmid pJY1394.1, which contained the synthetic A/chicken/Indonesia/03 H5 HA gene, was linearized with NotI restriction enzyme. The linearized fragments were individually transfected into TROVAC-infected primary CEF cells by using the calcium phosphate precipitation method described previously (Panicali et al. 1982; Piccini et al. 1987). After 42 h, the transfected infected cells were harvested, sonicated and used for recombinant virus screening.

Recombinant plaques were screened based on the plaque lift hybridization method using an AIV-specific probe which was labeled with horse radish peroxidase according to the manufacturer's protocol (Amersham Cat# RPN-3000). After four sequential rounds of plaque purification, the recombinants, designated as vFP2211 and vFP2211, were generated. vFP2211 was confirmed by hybridization as 100% positive for the AIV insert and 100% negative for the F8 ORF.

The vFP2211 recombinants were expanded and concentrated to produce virus stock

### Analysis of Recombinant vFP2211

To re-determine genetic purity, the stock of vFP2211 was re-confirmed by hybridization as 100 % positive for the AIV probe and 100 % negative for the F8 ORF. The stock of vFP2211 was re-confirmed by hybridization but found to be contaminated with the parental virus.

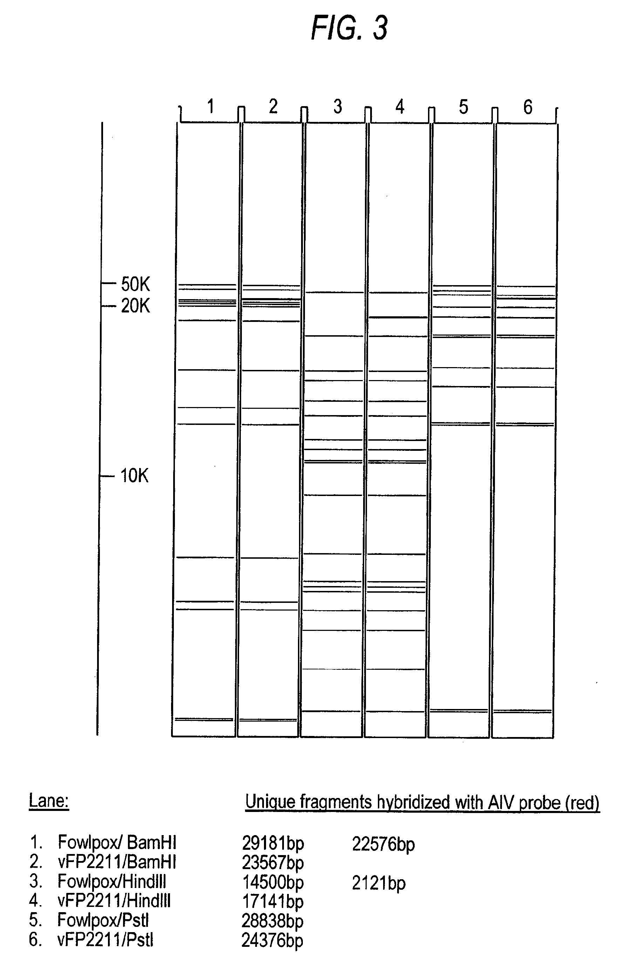

A theoretical restriction enzyme gel for the genomic DNA was created in Vector NTI and is shown in Fig. 3.

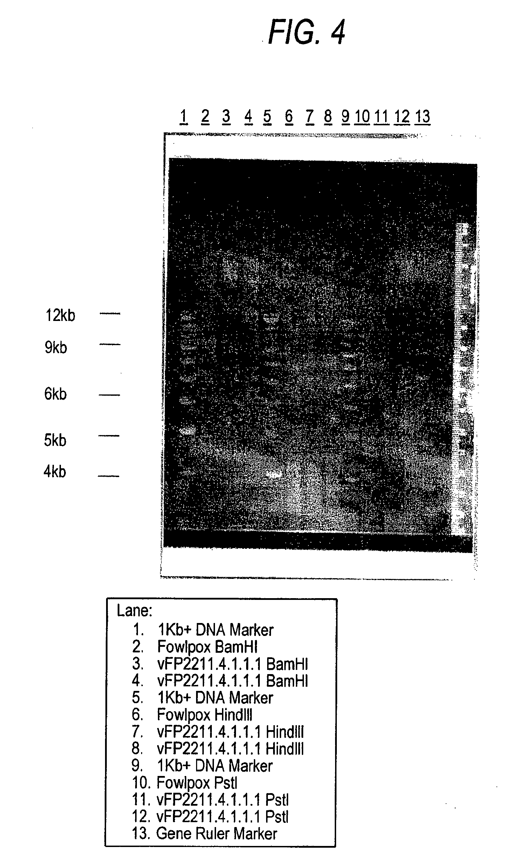

Genomic DNA was extracted and digested with BamHI, HindIII and PstI. The genomic DNA was transferred to nylon membrane and analyses were performed by probing with an AIV probe (see Fig. 4). Bands were observed at the expected sizes, indicating the correct insertion of AIV into the F8 locus (see table 1)

**Table 1. Size of the bands generated by digestion of genomic DNA with restriction enzymes.**

| **Restriction Enzymes** | **# of bp** |
|---|---|
| BamHI | 23567 |
| HindIII | 17141 |
| PstI | 24376 |

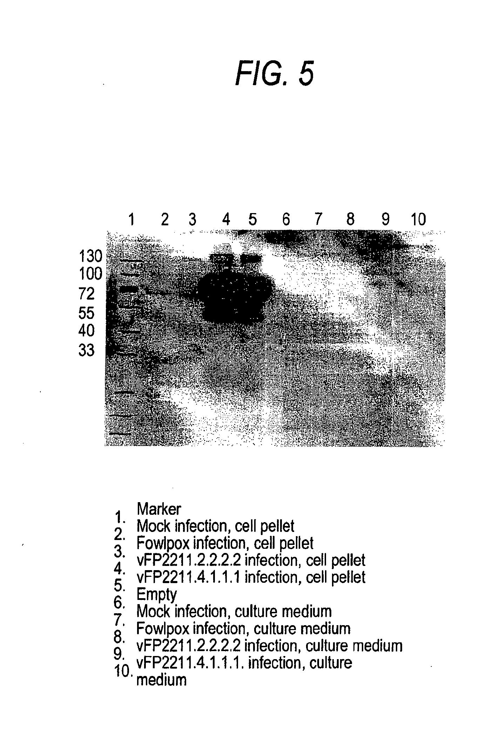

Expression of the recombinant was examined through Western Blot Analysis. Primary CEF cells were infected with vFP2211 at MOI of 10 and incubated for 24 hours. The supernatant was harvested and clarified, and the cells were harvested and suspended in water to lyse. Lysate and supernatant were separated by 10 % SDS-PAGE. The protein was transferred to nylon membrane and then incubated with HA-specific chicken polyserum TK/W1/68. The AIV-specific proteins were visualized using the Amersham ECL chemiluminescence detection system. The results indicated that the vFP2211 recombinants express the AIV HA genes in the cell lysates (see Fig. 5). There is no HA protein secretion into the supernatant.

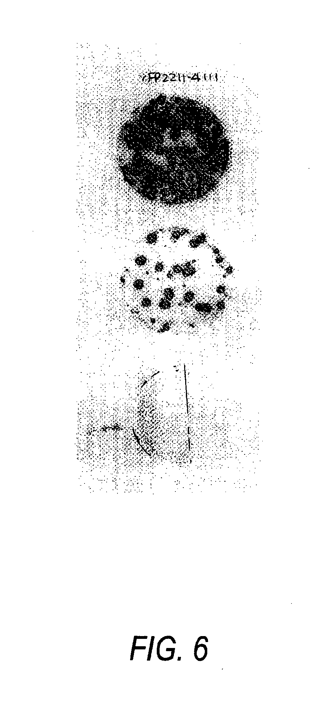

In addition, an immunoplaque assay of stock using HA-specific chicken polyclonal antiserum TK/W1/68 reveled that the homogeneity of the vFP2211 population was 100 % (see Fig. 6)

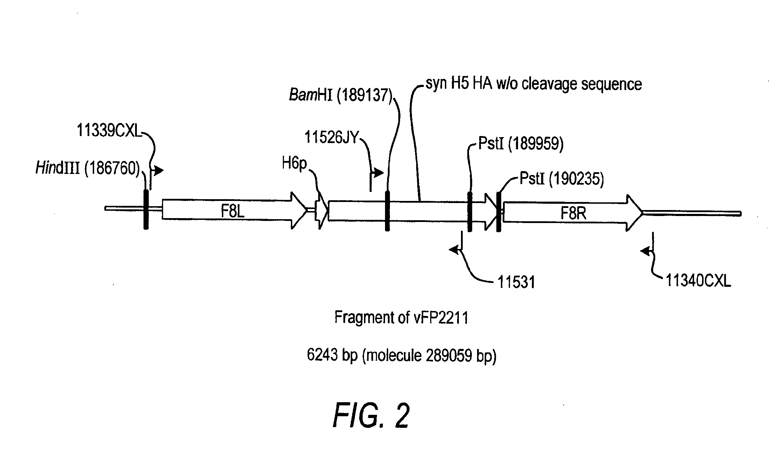

Furthermore, a more detailed analysis of the P2 stock genomic DNA was performed by PCR amplification and sequence analysis of the flanking arms of the F8 locus and the H6p AIV synthetic H5 HA cleavage mutant insert. Primers 11339 (SEQ ID NO: 12) and 11340(SEQ ID NO: 13), located beyond the arms of the F8 locus in the donor plasmid, were used to amplify the entire F8L-insert-F8R fragment (SEQ ID NO: 14; see Fig. 2). The results showed that the sequences of the H6p AIV synthetic H5 HA cleavage mutant insert and the F8 left and right arms in vFP2211 were correct.

### Example 3: Construction and Development of ALVAC AIV H5 (vCP2241)

### AIV H5 HA Gene

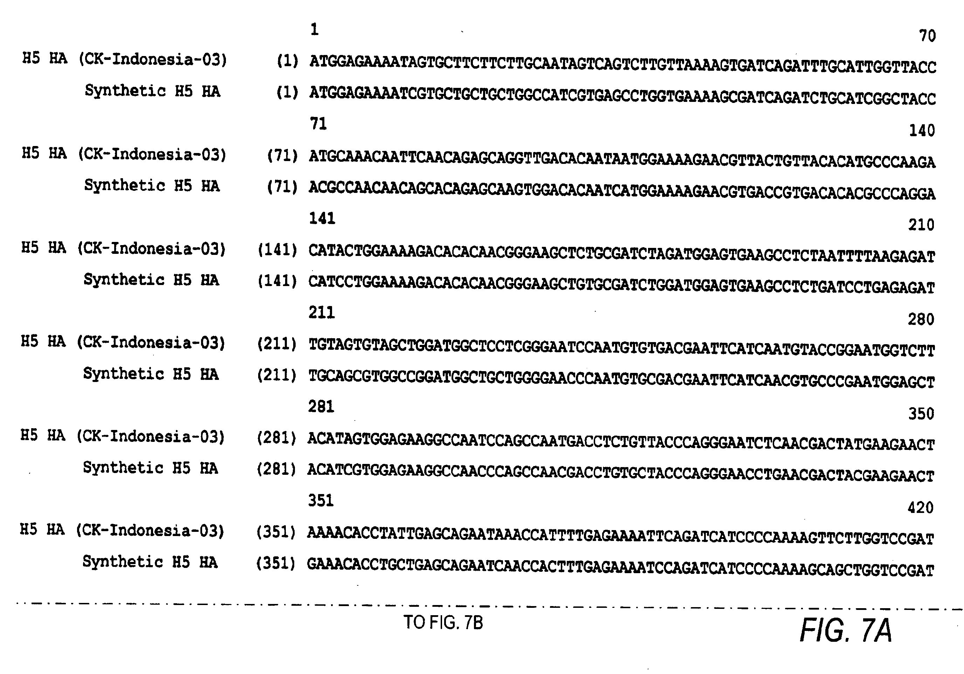

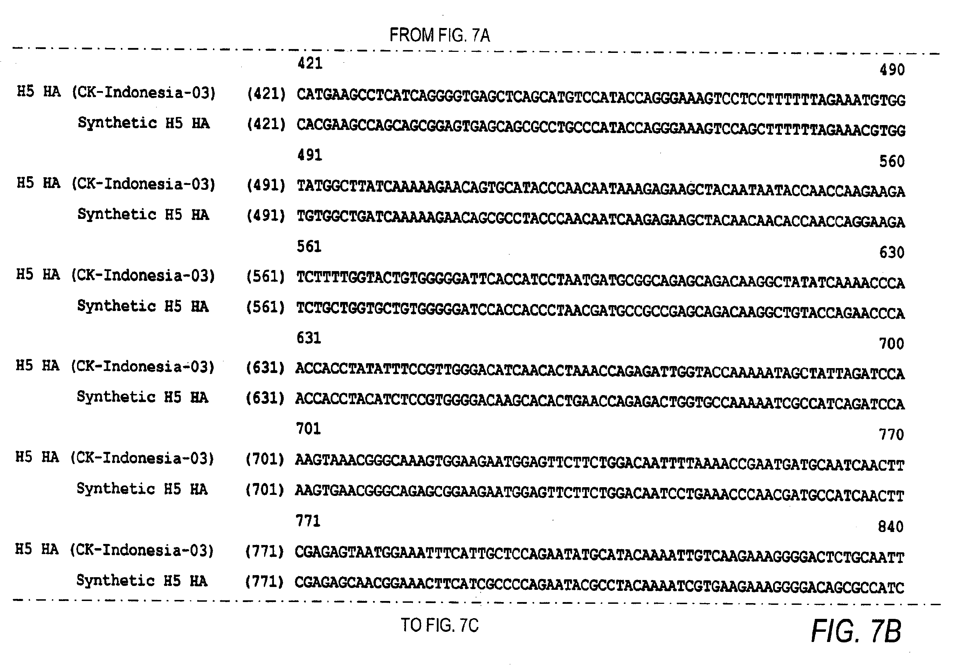

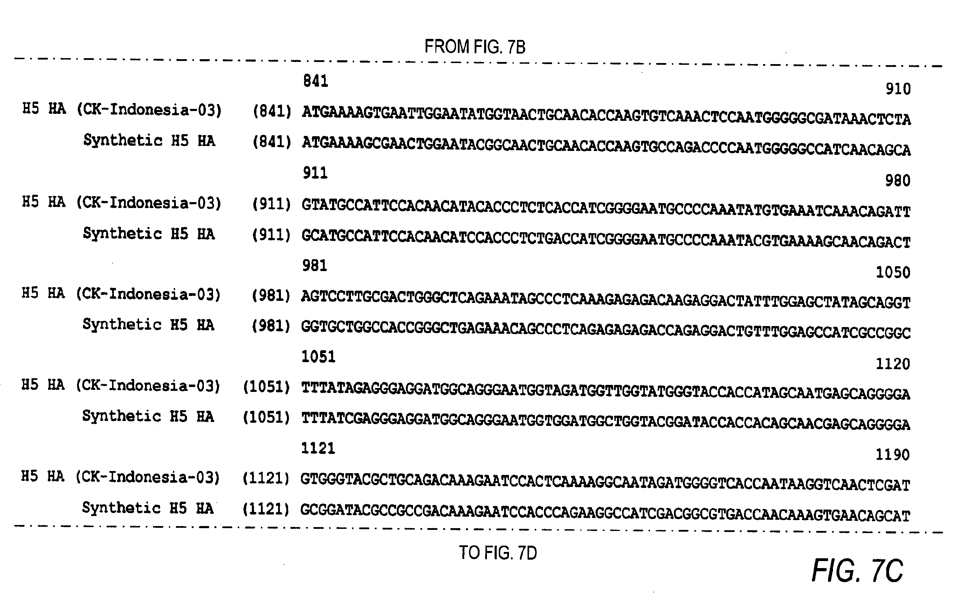

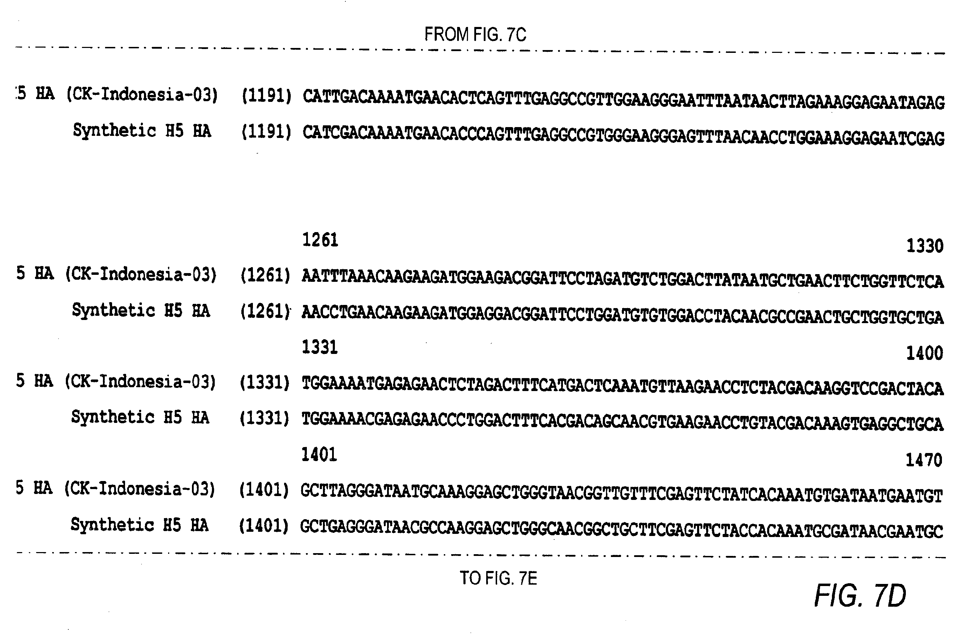

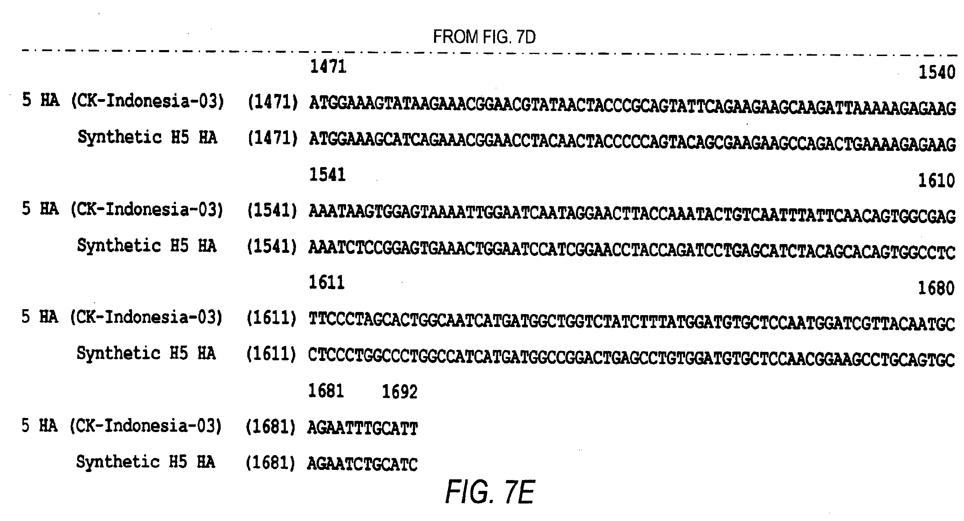

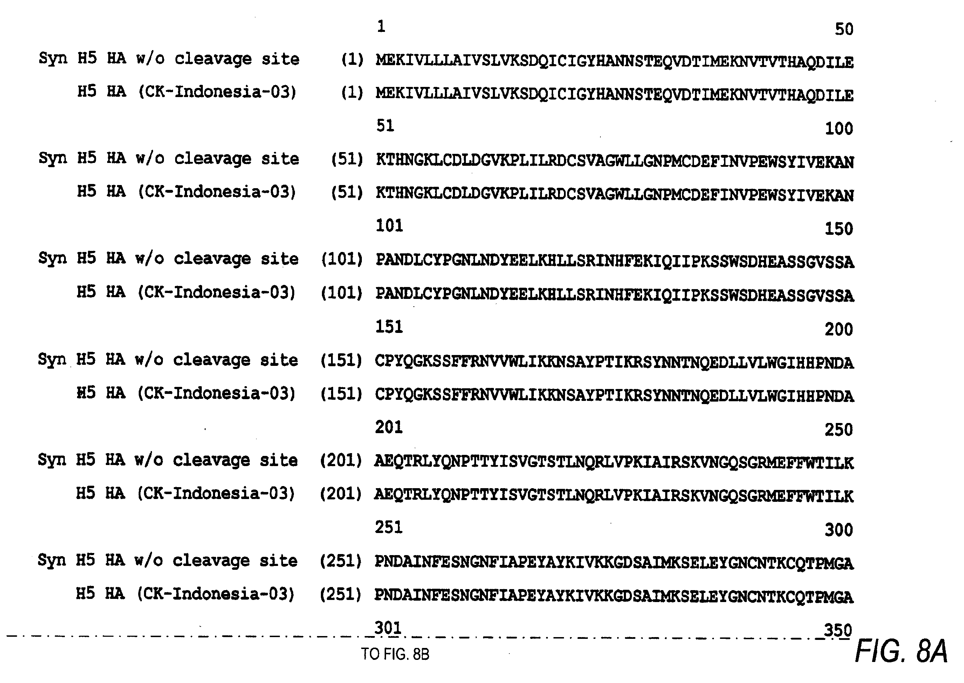

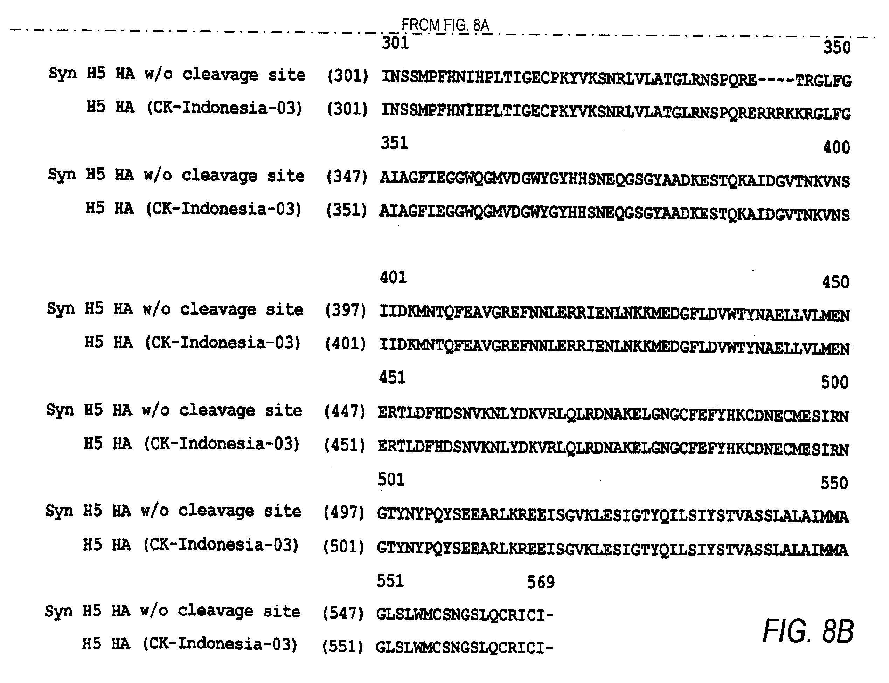

The nucleotide sequence used in the construction of vFP2241 was derived from AIV A/Chicken/Indonesia/03 H5 HA gene supplied by GeneArt GmbH (Regensburg, Germany). The sequence is contained in plasmid pCR-Script/HA-CK/Indonesia/03-(modified)-avipox and is synthetic with codon optimization for expression in avian cells and with a modification of HA cleavage site (SEQ ID NO: 15, SEQ ID NO: 16). Comparison between the synthetic H5 HA without cleavage site (from plasmid pCR-Script/HA-CK/Indonesia/03-(modified)-avipox) and wild type H5 HA without cleavage site (Ck/Indonesia/2003) indicate that the nucleotide and amino acid sequences were very similar (see Figs. 7, 8)

### Plasmid Construction of pLH1852.5

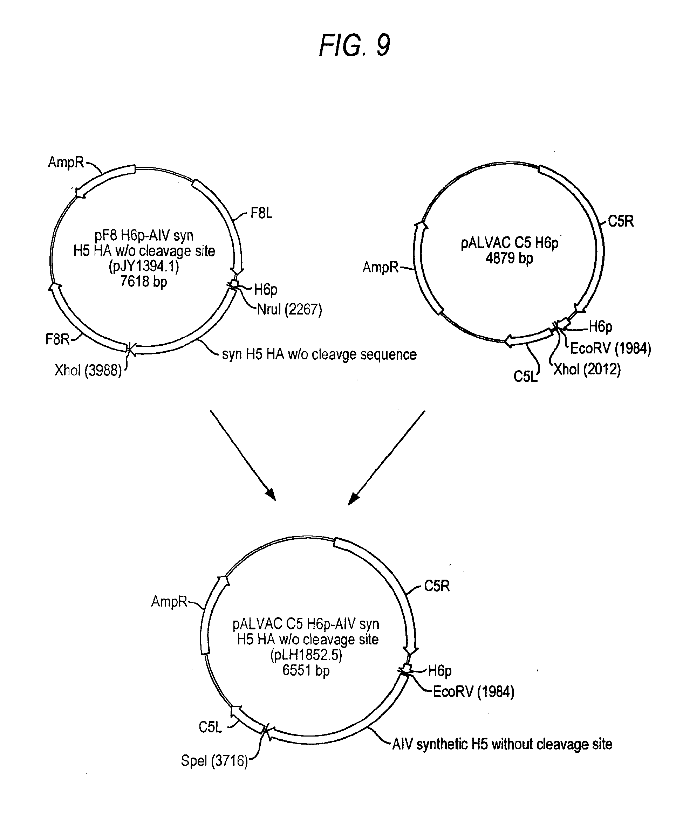

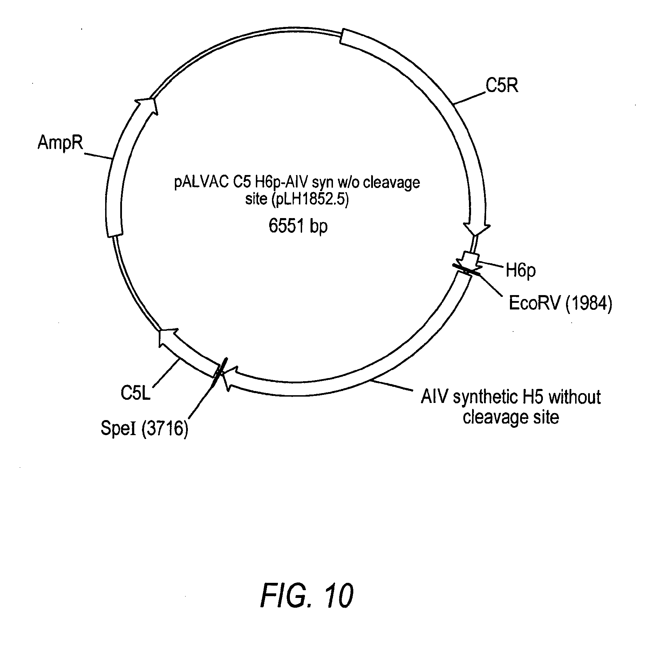

To construct the ALVAC plasmid pALVAC C5 H6p-AIV synthetic H5 HA without cleavage site (pLH1852.5), the plasmid pJY1394.1 (see-Example 2) was digested using EcoRV/Spel digestion in order to isolate the expression cassette comprised of the H6 promoter and synthetic AIV H5 HA gene. This fragment was then ligated to Eco DNA digested pALVAC C5 H6p donor (pCXL148.2) (see Fig. 9). The resulting plasmid pLH1852.5 (Fig. 10) was confirmed to contain the correct nucleotide (SEQ ID NO: 18) and amino amino acid (SEQ ID NO: 17) sequences.

### Generation of ALVAC AIV H5 Recombinants vCP2241

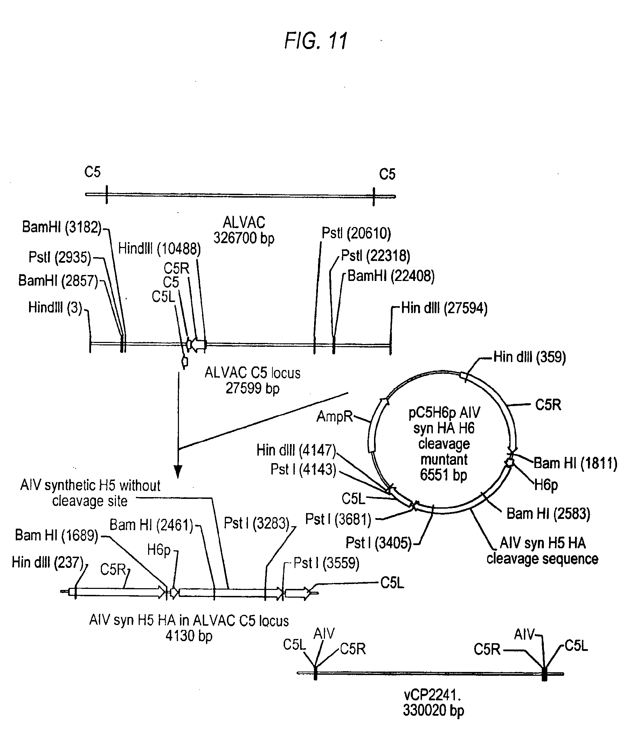

To generate vCP2241, plasmid pLH1852.5, which contained the synthetic A/Chicken/Indonesia/03 H5 HA gene, was linearized with NotI restriction enzyme. The linearized fragments were individually transfected into ALVAC-infected primary CEF cells by using the calcium phosphate precipitation method described previously (Panicali et al. 1982; Piccini et al. 1987) (Fig. 11). After 24h, the transfected-infected cells were harvested, sonicated and used for recombinant virus screening.

Recombinant plaques were screened based on the plaque lift hybridization method using an AIV synthetic H5-specific probe which was labeled with horse radish peroxidase according to the manufacturer's protocol (Amersham Cat# RPN-3001). After four sequential rounds of plaque purification, the recombinants, designated as vCP2241 was generated and confirmed by hybridization as 100 % positive for the AIV synthetic H5 insert and 100 % negative for the C5 ORF.

A single plaque was selected from the final round of plaque purification and expanded to obtain stocks to amplify vCP2241. The recombinant was re-confirmed by hybridization and was again found to be 100 % positive for the AIV synthetic H5 insert and 100 % negative for the C5 ORF. The infected cell culture fluid from the roller bottles was harvested and concentrated to produce virus stock.

### Analysis of Recombinant vCP2241

To re-determine genetic purity, vCP2241 was re-confirmed by hybridization as 100% positive for the AIV probe and 100% negative for the C5 ORF.

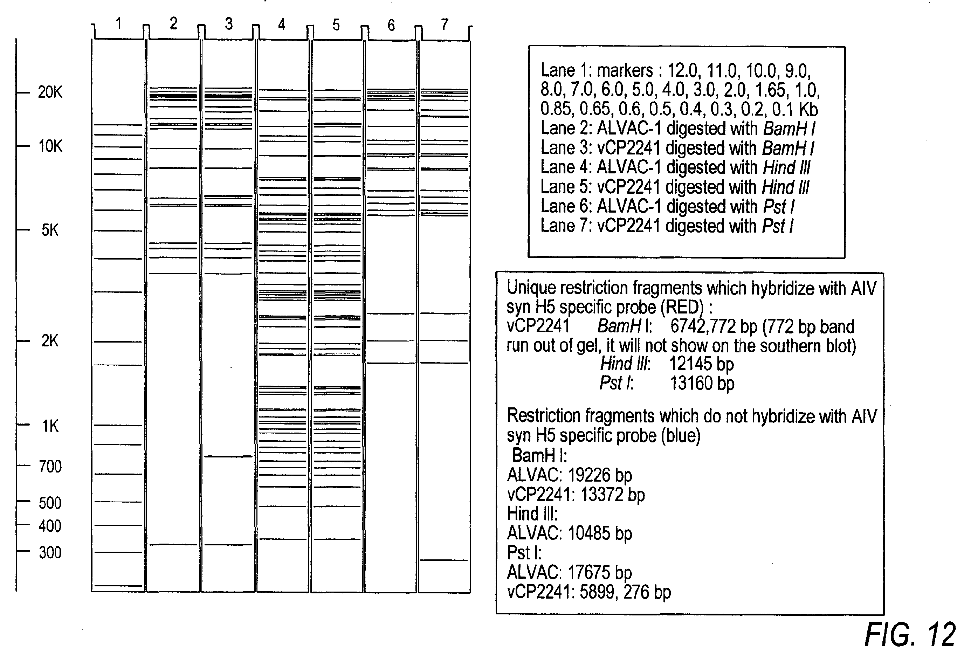

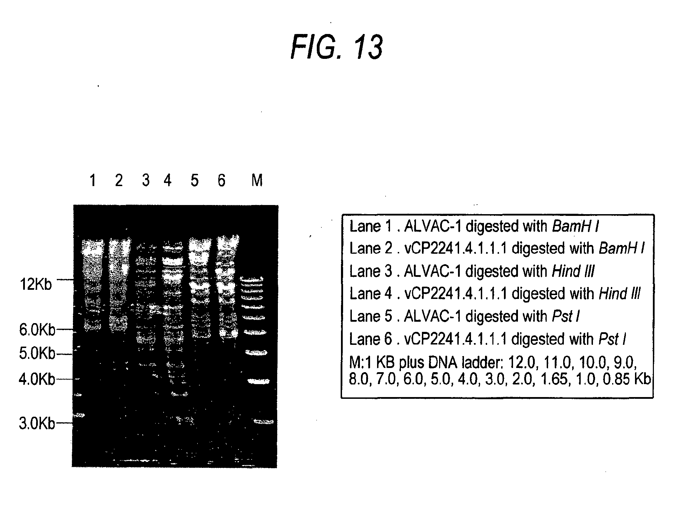

A theoretical restriction enzyme gel for the genomic DNA was created in Vector NTI and is shown in Fig. 12. Genomic DNA was extracted from vCP2241 and digested with BamHI, HindIII and PstI, and separated by 0.8 % agarose gel electrophoresis. The results revealed the correct insertion of the foreign gene sequence (see Fig. 13).

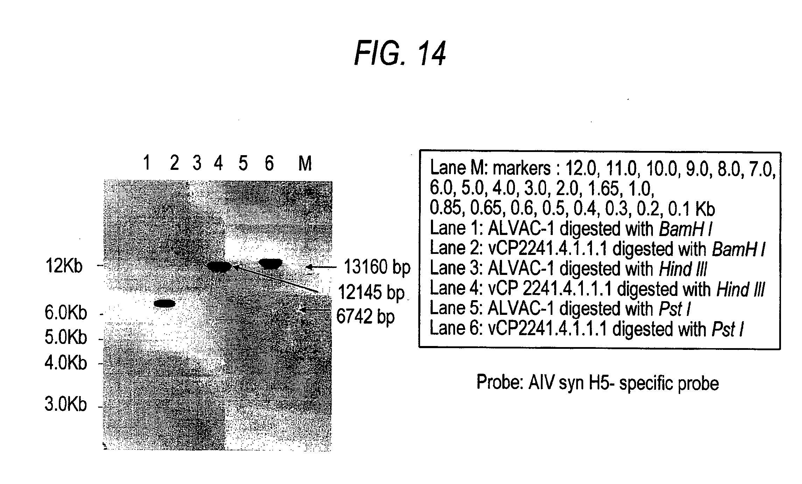

The genomic DNA digested with BamHI, HindIII and PstI was transferred to nylon membrane and Southern blot analysis was performed by probing with AIV synthetic H5 probe. Bands were observed at the expected sizes, indicating the correct insertion of AIV synthetic H5 into the C5 locus (Fig. 14)

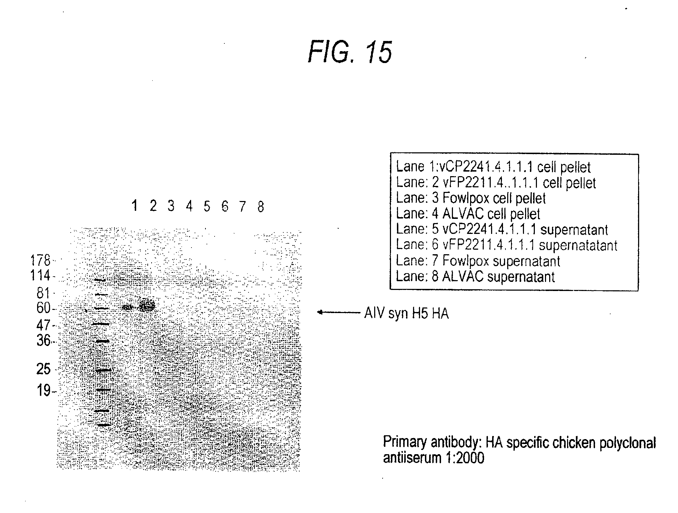

Expression of the recombinant was examined through Western Blot Analysis. Primary CEF cells were infected vCP2241 at MOI of 10 and incubated at 37°C for 24 hours. The cells and culture supernatant were then harvested. Sample proteins were separated on a 10 % SDS-PAGE gel, transferred to Immobilon nylon membrane, and probed with HA specific chicken polyclonal antiserum (TK/WI/68) at 1:2000. Peroxidase conjugated donkey anti-chicken antiserum was used as a secondary antibody and the bands were visualized using Amersham detection reagents. One protein band was detected in the cell pellets of vCP2241 and vFP2211, indicating the expression of the hemagglutinin protein. The expressed protein did not secrete into the cell culture (Fig. 15)

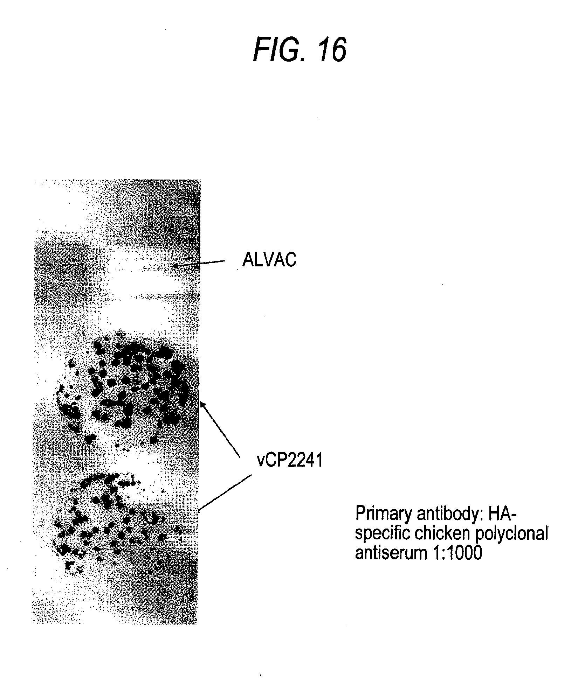

The homogeneity of the vCP2241 population was 100 % as evidenced by an immunoplaque assay, using HA-specific chicken polyclonal antiserum TK/WI/68 (Fig. 16).

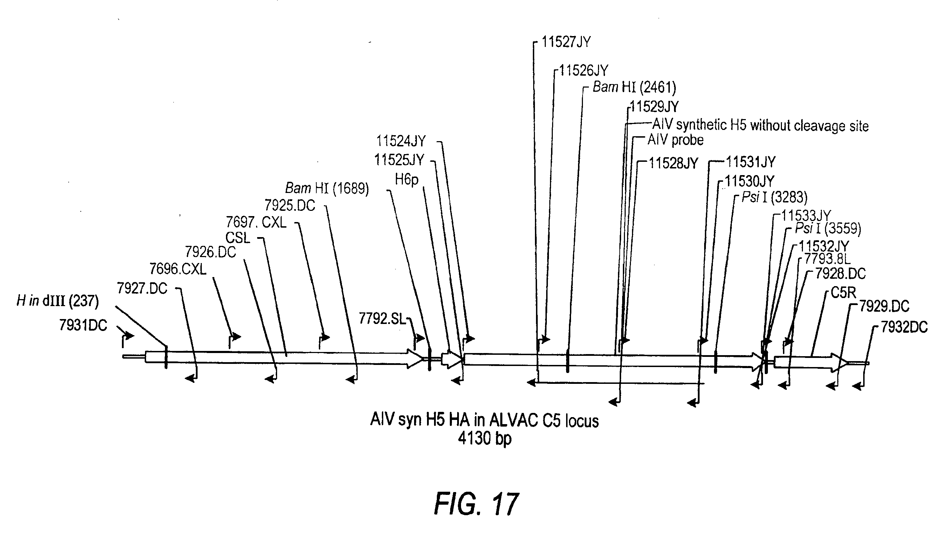

A more detailed analysis of the P3 stock genomic DNA was performed by PCR amplification and sequence analysis of the flanking arms of the C5 locus and the AIV synthetic H5 insert. Primers 7931.DC (SEQ ID NO. 20).and 7932.DC (SEQ ID NO: 21) located beyond the arms of the C5 locus (SEQ ID NO. 22, Fig. 17), were used to amplify the entire C5R- AIV synthetic H5 insert-C5L fragment. The results showed that the sequence of the AIV synthetic H5 insert and the C5 left and right arms around the AIV synthetic H5 insert in vCP2241 was correct.

### Example 4: Vaccination of Cats with vFP89 (TROVAC AIV-H5)

### Methods and Materials

A study was conducted in which 20 cats, aged 16-18 weeks-old, were randomly divided into 2 groups. The first group was immunized with vFP89 (TROVAC fowlpox virus expressing H5 genes from the avian influenza strain A/Turkey/Ireland/1378/83), while the second groups served as a control. On day 0 and day 29, the vaccinated cats were subcutaneously administered 6.8 log10 of 50 % cell culture infective dose, per dose (CCID50/dose).

Blood samples were collected from all cats 3 days prior to the first vaccination, and on days 7, 14, 21, 29, 35, and 42 after the first vaccination. A hemagglutination inhibition (HI) test was performed as described previously (Swayne et al., 1997). Briefly, chicken red blood cell-(cRBC)-treated cat serum was serially diluted and then incubated in wells with 4 HA units of homologous (H5N8 AIV A/Turkey/Ireland/1378/83) and heterologous (H5N1 AIV A/Chicken/Indonesia/03) H5 AIV antigens. A 0.5 % (vol/vol) suspension of cRBCs were allotted per well. Antibodies titers corresponding to the reciprocal of the highest dilution that inhibited hemagglutination were presented as geometric mean titers (GMTs).

### Results

Vaccination did not induce systemic or local adverse reactions. Antibodies to homologous AIV antigen were first detected in 8 of 10 cats at 1 week, and all cats thereafter. The GMTs at days 14, 21, and 29 were 91, 97, and 79, respectively. After the second dose on day 29, the GMTs on day 35 and 42 were 446.

Antibodies to heterologous AIV antigen were detectable after the second vaccination on days 35 and 42, in which the GMTs were 34 and 39, respectively.

Together, these results indicate that the vaccines induced high levels of antibodies to H5 avian influenza virus.

### Example 5: Vaccination of Cats with vFP89 (TROVAC AIV-H5), vFP2211 (TROVAC AIV H5) and vCP2241 (ALVAC AIV H5)

### Methods and Materials

A study was conducted in which 24 cats, aged 16-18 weeks old, were randomly divided into 4 groups. Three groups were immunized with either vFP89 (TROVAC fowlpox cvirus expressing H5 gene from the avian influenza strain A/Turkey/Ireland/1378/83), vFP2211 (TROVAC fowlpox virus expressing H5 gene from the avian influenza strain A/Chicken/Indonesia/03, or vCP2241 (ALVAC canarypox virus expressing H5 gene from the avian influenza strain A/Chicken/Indonesia/03). The fourth group was unvaccinated and served as a control. On day 0 and day 21, the vaccinated cats received subcutaneous injections in the interscapula area of approximately 7.2 log 10 of 50 % cell culture infective dose, per dose (CCID50/dose).

The vaccines were tirated, as shown in Table 2.

**Table 2. Injection titers at Day 0 and Day 21 for vFP89, vFP2211, and vCP2241.**

| **Group** | **Vectors** | **Injection titer at D0 (log10 CCID₅₀/dose)** | **Injection titer at D21 (log10 CCID₅₀/dose)** |
|---|---|---|---|
| vFP89 | TROVAC AIV H5 vector A/Turkev/Ireland/1378/83 | 7.0 | 6.7 |
| vFP2211 | TROVAC AIV H5 vector A/chicken/Indonesia/03 | 7.4 | 7.1 |
| vCP2241 | ALVAC AIV H5 vector A/chicken/Indonesia/03 | 6.4 | 6.7 |

Individual clinical examinations, including temperature records, were conducted daily on all cats for 3 days after each vaccination, i.e., days 1 to 3 and days 22 to 25. The cats were observed for presence of local reaction at the injection point (e.g., pain, oedema, pruritis, and heat).

Blood samples were collected for serology on days 0, 15, 21, and 35 for all groups, and additionally on day 84 for the vaccinated groups. A hemagglutination inhibition (HI) test was performed as described in Example 4. For this study, the homologous AIV antigen was H5N8 AIV A/Turkey/Ireland/1378/83, and the heterologous antigen was H5N1 AIV A/Vietnam/1194/04 (NIBRG14 strain). A 0.5 % (vol/vol) suspension of cRBCs were allotted per well. The results are presented as log 10 of the highest dilution of cat serum that inhibited hemaggluntination.

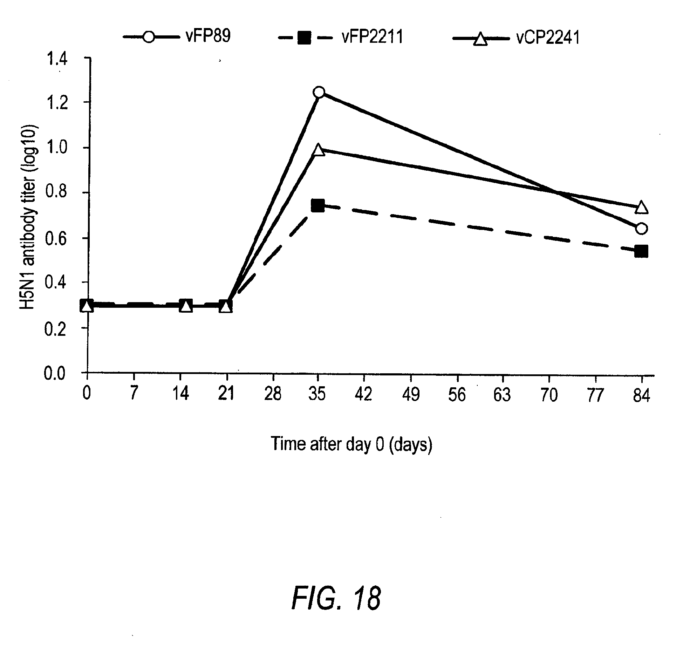

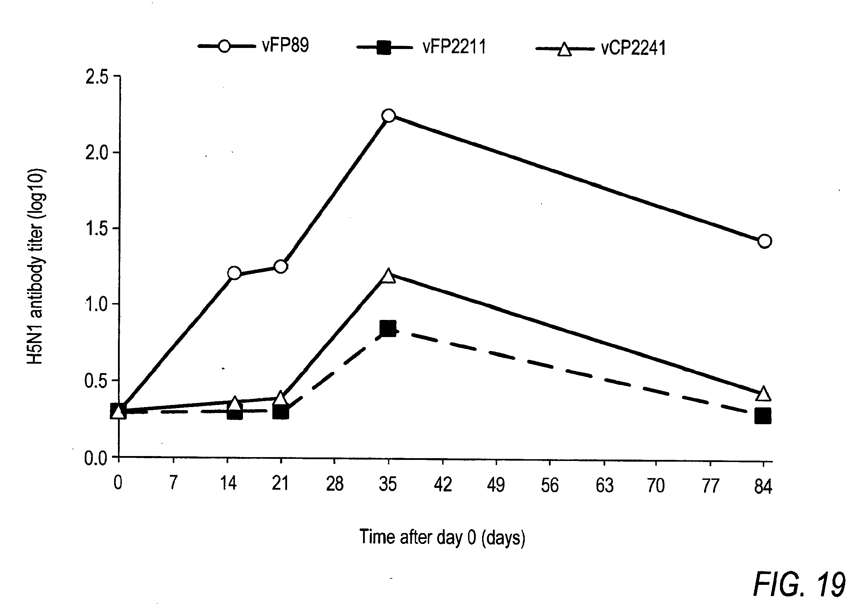

Injection with the recombinant vaccines induced high levels of antibodies to H5 avian influenza virus. Among the vaccinated groups, cats injected with the vFP89 plasmid displayed higher HI responses against both the homologous and heterologus antigens (see Figures 18 and 19). In addition, vCP2241 showed greater HI responses than vFP2211 against both types of antigens. Nonetheless, all three vaccines induced an immunogenic response in cats.

### Example 6: Vaccination of Cats with vCP2241 (ALVAC AIV H5) and vFP89 (TROVAC AIV H5) with AIV H5 Challenge

### Challenge with AIV strain A/Indonesia/5/2005

### Methods and Materials

Eighteen cats, aged 7 months old, were divided into 3 groups with 3 males and 3 females in each group. Two groups were subcutaneously vaccinated on day 0 and day 21 with either vCP2241 (ALVAC canarypox virus expressing H5 gene from the AIV strain A/Chicken/Indonesia/03, H5N1) at a dose of 6.8 log₁₀ CCID50, or vFP89 (TROVAC fowlpox virus expressing H5 gene from the AIV strain A/Turkey/Ireland/1378/83, H5N8) at a dose of 7.4 log₁₀ CCID50. The third group served as challenge controls and received no vaccination. All animals were challenged on day 69 by intratracheal infection with 3 mL containing 1.0 x 10⁵ TCID₅₀ of AIV strain A/Indonesia/5/2005 (H5N1). The day the challenge was administered is hereinafter referred to as day 0.

Individual clinical examinations, including animal weight and body temperature recordings, were conducted on all animals.

Blood was collected from the jugular vein on day 0 prior to the challenge and on days 6 and 14 after challenge. Serum was extracted and antibody titers to AIV strain A/Vietnam/1194/2004 and A/Indonesia/5/05 were determined by hemagglutination inhibition (HI) assay.

Pharyngeal, nasal, and rectal swabs were collected from all animals on day 0 prior to challenge and on days 2, 4, 6, 8, 10, 12, and 14 after the challenge. Levels of viral RNA were determined using a H5N1-specific TaqMan PCR, and levels of infectious virus were determined using a virus titration assay on Madine Darby canine kidney (MDCK) cells. The PCR results were expressed as control dilution units (CDU), which were determined from a standard curve produced from a stock of virus, serially diluted; each dilution underwent nucleic acid extraction and TaqMan PCR amplification in the same manner as test samples. The virus titer results were expressed as TCID₅₀.

On day 14, all surviving animals were euthanized by exsanguination under anaesthesia. A complete macroscopic post-mortem examination was performed and all abnormalities were recorded. The lung lobes were inspected and the cranioventral, craniodorsal, caudoventral, and caudodorsal sections of the right lungs were pooled and homogenized for virus titration analysis. Levels of viral RNA were determined using a H5N1-specific TaqMan PCR, and levels of infectious virus were determined using a virus titration assay on MDCK cells.

### Results

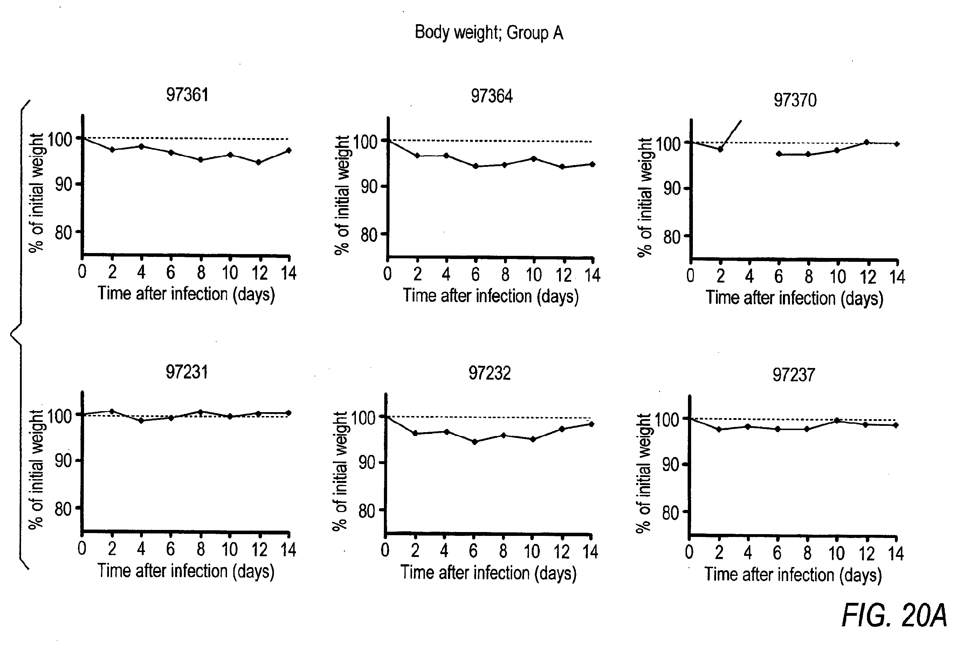

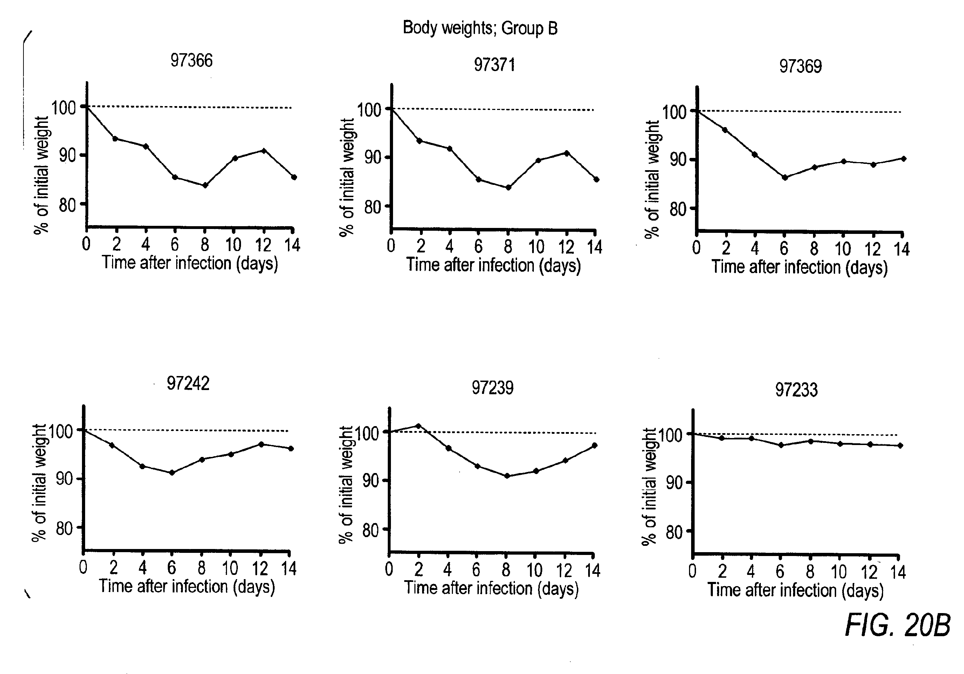

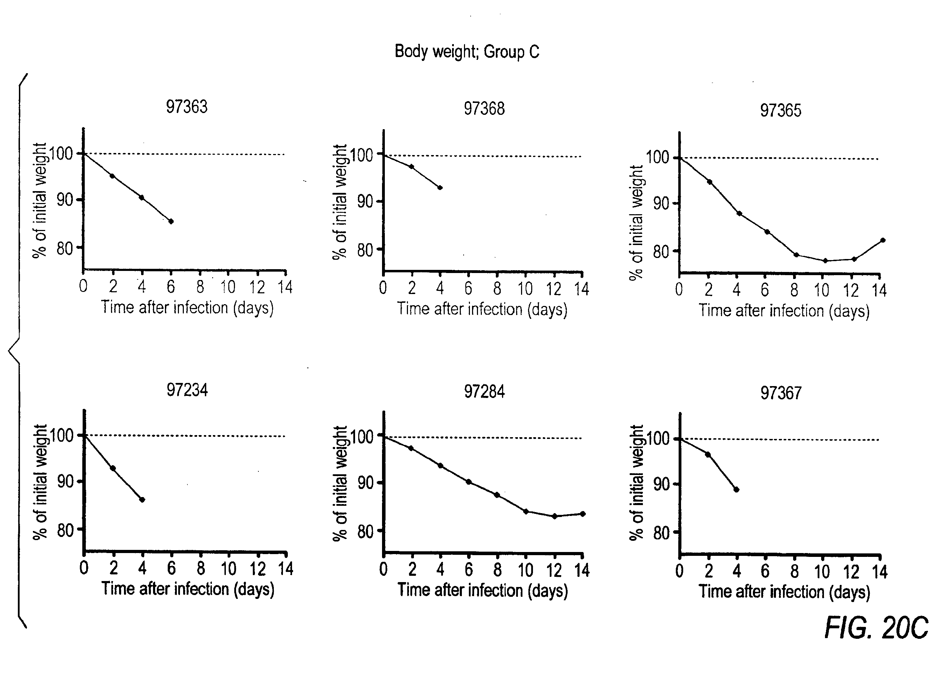

The animals vaccinated with vCP2241 experienced weight less of less than 6 % of their initial weight (see Figure 20A), while the animals vaccinated with vFP89 showed weight loss from 2.3 % to 16.2 % that occurred on days 6-8 (see Figure 20B). The weights of the animals that experienced the greater weight loss began to normalize to initial values after the maximum loss was reached. In the control group, only two animals survived; one animal had suffered a maximum weight loss of 22.3 % by day 10, while the other animal loss 17 % of its initial weight by day 12 (see Figure 20C).

Body temperature (BT) was recorded throughout the study. In the animals vaccinated with vCP2241, three animals did not show clear changes in BT, two animals displayed a temporary elevation in BT, and one animal showed a fever that lasted from day 1 to day 4 with a maximal BT at ~40°C (see Figures 21A and 21B). In animals vaccinated with vFP89, one animal died on day 1 and experienced a rapid increase in BT, while the other animals experienced a fever from ~40°C to ~41°C on days 1 to 3 (see Figures 21C and 21D). In the control group one animal experienced two episodes of fever lasting 5 days and 3 days with a maximal BT ~41.2°C (see Figures 21E and 21F). Four of the animals experienced a fever on day 1 that lasted until they died or were moribund sacrificed, while one animal showed normal BT by day 6 after initially experiencing a fever.

The animals vaccinated with vCP2241 did not display signs of respiratory distress, while only one of the animals vaccinated with vFP89 exhibited respiratory distress. In contrast, all animals of the control group experienced severe respiratory distress after AIV H5 challenge.

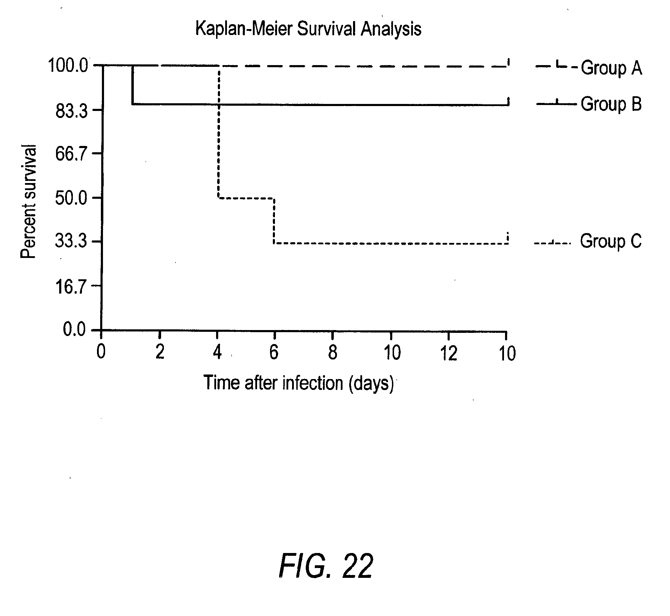

No animal vaccinated with vCP2241 died before the end of the study, while one animal in the group vaccinated with vFP89 died on day 1 due to complications of the anaesthesia. In the challenge control group, one animal was found dead and two other animals were moribund sacrificed on day 4. Another animal was found dead on day 6, such that only two animals survived the duration of the study (see Figure 22).

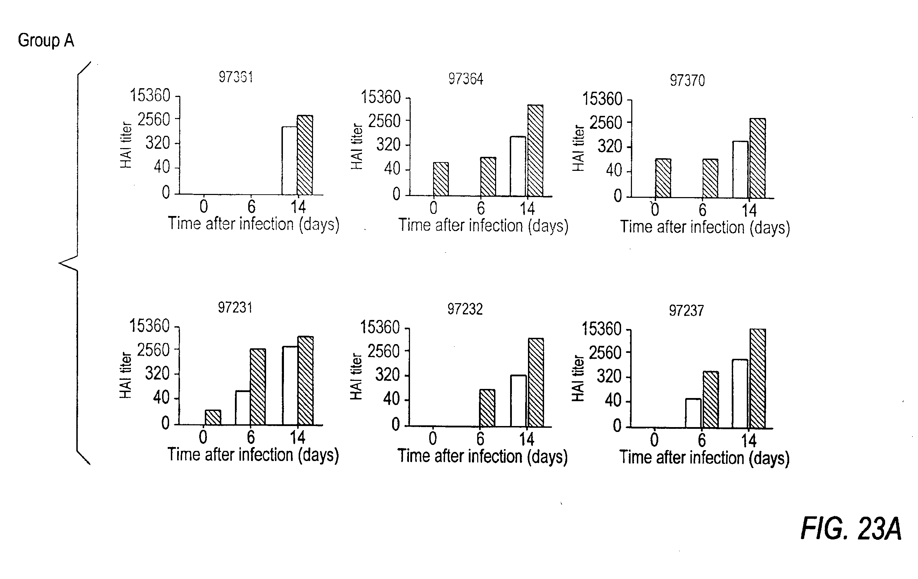

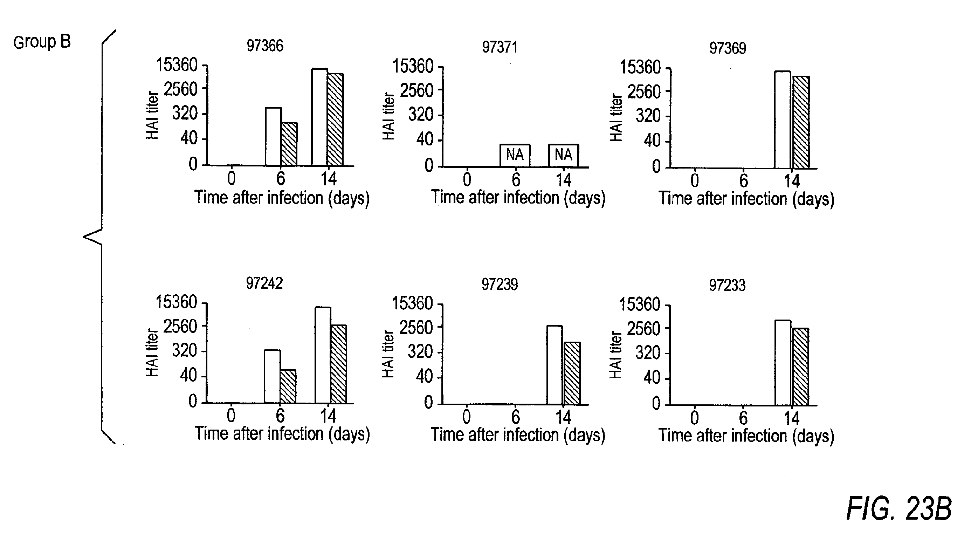

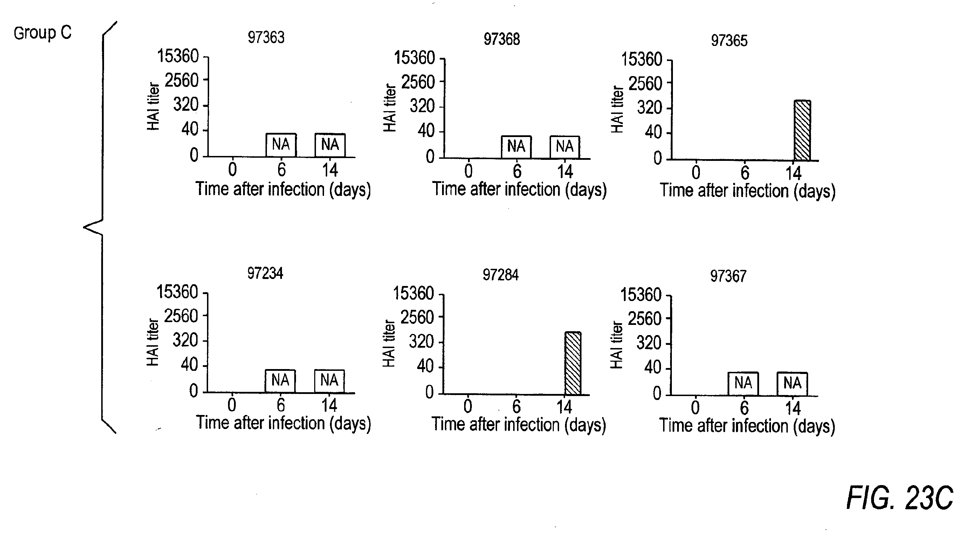

Analysis of antibody titers using the HI assay revealed that, in animals vaccinated with vCP2241, antibody titers against the Vietnam strain were generally lower as compared to antibody titers against the Indonesia strain (see Figure 23). Two animals of this group had detectable antibody titers against the Indonesia strain on day 0 and all six animals had antibody titers against the Indonesia strain by day 14. In animals vaccinated with vFP89, the antibody titers against the Vietnam strain were slightly higher as compared to the antibody titers against the Indonesia strain. Three animals remained sero-negative until day 14 (see Figure 23). In the control group, one animal was sero-positive against the Indonesia strain on the day of the challenge, and showed antibody titers against the Vietnam strain by day 14. The other animal that survived for the duration of the study also showed antibody titers against the Indonesia strain on day 14 (see Figure 23).

No detectable virus was measured in the plasma sample of any of the animals.

No virus was detectable in the pharyngeal, nasal, or rectal swabs in the animals vaccinated with vCP2241 (see Figure 24). In animals vaccinated with vFP89, all five of the surviving animals shed high levels of virus in the pharynx from day 2 to day 6, although no virus could be measured in the nasal or rectal swabs (see Figure 24). In the control group, five of the six animals showed virus shedding in the pharynx and two of these animals also showed virus shedding in the nasal cavity (see Figure 24). One animal did not show any detectable virus in the pharynx or nasal, but was moribund sacrificed on day 4. None of the challenge controls showed any detectable virus in the rectal swabs.

Virus detection measurements in the lungs revealed that the vaccinated animals displayed low levels of virus as assessed through PCR and virus titration assay. In contrast, the four animals in the challenge control group that died or were moribund sacrificed early displayed high viral titers in the lungs. These results are shown in Table 3.

**Table 3. Virus detection results in the lungs as measured by PCR and virus titration.**

| **Group** | **Animal #** | **Day of death/ euthanasia** | **PCR (10 log CDU)** | **Virus titration (10 log TCID₅₀/g)** |
|---|---|---|---|---|
| vCP2241 | 97361 | 14 | 2.00 | 1.21 |
| vCP2241 | 97364 | 14 | 2.06 | 1.27 |
| vCP2241 | 97365 | 14 | 2.03 | 1.24 |
| vCP2241 | 97231 | 14 | 2.18 | 1.39 |
| vCP2241 | 97232 | 14 | 2.10 | 1.31 |
| vCP2241 | 97237 | 14 | 2.22 | 1.43 |
| vFP89 | 97366 | 14 | 2.12 | 1.33 |
| vFP89 | 97371 | 1 | nd | nd |
| vFP89 | 97369 | 14 | 2.18 | 1.39 |
| vFP89 | 97242 | 14 | 2.32 | 1.53 |
| vFP89 | 97239 | 14 | 2.42 | 1.63 |
| vFP89 | 97233 | 14 | 2.18 | 1.39 |
| Challenge controls | 97363 | 6 | 6.39 | 6.56 |
| Challenge controls | 97368 | 4 | 5.06 | 6.91 |
| Challenge controls | 97370 | 14 | 2.25 | 1.46 |
| Challenge controls | 97234 | 4 | 5.17 | 6.92 |
| Challenge controls | 97284 | 14 | 2.06 | 1.27 |
| Challenge controls | 97367 | 4 | 6.07 | 7.37 |

Observation of the lungs showed a lower incidence and/or severity of diffuse dark red areas and lesions in animals vaccinated with vCP2241. In the animals vaccinated with vFP89, there was a slightly higher incidence/severity of subacute bronchiolitis as compared to the other groups.

### Challenge with A/Vietnam/1194/2004

### Methods and Materials

Eighteen cats, aged 7 months old, were divided into 3 groups with 3 males and 3 females in each group. Two groups were subcutaneously vaccinated on day 0 and day 21 with either vCP2241 (ALVAC canarypox virus expressing H5 gene from the AIV strain A/Chicken/Indonesia/03, H5N1) or vFP89 (TROVAC fowlpox virus expressing H5 gene from the AIV strain A/Turkey/Ireland/1378/83, H5N8), both at a dose of 7.2 log10 CCID 50. The third group served as challenge controls and received no vaccination. All animals were challenged on day 69 by intratracheal infection with 3 mL containing 1.0 x 10⁵ TCID₅₀ of AIV strain A/Vietnam/1194/2004 (H5N1).

Clinical examinations, HI assays of collected blood, and viral RNA levels in pharyngeal, nasal, and rectal swabs were all conducted as described above. Further, similar to the previous AIV challenge study, all surviving animals were euthanized on day 14, and a complete post-mortem examination was performed, including detection of levels of viral RNA in the lungs.

### Results

In general, the animals challenged with AIV strain A/Vietnam/1194/2004 exhibited similar clinical results as the animals challenged with AIV than A/Indonesia/5/2005, although the effects of the AIV strain A/Vietnam/1194/2004 were more severe. Animals vaccinated with vCP2241 experienced a lower incidence of weight loss and hyperthermia than animals vaccinated with vFP89.

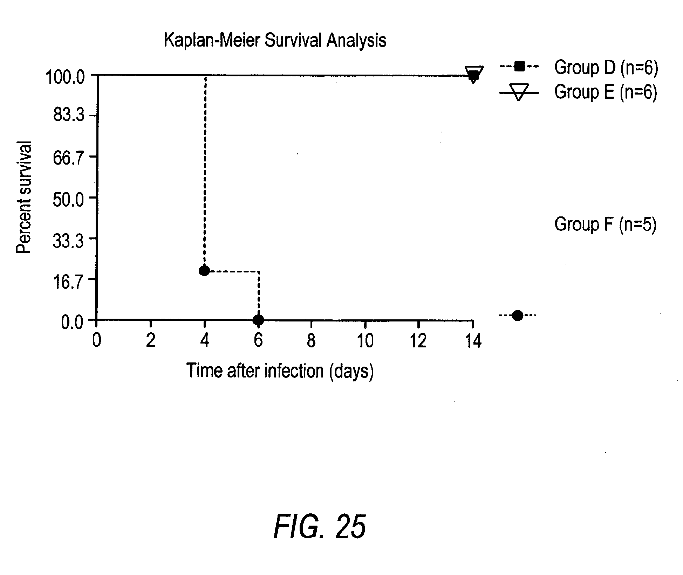

No animal vaccinated with either vCP2241 or vFP89 died before the end of the study. This is in contrast to the animals of the challenge control group, wherein no animals survived the duration of the study (see Figure 25).

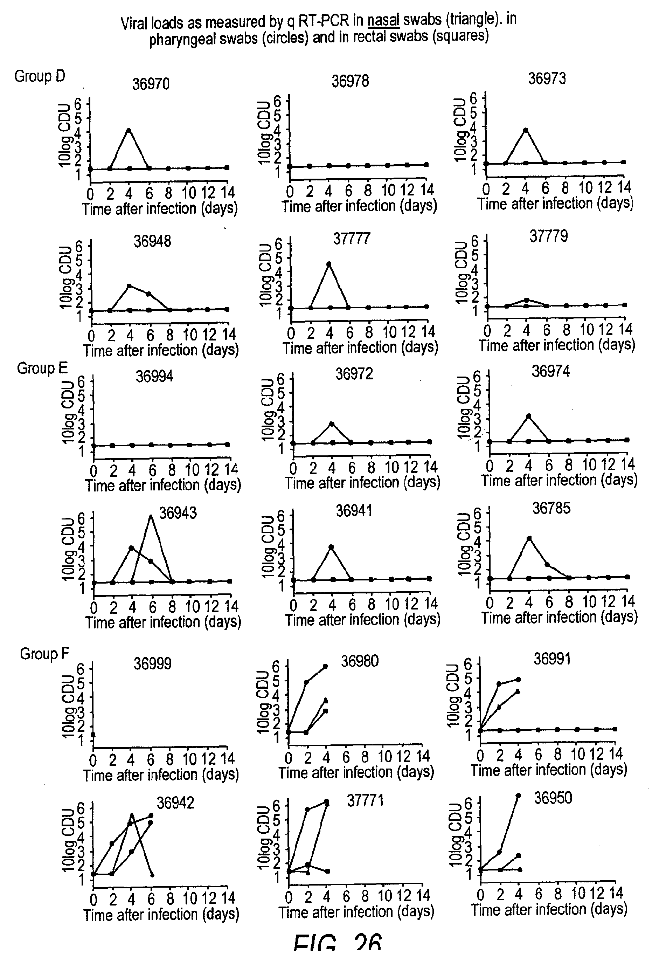

Analysis of antibody titers using the HI assay revealed that, in animals vaccinated with vCP2241, antibody titers against the Vietnam strain were generally equal or higher as compared to antibody titers against the Indonesia strain (see Figure 26). Three animals of this group had no detectable antibody titers against either AIV strain on day 0 and all six animals had antibody titers against both strains by day 6. In animals vaccinated with vFP89, the antibody titers against both strains were essentially equal except for one animal. Moreover, only one animal had detectable antibody titers in either AIV strain on day 0, but all animals vaccined with vFP89 had detectable titer levels of both strains by day 6 (see Figure 26). No animal of the challenge control group survived through day 6 in order for blood to be collected for the HI assay.

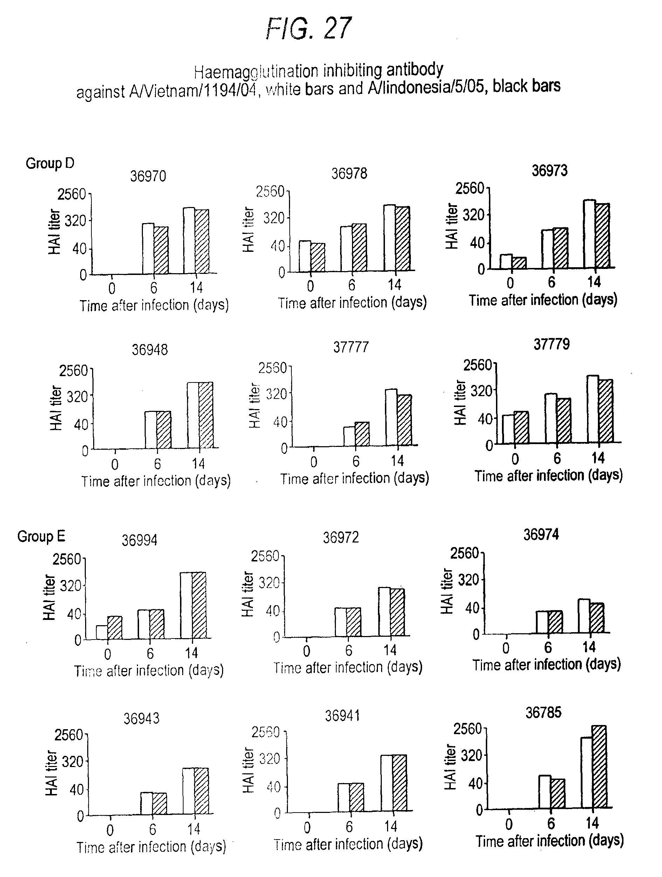

Virus was detectable in the pharyngeal, nasal, or rectal swabs in five of the six animals vaccinated with vCP2241, although no virus was measured in these animals after day 8 (see Figure 27). Simiarly, in animals vaccinated with vFP89, five of the six animals exhibited a measurable presence of the virus at day 4 of the study, but none displayed detectable levels atfter day 8 (see Figure 27). In the control group, five of the six animals showed virus shedding in all of the swabbed cavities.

Overall, vaccination with vCP2241 and vFP89 protected cats from mortality and morbidity, thereby demonstrating the protective capacity of both vaccines.

### Example 7: Vaccination of cats with inactivated AIV

A study was conducted in which two groups of 20 week-old cats received vaccines comprising inactivated AIV. The viruses (H5N9 AIV A/Chicken/Italy/22A/98) were inactivated by 0.1 % beta-propiolactone at 5°C for 18 hours, and were then harvested and filtrated through a 10µm cutoff filter. The vaccine was adjuvanted with oil-in-water emulsion.

All cats experienced subcutaneous injections in the interscapula area of a 1ml dose on days 0 and 21. The first group (n=5) received approximately 512 HA units (UHA) of inactivated vaccine, while the second group (n=5) received approximately 1536 UHA of inactivated vaccine.

Blood samples were collected on days 0, 14, 21, and 35 for all animals. A hemagglutination inhibition (HI) test was performed as described in Example 4. For this study, the homologous AIV antigen was H5N9 AIV A/Turkey/Wisconsin/68, and the heterologous antigen was H5N1 AIV A/Vietnam/1194/04 (NIBRG14 strain). A 0.5 % (vol/vol) suspension of cRBCs were allotted per well. The results are presented as log 10 of the highest dilution of cat serum that inhibited hemaggluntination.

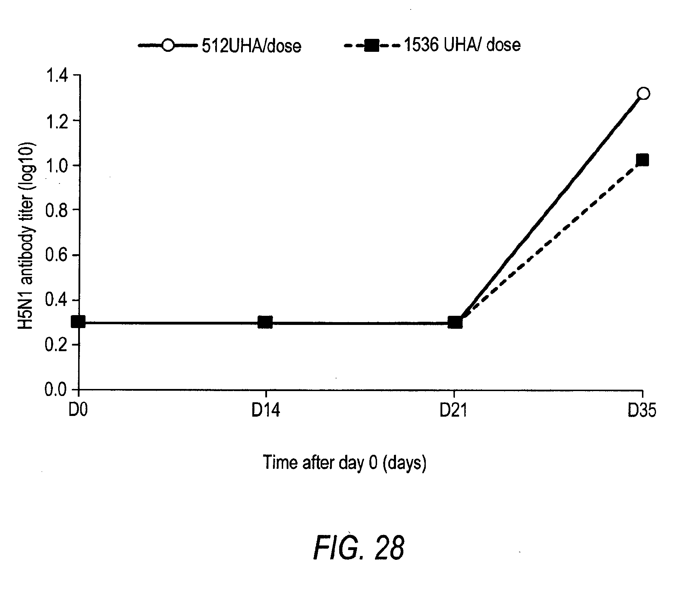

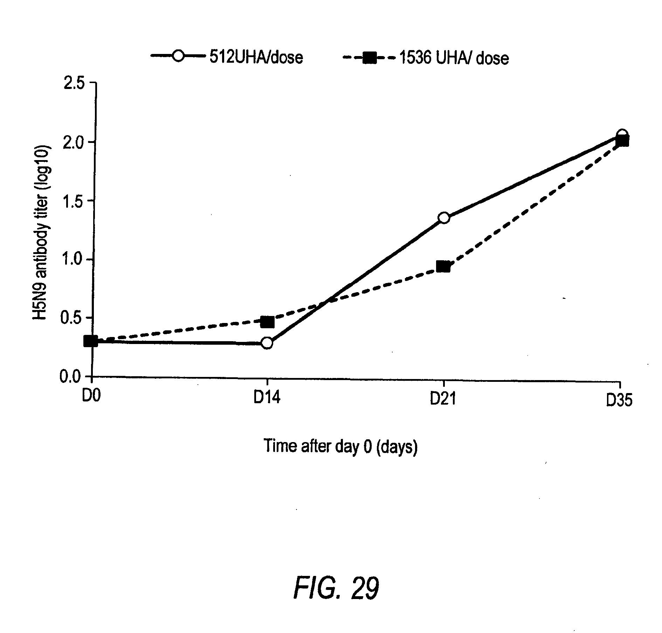

The results indicate that cats injected with the inactivated vaccine induced high levels of antibodies to H5 avian influenza. While cats injected with 512 UHA/dose displayed higher HI responses against both homologous (see Figure 28) and heterologous (see Figure 29) antigens than cats injected with 1536 UHA/dose, both dosages induced an immunogenic response.

### REFERENCES

1. Abed et al. J Infect Dis. 186: 1074-1080, 2002
2. Air et al. Virology. 160: 346-54, 1987
3. Alexander et al. Res Vet Sci. 26: 17-20, 1979
4. Altschul et al. J Mol Biol.215: 403-410, 1990
5. Altschul et al. Local alignment statistics. Methods Enzymol. 266: 460-480, 1996
6. Amonsin et al. Virology. 344:480-491, 2006
7. Andreansky et al. Proc Natl Acad Sci USA. 93: 11313-11318, 1996
8. Antoine. Virology 244: 365-396, 1998
9. Anwar et al. In Silico Biology 6: 0015, 2006
10. Appleton et al. Vet Immunol Immunopathol 23: 257-266, 1989
11. Audsley et al. Expert Opin Biol Ther. 4: 709-717, 2004
12. Audsley et al. J Virol Methods. 123: 187-193, 2005
13. Austin et al. J Gen Virol. 71: 2471-2474, 1990
14. Ayora-Talavera et al. Emerg Infect Dis. 11:158-161, 2005
15. Babiuk et al. Vaccine. 20: 3399-3408, 2002
16. Baez et al. Virology. 113: 397-402, 1981
17. Ballay et al. EMBO J. 4:3861-3865, 1994
18. Banks et al. Avian Pathol. 29: 353-360, 2000
19. Bantia et al. Antimicrob Agents Chemother. 42: 801-807, 1998
20. Bean et al. J Virol. 54: 151-160, 1985
21. Behr. Bioconjug Chem. 5: 382-389, 1994
22. Bergmann et al. Eur J Immunol. 23: 2777-2781, 1993
23. Bergmann et al. J Immunol. 157: 3242-3249; 1996
24. Berkner et al. Nucl Acid Res. 11: 6003-6020, 1983
25. Berkner. Bio Techniques. 6: 616-629, 1988
26. Blok et al. Biochemistry 21: 4001-4007, 1982
27. Boshart et al. Cell. 41: 521-530, 1985
28. Brands et al. Dev Biol Stand. 98: 93-100, 1999
29. Breathnach et al. Vet Immunol Immunopathol. 98: 127-136, 2004
30. Brown et al. Clin Microbiol. 26: 313-318, 1988
31. Brown et al. Mol Immunol. 19: 329-338, 1982
32. Brown et al. Vet Rec. 132: 598-602, 1993
33. Budowsky et al. Vaccine. 11: 343-348, 1993
34. Budowsky et al. Vaccine. 9: 398-402, 1991
35. Butler. Nature. 440: 135, 2006
36. Capua et al. Avian Pathol. 28: 455-460, 1999
37. Carroll et al. Vaccine 15: 387-394, 1997
38. Carter et al. Arch Virol. 71: 13-25, 1982
39. Cauthen et al. J Virol. 74: 6592-6599, 2000
40. Chambers et al. Equine Vet J. 33: 630-636, 2001
41. Chare et al. J Gen Virol. 84: 2691-2703, 2003
42. Chavier et al J Virol. 70: 4805-4810, 1996
43. Chen et al. Avian Dis. 47: 1127-1130, 2003
44. Cheung et al. Lancet. 360: 1831-1137, 2002
45. Chin et al. J. Virol. 76: 507-516, 2002
46. Chroboczek et al. Virol 186: 280-285, 1992
47. Claas et al. Lancet. 351: 472-477, 1998
48. Clavijo et al. Can J Vet Res. 66 :117-121,2002
49. Cochran et al. J Virol. 54: 30-35, 1985
50. Crouch et al. Vaccine. 23: 418-425, 2004
51. Danthinne et al. Gene Therapy. 7: 1707-1714, 2000
52. De et al. Nucleic Acids Res. 16: 4181-4182, 1988
53. De Groot et al. Nat Biotechnol. 17: 533-561, 1990
54. de Jong et al. J Clin Virol. 35: 2-13, 2006
55. Donofrio et al. J Vet Diagn Invest. 6: 39-43, 1994
56. Duhaut et al. Virology. 248: 241-253, 1998
57. Duhaut et al. Virology. 275: 278-285, 2000
58. Egorov et al. J Virol. 72: 6437-6441, 1998
59. Elleman et al. Nucleic Acids Res. 10: 7005-7015, 1982
60. EP 0370573
61. EP 265785
62. EP-A-260148
63. EP-A-323597
64. Falloux et al. Hum Gene Ther. 9: 1909-1917, 1998
65. Felgner et al. J Biol Chem. 269:2550-2561, 1994
66. Fields et al. Nature. 186: 778-780, 1960
67. Fischer et al. Vaccine. 20: 3485-3497, 2002
68. Foni et al. New Microbiol. 28: 31-35, 2005
69. Fouchier et al. J Virol. 79: 2814-2822, 2005
70. Frolov et al. Proc Natl Acad Sci USA. 93: 11371-11377, 1996
71. Funahashi et al. J Gen Virol. 69: 35-47, 1988
72. Gambaryan et al. Virus Res. 114: 15-22, 2005
73. Gao et al. J Virol. 80: 1959-1964, 2006
74. Garcia et al. Avian Dis. 42: 248-256, 1998
75. Gardner et al. 12th World AIDS Conference, Geneva, Switzerland, 1998.
76. Gething et al. Nature 287: 301-306, 1980
77. Geysen et al. Molec Immunol. 23: 709-715, 1986
78. Geysen et al. Proc Natl Acad Sci USA. 81: 3998-4002, 1984
79. Geysen et al. Proc Natl Acad Sci USA. 82: 178-182, 1985
80. Geysen. Southeast Asian J Trop Med Public Health 21: 523-533, 1990
81. Ghendon et al. Vaccine. 23: 4678-4684, 2005
82. Gibson et al. Virus Res. 22: 93-106, 1992
83. Gish et al. Nat Genet. 3: 266-272, 1993
84. Goto et al. J Vet Med Sci. 55: 33-37, 1993
85. Govorkova et al. J Virol. 70: 5519-5524, 1996
86. Govorkova et al. J. Infect. Dis. 172: 250-253, 1995
87. Graham et al. Gene Transfer and Expression Protocols. Methods in Mol. Biol. The Human Press Inc. 7: 109-128, 1991
88. Graham et al. J Gen Virol 36: 59-72, 1977
89. Graham. Tibtech 8: 85-87; , 1990
90. Gross et al. Equine Vet J. 30: 489-497, 1998
91. Grunhaus et al. Sem Virol. 3: 237-252, 1992
92. Guan et al. J Virol. 74: 9372-9380, 2000
93. Guan et al. Proc Natl Acad Sci U S A. 10: 8156-8161, 2004
94. Gubareva et al. J Gen Virol. 83: 2683-2692, 2002
95. Gubareva et al. J Infect Dis. 183: 523-531, 2001
96. Gubareva et al. J Virol. 71: 3385-3390, 1997
97. Gujuluva et al. Virology. 204: 491-505, 1994
98. Guo et al. J Virol 63: 4189-4198, 1989
99. Halperin et al. Vaccine. 16: 1331-1335, 1998
100. Halperin et al. Vaccine. 20: 1240-1247, 2002
101. Hannant et al Vet Immunol Immunopathol. 21: 327-337, 1989
102. Hannant et al. Vet Microbiol. 19: 293-303, 1989
103. Hannant et al. Vet Rec. 122: 125-128, 1988
104. Hardy et al. Virus Res. 77: 89-96, 2001
105. Harley et al. Arch Virol. 113: 267-277, 1990
106. Hartikka et al. Hum Gene Ther. 7: 1205-1217, 1996
107. Hatta et al. Virology 295: 250-55, 2002
108. Hauptmann et al. J Gen Virol. 64: 215-20, 1983
109. Hayden et al. Antiviral Res. 25: 123-131, 1994
110. Heldens et al. J Immunol Methods. 264: 11-17, 2002
111. Heldens et al. Vet J. 167:150-157, 2004
112. Hemmer et al. Immunol Today, 19: 163-168, 1998
113. Hinshaw et al. Infect Immun. 24 : 354-361, 1981
114. Hoffmann et al. Proc Natl Acad Sci USA. 99: 11411-11416, 2002
115. Horimoto et al. Clin Microbiol Rev. 14: 129-149, 2001
116. Homer et al. Ledgard, N Z Vet J. 36: 205-206, 1988
117. Homer et al. N Z Vet J. 36:205-206, 1988
118. Howard et al. Avian Dis. 2006
119. Iamnikova et al. Vopr Virusol. 34: 568-572, 1989
120. Iftimovici et al. Virologie. 31: 243, 1980
121. Ilan et al. Proc Natl Acad Sci USA. 94: 2587-2592, 1997
122. Ilobi et al. Arch. Virol. 143: 891-901, 1998
123. Influenza team. Euro Surveill. 11: E060413.4, 2006
124. Iuferov et al. Dokl Akad Nauk SSSR. 278: 738-742, 1984
125. Johansson et al. Vaccine. 16: 1009-1015, 1998
126. Ju et al. Diabetologia 41: 736-739, 1998
127. Kanegae et al. Arch Virol. 134: 17-28, 1994
128. Karaca et al. Clin Diagn Lab Immunol. 12: 1340-1342, 2005
129. Karasin et al. J Clin Microbiol. 38: 2453-2456, 2000
130. Karasin et al. J Clin. Microbiol. 40: 1073-1079, 2002
131. Karasin et al. J Clinical Microbiol. 44: 1123-1126, 2006
132. Karlin et al. Proc Natl Acad Sci USA. 87: 2264-2268, 1990
133. Karlin et al. Proc Natl Acad Sci. USA 90: 5873-5877, 1993
134. Kawaoka et al. Virology 179: 759-767, 1990
135. Keawcharoen et al. Emerg Infect Dis. 10: 2189-91, 2004
136. Keverin et al. Arch Virol. 145: 1059-1066, 2000
137. Kistner et al. Vaccine. 16: 960-968, 1998
138. Kitson et al. J Virol. 65: 3068-3075, 1991
139. Klinman et al. Proc Natl Acad Sci USA. 93: 2879-2883, 1996
140. Kuiken et al. Nature. 440: 741-742, 2006,
141. Kuiken et al. Science. 306: 241-242, 2006
142. Kwissa et al. Vaccine. 18: 2337-2344, 2000
143. Landolt et al. Am J Vet Res. 66: 119-124, 2005
144. Lee et al. J Virol. 79: 11412-11421, 2005
145. Leneva et al. Antimicrob Agents Chemother. 45: 1216-1224, 2001
146. Li et al. Nat Biotechnol. 17: 241-245, 1999
147. Lin et al. Virology. 287: 202-213, 2001
148. Lindstrom et al. Arch Virol. 143: 1585-1598, 1998
149. Liu et al. Virology 305: 267-275, 2003
150. Lu et al. Arch Virol. 147: 273-284, 2002
151. Luke et al. J Infect Dis. 175: 91-97, 1997
152. Macklin et al. J Virol. 72: 1491-1496, 1998
153. Mantani et al. Planta Med. 67: 240-243, 2001
154. Markoff et al. Virology. 119: 288-297, 1982
155. Marozin et al. J. Gen. Virol. 83:735-745, 2002
156. Mazanec et al. J Virol. 69: 1339-1343, 1995
157. McClements et al. Proc Natl Acad Sci USA. 93: 11414-11420, 1996
158. Merten et al Adv Exp Med Biol. 397: 141-151, 1996
159. Miyamoto et al. Antiviral Res. 39: 89-100, 1998
160. Miyazaki et al. Gene. 79: 269-277, 1989
161. Mohler et al. Biotechnol Bioeng. 90: 46-58, 2005
162. Morley et al. Vet Microbiol. 45: 81-92, 1995
163. Morris GE, ed. Epitope Mapping Protocols in Methods in Mol. Bio. Humana Press Inc. 66, 1996
164. Moss. Proc Natl Acad Sci USA. 93: 11341-11348, 1996
165. Mumford et al. Epidemiol Infect. 100: 501-510, 1988
166. Mumford et al. J Hyg (Lond). 9: 385-395, 1983
167. Mumford et al. Vet Rec. 134:158-162, 1994
168. Naeve et al. EMBO J. 9: 3857-3866, 1990
169. Nagai et al. Antiviral Res. 26: 11-25, 1995
170. Nagai et al. Biol Pharm Bull. 18: 295-299, 1995
171. Nakagawa et al. J Virol Methods. 79: 113-120, 1999
172. Nayak et al. J Chromatogr B Analyt Technol Biomed Life Sci. 823: 75-81, 2005
173. Nelson et al. FASEB J. 15: 1846-1848, 2001
174. Nerome et al. Arch Virol. 86: 197-211, 1985
175. Nestorowicz et al. Virology 160: 411-418, 1987
176. Noble et al. J Gen Virol. 75: 3485-3491, 1994
177. Nunes-Correia et al. Biochemistry. 38: 1095-1101, 1999
178. Ohuchi et al. J Virol. 68: 920-926, 1994
179. Olsen et al. Arch Virol. 145: 1399-1419, 2000
180. Olsen et al. Science. 312: 384-388, 2006
181. Olsen et al. Vaccine. 15: 1149-1156, 1997
182. Orlich et al. Virology. 176: 531-538, 1990
183. Ottis et al. Arch Virol. 63: 185-190, 1980
184. Ozaki et al. Vet Microbiol. 82: 111-119, 2001
185. Palker et al. Virus Res. 105: 183-194, 2004
186. Panicali et al. Proc. Natl Acad Sci USA. 79: 4927-4931, 1982
187. Panigrahy et al. Avian Dis. 40: 600-604, 1996
188. Paoletti. Proc Natl Acad Sci USA. 93: 11349-11353, 1996
189. PCT Application No. WO87/03905.
190. PCT Application No. WO89/01036
191. PCT Application No. WO95/14102
192. PCT Application No. WO98/00166
193. PCT Application No. WO99/01158
194. PCT Application Serial No. PCT/US2004/022605
195. Pearson et al. Proc Natl Acad Sci USA. 85: 2444-2448, 1988
196. Pennock et al. Mol Cell Biol. 4: 399-406, 1984
197. Perkins et al. Avian Dis. 46: 877-885, 2002
198. Perkins et al. Vet Pathol. 38: 149-164, 2001
199. Perkus et al. J Virol 63: 3829-3836, 1989
200. Pharmeuropa 8(2), 1996
201. Philpott et al. J Virol 63: 3453-3458, 1989
202. Piccini et al. Methods Enzymol. 153: 545-563, 1987
203. Powell. Vaccine Design, The Subunit and Adjuvant Approach. Plenum Press. 147, 1995
204. Pruett et al. Biochemistry. 37: 10660-10670, 1998
205. Puthavathana et al. J Gen Virol. 86: 423-433, 2005
206. Quinlivan et al. J Clin Microbiol. 42: 759-763, 2004
207. Richards et al. Vet Immunol Immunopathol. 33: 129-143, 1992
208. Richardson (Ed). Baculovirus Expression Protocols. Methods in Mol. Biol. 39, Humana Press Inc., 1995
209. Rimmelzwaan et al. Am J Pathol. 168: 176-183, 2006
210. Riviere et al. J Virol. 66: 3424-3434, 1992
211. Robertson et al. Proc Natl Acad Sci USA. 93: 11334-11340, 1996
212. Robinson et al. Sem Immunol. 9: 271-283, 1997
213. Rohm et al. Virology 218: 253-257, 1996
214. Rohin et al. Virology. 217: 508-516, 1996
215. Roizman. Proc Natl Acad Sci. USA. 93: 11307-11312, 1996
216. Ross et al. Archiv Für die gesamte Virusforschung. 30: 82-88, 1970
217. Rott et al. J Gen Virol. 44: 471-77, 1979
218. Saito et al. Virology. 193: 868-876, 1993
219. Sambrook et al. Molecular Cloning: A Laboratory Manual 2nd ed, 1989
220. Scholtissek et al. J Virol. 76: 1781-1786, 2002
221. Shida. Virology. 150: 451-457, 1986
222. Shimizu et al. Virology. 254: 213-219, 1999
223. Shinya et al. J Virol. 79: 9926-9932, 2005
224. Short et al. J Vet Pharmacol Ther. 9: 426-432, 1986
225. Shortridge et al. Virology 252: 331-342, 1998
226. Shriver et al. Nature. 415: 331-335, 2002
227. Sidwell et al. Antiviral Res. 37: 107-120, 1998
228. Slemons et al. Bull World Health Organ. 47: 521-525, 1972
229. Smirnov et al. Acta Virol. 44: 1-8, 2000
230. Smith et al. Mol Cell Biol. 3: 2156-2165, 1983
231. Staschke et al. Virology. 248: 264-274, 1998
232. Stenberg et al. J Virol. 49: 190-199, 1984
233. Stickl et al. Munch Med Wschr. 113: 1149-1153, 1971
234. Stittelaar et al. J Virol. 74: 4236-4243, 2000
235. Sugiura et al. J Virol Methods. 98: 1-8, 2001
236. Suhrbier. Immunol. Cell Biol. 75: 402-408; 1997
237. Sutter et al. Proc Natl Acad Sci USA. 89: 10847-10851, 1992
238. Sutter et al. Vaccine. 12: 1032-1040, 1994
239. Suzuki et al. Biochem J. 318: 389-393, 1996
240. Swayne et al. Avian Dis. 38: 151-157, 1994
241. Swayne et al. Avian Dis. 45: 355-365, 2001
242. Takeuchi et al. J Virol. 68: 911-919, 1994
243. Tapnell. Adv Drug Deliv Rep.12: 185-199, 1993
244. Taylo et al. J-Virol. 64, 1441-1450, 1990
245. Taylor et al. Vaccine. 6: 497-503, 1988a
246. Taylor et al. Vaccine. 6: 504-508, 1988b
247. Terregino et al. Vet Rec. 158: 491, 2006
248. Thomson et al. Vet Rec. 100: 465-468, 1977
249. Tollefsen et al. Scand. J. Immunol. 57: 229-238, 2003
250. Tollefsen et al. Vaccine. 20: 3370-3378, 2002
251. Tolstova et al. Acta Virol. 10: 315-321, 1966
252. Tomita et al. Kitasato Arch Exp Med. 44: 185-196, 1971
253. Tonew et al. Acta Virol. 26: 444-452, 1982
254. Toulemonde et al. Vet Rec. 156: 367-371, 2005
255. Treanor et al. J Virol. 68: 7684-7688, 1994
256. Tree et al. Vaccine. 19: 3444-3450, 2001
257. Tripathy et al. Proc Natl Acad Sci USA. 91: 11557-11561, 1994
258. Tumpey et al. J Virol. 76: 6344-6355, 2002
259. U.S. Patent Application Serial No. 920,197
260. U.S. Patent No. 2,909,462
261. U.S. Patent No. 4,394,448
262. U.S. Patent No. 4,603,112
263. U.S. Patent No. 4,603,112
264. U.S. Patent No. 4,708,871
265. U.S. Patent No. 4,722,848
266. U.S. Patent No. 4,722,848
267. U.S. Patent No. 4,745,051
268. U.S. Patent No. 4,769,330
269. U.S. Patent No. 4,769,330
270. U.S. Patent No. 4,769,331
271. U.S. Patent No. 4,945,050
272. U.S. Patent No. 4,968,615
273. U.S. Patent No. 5,110,587
274. U.S. Patent No. 5,122,458
275. U.S. Patent No. 5,168,062
276. U.S. Patent No. 5,174,993
277. U.S. Patent No. 5,174,993
278. U.S. Patent No. 5,338,683
279. U.S. Patent No. 5,382,425
280. U.S. Patent No. 5,385,839
281. U.S. Patent No. 5,494,807
282. U.S. Patent No. 5,494,807
283. U.S. Patent No. 5,494,807
284. U.S. Patent No. 5,494,807
285. U.S. Patent No. 5,494,807
286. U.S. Patent No. 5,505,941
287. U.S. Patent No. 5,505,941
288. U.S. Patent No. 5,505,941
289. U.S. Patent No. 5,505,941
290. U.S. Patent No. 5,514,375
291. U.S. Patent No. 5,529,780
292. U.S. Patent No. 5,552,143
293. U.S. Patent No. 5,580,859
294. U.S. Patent No. 5,589,466
295. U.S. Patent No. 5,591,439
296. U.S. Patent No. 5,591,639
297. U.S. Patent No. 5,677,178
298. U.S. Patent No. 5,688,920
299. U.S. Patent No. 5,744,140
300. U.S. Patent No. 5,744,141
301. U.S. Patent No. 5,756,103
302. U.S. Patent No. 5,756,103
303. U.S. Patent No. 5,756,103
304. U.S. Patent No. 5,756,103
305. U.S. Patent No. 5,762,938
306. U.S. Patent No. 5,762,938
307. U.S. Patent No. 5,766,599
308. U.S. Patent No. 5,766,599
309. U.S. Patent No. 5,766,599
310. U.S. Patent No. 5,846,946
311. U.S. Patent No. 5,990,091
312. U.S. Patent No. 6,004,777
313. U.S. Patent No. 6,033,670
314. U.S. Patent No. 6,045,803
315. U.S. Patent No. 6,048,537
316. U.S. Patent No. 6,048,537
317. U.S. Patent No. 6,074,649
318. U.S. Patent No. 6,090,393
319. U.S. Patent No. 6,090,393
320. U.S. Patent No. 6,103,526
321. U.S. Patent No. 6,130,066
322. U.S. Patent No. 6,130,066
323. U.S. Patent No. 6,133,028
324. U.S. Patent No. 6,153,199
325. U.S. Patent No. 6,156,567
326. U.S. Patent No. 6,156,567
327. U.S. Patent No. 6,159,477
328. U.S. Patent No. 6,159,477
329. U.S. Patent No. 6,207,165
330. U.S. Patent No. 6,207,166
331. U.S. Patent No. 6,217,883
332. U.S. Patent No. 6,221,362
333. U.S. Patent No. 6,221,362
334. U.S. Patent No. 6,224,882
335. U.S. Patent No. 6,228,846
336. U.S. Patent No. 6,306,400
337. U.S. Patent No. 6,312,682
338. U.S. Patent No. 6,348,196
339. U.S. Patent No. 6,348,450
340. U.S. Patent No. 6,368,603
341. U.S. Patent No. 6,376,473
342. U.S. Patent No. 6,376,473
343. U.S. Patent No. 6,387,376
344. U.S. PatentNo. 6,391,314
345. U.S. Patent No. 6,451,769
346. U.S. Patent No. 6,451,770
347. U.S. Patent No. 6,451,770
348. U.S. Patent No. 6,464,984
349. U.S. Patent No. 6,464,984
350. U.S. Patent No. 6,485,729
351. U.S. Patent No. 6,497,883
352. U.S. Patent No. 6,558,674
353. U.S. Patent No. 6,576,243
354. U.S. Patent No. 6,586,412
355. U.S. Patent No. 6,596,279
356. U.S. Patent No. 6,692,956
357. U.S. Patent No. 6,818,628
358. U.S. Patent No. 6,852,705
359. U.S. Patent No. 6;312,683
360. van der Goot et al. Avian Dis. 47: 939-941, 2002
361. Van der Zee et al. Eur J Immunol. 19: 43-47, 1989
362. van Maanen et al. Vet Microbiol. 93: 291-306, 2003
363. van Ooyen et al. Science. 206: 337-344, 1979
364. Viseshakul et al. Virology 328: 169-176, 2004
365. Walker et al. J Gen Virol. 74: 311-4, 1993
366. Walker et al. Virology. 190: 278-87, 1992
367. Wattrang et al. Viral Immunol. 16: 57-67, 2003
368. Webster Emerging Infectious Diseases 4, 1998
369. Webster et al. Vaccine. 11: 987-993, 1993
370. Wilbur et al. Proc Natl Acad Sci USA 80: 726-730, 1983
371. WO 00/03030
372. WO 01/05934
373. WO 90/01543
374. WO 91/11525
375. WO 94/16716
376. WO 96/34109
377. WO 96/39491
378. WO 98/16247
379. WO 98/33510
380. Wood et al. Arch. Virol. 130: 209-217, 1993
381. Xu, et al. Virology 261: 15-19, 1999
382. Yamane et al. Tohoku J Exp Med. 245-55,1981 1
383. Yamnikova et al. Virology. 197: 558-563, 1993
384. Youil et al. J Virol Methods 120: 23-31, 2004
385. Youil et al. Virus Res. 102: 165-176, 2004
386. Youngner et al. Am J Vet Res. 62: 1290-1294, 2001
387. Yuen et al. Proc Natl Acad Sci USA. 84: 6417-6421, 1987
388. Zakay-Rones et al. J Altern Complement Med. 1: 361-369, 1995
389. Zakstel'skaia et al. Vopr Virusol. 551-557, 1977
390. Zambon. Rev Med Virol. 11: 227-241, 2001
391. Zhukova et al. Acta Virol. 19: 281-286, 1975

SEQ ID NO 1
   LENGTH: 3659
   TYPE: DNA
   ORGANISM: Fowlpox virus
SEQUENCE:
SEQ ID NO 2
   LENGTH: 68
   TYPE: DNA
   ORGANISM: Artificial Sequence
   OTHER INFORMATION: Double-stranded oligonucleotide referred to as JCA017
   SEQUENCE:
SEQ ID NO 3
   LENGTH: 65
   TYPE: DNA
   ORGANISM: Artificial Sequence
   OTHER INFORMATION: Double-stranded oligonucleotide referred to as JCA018
   SEQUENCE: 38
SEQ ID NO 4
   LENGTH: 60
   TYPE: DNA
   ORGANISM: Artificial Sequence
   OTHER INFORMATION: Oligonucleotide referred to as RW178
   SEQUENCE:
      1 tcattatcgc gatatccgtg ttaactagct agctaatttt tattcccggg atccttatca
SEQ ID NO 5
   LENGTH: 60
   TYPE: DNA
   ORGANISM: Artificial Sequence
   OTHER INFORMATION: Oligonucleotide referred to as RW179
      1 gtataaggat cccgggaata aaaattagct agctagttaa cacggatatc gcgataatga
SEQ ID NO 6
   LENGTH: 66
   TYPE: DNA
   ORGANISM: Artificial Sequence
   OTHER INFORMATION: Synthetic oligonucleotide referred to as RW10
   SEQUENCE: 58
SEQ ID NO 7
   LENGTH: 66
   TYPE: DNA
   ORGANISM: Artificial Sequence
   OTHER INFORMATION: Synthetic oligonucleotide referred to as RW11
   SEQUENCE:
SEQ ID NO 8
   LENGTH: 87
   TYPE: DNA
   ORGANISM: Artificial Sequence
   OTHER INFORMATION: Synthetic oligonucleotide referred to as RW12
   SEQUENCE:
SEQ ID NO 9
   LENGTH: 49
   TYPE: DNA
   ORGANISM: Artificial Sequence
   OTHER INFORMATION: Synthetic oligonucleotide referred to as RW13
   SEQUENCE:
      1 taccacgcaa acaactcaac aaaacaggtc gactttaaat ttttctgca
SEQ ID NO 10
   LENGTH: 21
   ORGANISM: Artificial Sequence
   OTHER INFORMATION: Primer for amplifying AIV probe referred to as 11526JY
   SEQUENCE:
      1 ACGAAGCCAGCAGCGGAGTGA
SEQ ID NO 11
   LENGTH: 21
   ORGANISM: Artificial Sequence
   OTHER INFORMATION: Primer for amplifying AIV probe referred to as 11531JY
   SEQUENCE:
      1 TCAGCACCAGCAGTTCGGCGT
SEQ ID NO 12
   LENGTH: 24
   ORGANISM: Artificial Sequence
   OTHER INFORMATION: Primer for PCR amplification of the F8 arms plus insert, referred to as 11339CXL
   SEQUENCE:
      1 GTAGTGATCAAAATACAGAACCAT
SEQ ID NO 13
   LENGTH: 24
   ORGANISM: Artificial Sequence
   OTHER INFORMATION: Primer for PCR amplification of the F8 arms plus insert, referred to as 11340CXL
   SEQUENCE:
      1 GAATCCGTCATTCAACTTTCTAGT
SEQ ID NO 14
   LENGTH: 4737
   OTHER INFORMATION: F8 right arm (1-1429), H6 promoter (1516-1639), AIV synethetic H5 HA (1640-3334), and F8 left arm (3362-4737) determined through sequence analysis
   SEQUENCE:
SEQ ID NO 15
   LENGTH: 1692
   OTHER INFORMATION: nucleotide sequence of wild type H5 HA without cleavage sequence from plasmid pCR-Script/HA-CK/Indonesia/03-(modified)-avipox
   SEQUENCE:
SEQ ID NO 16
   LENGTH: 564
   OTHER INFORMATION: amino acid sequence of wild type H5 HA without cleavage sequence from plasmid pCR-Script/HA-CK/Indonesia/03-(modified)-avipox
   SEQUENCE:
SEQ ID NO 17
   LENGTH: 564
   OTHER INFORMATION: Predicted amino acid sequence of H5 HA without cleavage sequence
   SEQUENCE:
SEQ ID NO 18
   LENGTH: 3956
   ORGANISM: Artificial Sequence
   OTHER INFORMATION: Nucleotide sequence of C5 right arm (43-1578), H6 promoter (1675-1799) AIV synthetic H5 HA (1800-3494), and C5 left arm (3529-3932) in plasmid
   SEQUENCE:
SEQ ID NO 19
   LENGTH: 6551
   OTHER INFORMATION: Theoretical sequence of entire vector
   SEQUENCE:
SEQ ID NO 20
   LENGTH: 25
   OTHER INFORMATION: Primer for PCR amplification of the vCP2241 C5 arms plus insert, referred to as 7931.DC
   SEQUENCE:
      1 GAATCTGTTAGTTAGTTACTTGGAT
SEQ ID NO 21
   LENGTH: 25
   OTHER INFORMATION: Primer for PCR amplification of the vCP2241 C5 arms plus insert, referred to as 7932.DC
   SEQUENCE:
      1 TGATTATAGCTATTATCACAGACTC
SEQ ID NO 22
   LENGTH: ORGANISM: Artificial Sequence
   OTHER INFORMATION: C5 right arm (1-1536), H6 promoter (1634-1757), AIV synethetic H5 HA (1758-3452), and C5 left arm (3485-3890) determined through sequence analysis SEQUENCE:

### SEQUENCE LISTING

<110> MERIAL LIMITED
<120> FELINE VACCINES AGAINST AVIAN INFLUENZA
<130> MER 05-051.3PCT
<140> PCT/US07/083648
   <141> 2007-11-05
<150> 11/557,040
   <151> 2006-11-06
<150> 11/211,983
   <151> 2005-08-25
<160> 25
<170> PatentIn Ver. 3.3
<210> 1
   <211> 3659
   <212> DNA
   <213> Fowlpox virus
<400> 1
   gatatctgtg gtctatatat actacaccct accgatatta accaacgagt ttctcacaag 60
   aaaacttgtt tagtagatag agattctttg attgtgttta aaagaagtac cagtaaaaag 120
   tgtggcatat gcatagaaga aataaacaaa aaacatattt ccgaacagta ttttggaatt 180
   ctcccaagtt gtaaacatat tttttgccta tcatgtataa gacgttgggc agatactacc 240
   agaaatacag atactgaaaa tacgtgtcct gaatgtagaa tagtttttcc tttcataata 300
   cccagtaggt attggataga taataaatat gataaaaaaa tattatataa tagatataag 360
   aaaatgattt ttacaaaaat acctataaga acaataaaaa tataattaca tttacggaaa 420
   atagctggtt ttagtttacc aacttagagt aattatcata ttgaatctat attgtttttt 480
   agttatataa aaacatgatt agcccccaat cggatgaaaa tataaaagat gttgagaatt 540
   tcgaatacaa caaaaagagg aatcgtacgt tgtccatatc caaacatata aataaaaatt 600
   caaaagtagt attatactgg atgtttagag atcaacgtgt acaagataat tgggctttaa 660
   tttacgcaca acgattagcg ttaaaactca aaatacctct aagaatatgc ttttgtgtcg 720
   tgccaaaatt tcacactact acttctagac actttatgtt tttaatatcc ggtcttaaag 780
   aagtcgcgga agaatgtaaa agactatgta tagggttttc attgatatat ggcgtaccaa 840
   aagtaataat tccgtgtata gtaaaaaaat acagagtcgg agtaatcata acggatttct 900
   ttccattacg tgttcccgaa agattaatga aacagactgt aatatctctt ccagataaca 960
   taccttttat acaagtagac gctcataata tagtaccttg ttgggaagct tctgataaag 1020
   aagaatacgg tgcacgaact ttaagaaaaa agatatttga taaattatat gaatatatga 1080
   cagaatttcc tgttgttcgt aaacatccat acggtccatt ttctatatct attgcaaaac 1140
   ccaaaaatat atcattagac aagacggtat tacccgtaaa atgggcaacg cctggaacaa 1200
   aagctggaat aattgtttta aaagaattta taaaaaacag attaccgtca tacgacgcgg 1260
   atcataacaa tcctacgtgt gacgctttga gtaacttatc tccgtggcta cattttggtc 1320
   atgtatccgc acaacgtgtt gccttagaag tattaaaatg tatacgagaa agcaaaaaaa 1380
   acgttgaaac gtttatagat gaaataattg taagaagaga actatcggat aatttttgtt 1440
   actataacaa acattatgat agtatccagt ctactcattc atgggttaga aaaacattag 1500
   aagatcacat taatgatcct agaaagtata tatattccat taaacaactc gaaaaagcgg 1560
   aaactcatga tcctctatgg aacgcgtcac aaatgcagat ggtgagagaa ggaaaaatgc 1620
   atagtttttt acgaatgtat tgggctaaga agatacttga atggactaga acacctgaag 1680
   acgctttgag ttatagtatc tatttgaaca acaagtacga actagacggc acggatccta 1740
   acggatacgt aggttgtatg tggtctattt gcggattaca cgatagagcg tggaaagcaa 1800
   gaccgatatt tggaaagata agatatatga attatgagag ttctaagaag aaatttgatg 1860
   ttgctgtatt tatacagaaa tacaattaag ataaataata tacagcattg taaccatcgt 1920
   catccgttat acggggaata atattaccat acagtattat taaattttct tacgaagaat 1980
   atagatcggt atttatcgtt agtttatttt acatttatta attaaacatg tctactatta 2040
   cctgttatgg aaatgacaaa tttagttata taatttatga taaaattaag ataataataa 2100
   tgaaatcaaa taattatgta aatgctacta gattatgtga attacgagga agaaagttta 2160
   cgaactggaa aaaattaagt gaatctaaaa tattagtcga taatgtaaaa aaaataaatg 2220
   ataaaactaa ccagttaaaa acggatatga ttatatacgt taaggatatt gatcataaag 2280
   gaagagatac ttgcggttac tatgtacacc aagatctggt atcttctata tcaaattgga 2340
   tatctccgtt attcgccgtt aaggtaaata aaattattaa ctattatata tgtaatgaat 2400
   atgatatacg acttagcgaa atggaatctg atatgacaga agtaatagat gtagttgata 2460
   aattagtagg aggatacaat gatgaaatag cagaaataat atatttgttt aataaattta 2520
   tagaaaaata tattgctaac atatcgttat caactgaatt atctagtata ttaaataatt 2580
   ttataaattt tataaatttt aataaaaaat acaataacga cataaagata tttaatcttt 2640
   aattcttgat ctgaaaaaca catctataaa actagataaa aagttattcg ataaagataa 2700
   taatgaatcg aacgatgaaa aattggaaac agaagttgat aagctaattt ttttcatcta 2760
   aatagtatta ttttattgaa gtacgaagtt ttacgttaga taaataataa aggtcgattt 2820
   ttactttgtt aaatatcaaa tatgtcatta tctgataaag atacaaaaac acacggtgat 2880
   tatcaaccat ctaacgaaca gatattacaa aaaatacgtc ggactatgga aaacgaagct 2940
   gatagcctca atagaagaag cattaaagaa attgttgtag atgttatgaa gaattgggat 3000
   catcctcaac gaagaaatag ataaagttct aaactggaaa aatgatacat taaacgattt 3060
   agatcatcta aatacagatg ataatattaa ggaaatcata caatgtctga ttagagaatt 3120
   tgcgtttaaa aagatcaatt ctattatgta tagttatgct atggtaaaac tcaattcaga 3180
   taacgaacat tgaaagataa aattaaggat tattttatag aaactattct taaagacaaa 3240
   cgtggttata aacaaaagcc attacccgga ttggaaacta aaatactaga tagtattata 3300
   agattttaaa aacataaaat taataggttt ttatagattg acttattata tacaatatgg 3360
   ataaaagata tatatcaact agaaagttga atgacggatt cttaatttta tattatgatt 3420
   caatagaaat tattgtcatg tcgtgtaatc attttataaa tatatcagcg ttactagcta 3480
   agaaaaacaa ggactttaat gaatggctaa agatagaatc atttagagaa ataatagata 3540
   ctttagataa aattaattac gatctaggac aacgatattg tgaagaactt acggcgcatc 3600
   acattccagt gtaattattg aggtcaaagc tagtaactta atagatgaca ggacagctg  3659
<210> 2
   <211> 68
   <212> DNA
   <213> Artificial Sequence
<220>
   <223> Description of Artificial Sequence: Synthetic oligonucleotide
<400> 2
   ctagacactt tatgtttttt aatatccggt cttaaaagct tcccggggat ccttatacgg 60
   ggaataat                                                          68
<210> 3
   <211> 65
   <212> DNA
   <213> Artificial Sequence
<220>
   <223> Description of Artificial Sequence: Synthetic oligonucleotide
<400> 3
   attattcccc gtataaggat cccccgggaa gcttttaaga ccggatatta aaaaacataa 60
   agtgt                                                             65
<210> 4
   <211> 60
   <212> DNA
   <213> Artificial Sequence
<220>
   <223> Description of Artificial Sequence: Synthetic oligonucleotide
<400> 4
   tcattatcgc gatatccgtg ttaactagct agctaatttt tattcccggg atccttatca 60
<210> 5
   <211> 60
   <212> DNA
   <213> Artificial Sequence
<220>
   <223> Description of Artificial Sequence: Synthetic oligonucleotide
<400> 5
   gtataaggat cccgggaata aaaattagct agctagttaa cacggatatc gcgataatga 60
<210> 6
   <211> 66
   <212> DNA
   <213> Artificial Sequence
<220>
   <223> Description of Artificial Sequence: Synthetic oligonucleotide
<400> 6
   gaaaaattta aagtcgacct gttttgttga gttgtttgcg tggtaaccaa tgcaaatctg 60
   gtcact 66
<210> 7
   <211> 66
   <212> DNA
   <213> Artificial Sequence
<220>
   <223> Description of Artificial Sequence: Synthetic oligonucleotide
<400> 7
   tctagcaaga ctgactattg caaaaagaag cactatttcc tccattacga tacaaactta 60
   acggat 66
<210> 8
   <211> 87
   <212> DNA
   <213> Artificial Sequence
<220>
   <223> Description of Artificial Sequence: Synthetic oligonucleotide
<400> 8
   atccgttaag tttgtatcgt aatggaggaa atagtgcttc tttttgcaat agtcagtctt 60
   gctagaagtg accagatttg cattggt 87
<210> 9
   <211> 49
   <212> DNA
   <213> Artificial Sequence
<220>
   <223> Description of Artificial Sequence: Synthetic oligonucleotide
<400> 9
   taccacgcaa acaactcaac aaaacaggtc gactttaaat ttttctgca 49
<210> 10
   <211> 21
   <212> DNA
   <213> Artificial Sequence
<220>
   <223> Description of Artificial Sequence: Synthetic primer
<400> 10
   acgaagccag cagcggagtg a 21
<210> 11
   <211> 21
   <212> DNA
   <213> Artificial Sequence
<220>
   <223> Description of Artificial Sequence: Synthetic primer
<400> 11
   tcagcaccag cagttcggcg t 21
<210> 12
   <211> 24
   <212> DNA
   <213> Artificial Sequence
<220>
   <223> Description of Artificial Sequence: Synthetic primer
<400> 12
   gtagtgatca aaatacagaa ccat 24
<210> 13
   <211> 24
   <212> DNA
   <213> Artificial Sequence
<220>
   <223> Description of Artificial Sequence: Synthetic primer
<400> 13
   gaatccgtca ttcaactttc tagt 24
<210> 14
   <211> 4737
   <212> DNA
   <213> Artificial Sequence
<220>
   <223> Description of Artificial Sequence: Synthetic construct
<400> 14
   gaccctttac aagaataaaa gaagaaacaa ctgtgaaata gtttataaat gtaattcgta 60
   tgcagaaaac gataatatat tttggtatga gaaatctaaa ggagacatag tttgtataga 120
   catgcgctct tccgatgaga tattcgatgc ttttctaatg tatcatatag ctacaagata 180
   tgcctatcat gatgatgata tatatctaca aatagtgtta tattattcta ataatcaaaa 240
   tgttatatct tatattacga aaaataaata cgttaagtat ataagaaata aaactagaga 300
   cgatattcat aaagtaaaaa tattagctct agaagacttt acaacggaag aaatatattg 360
   ttggattagt aatatataac agcgtagctg cacggttttg atcattttcc aacaatataa 420
   accaatgaag gaggacgact catcaaacat aaataacatt cacggaaaat attcagtatc 480
   agatttatca caagatgatt atgttattga atgtatagac ggatcttttg attcgatcaa 540
   gtatagagat ataaaggtta taataatgaa gaataacggt tacgttaatt gtagtaaatt 600
   atgtaaaatg cggaataaat acttttctag atggttgcgt ctttctactt ctaaagcatt 660
   attagacatt tacaataata agtcagtaga taatgctatt gttaaagtct atggtaaagg 720
   taagaaactt attataacag gattttatct caaacaaaat atgatacgtt atgttattga 780
   gtggataggg gatgatttta caaacgatat atacaaaatg attaatttct ataatgcgtt 840
   attcggtaac gatgaattaa aaatagtatc ctgtgaaaac actctatgcc cgtttataga 900
   acttggtaga tgctattatg gtaaaaaatg taagtatata cacggagatc aatgtgatat 960
   ctgtggtcta tatatactac accctaccga tattaaccaa cgagtttctc acaagaaaac 1020
   ttgtttagta gatagagatt ctttgattgt gtttaaaaga agtaccagta aaaagtgtgg 1080
   catatgcata gaagaaataa acaaaaaaca tatttccgaa cagtattttg gaattctccc 1140
   aagttgtaaa catatttttt gcctatcatg tataagacgt tgggcagata ctaccagaaa 1200
   tacagatact gaaaatacgt gtcctgaatg tagaatagtt tttcctttca taatacccag 1260
   taggtattgg atagataata aatatgataa aaaaatatta tataatagat ataagaaaat 1320
   gatttttaca aaaataccta taagaacaat aaaaatataa ttacatttac ggaaaatagc 1380
   tggttttagt ttaccaactt agagtaatta tcatattgaa tctatattgc taattagcta 1440
   ataaaaaccc gggttaatta attagtcatc aggcagggcg agaacgagac tatctgctcg 1500
   ttaattaatt agagcttctt tattctatac ttaaaaagtg aaaataaata caaaggttct 1560
   tgagggttgt gttaaattga aagcgagaaa taatcataaa ttatttcatt atcgcgatat 1620
   ccgttaagtt tgtatcgtaa tggagaaaat cgtgctgctg ctggccatcg tgagcctggt 1680
   gaaaagcgat cagatctgca tcggctacca cgccaacaac agcacagagc aagtggacac 1740
   aatcatggaa aagaacgtga ccgtgacaca cgcccaggac atcctggaaa agacacacaa 1800
   cgggaagctg tgcgatctgg atggagtgaa gcctctgatc ctgagagatt gcagcgtggc 1860
   cggatggctg ctggggaacc caatgtgcga cgaattcatc aacgtgcccg aatggagcta 1920
   catcgtggag aaggccaacc cagccaacga cctgtgctac ccagggaacc tgaacgacta 1980
   cgaagaactg aaacacctgc tgagcagaat caaccacttt gagaaaatcc agatcatccc 2040
   caaaagcagc tggtccgatc acgaagccag cagcggagtg agcagcgcct gcccatacca 2100
   gggaaagtcc agctttttta gaaacgtggt gtggctgatc aaaaagaaca gcgcctaccc 2160
   aacaatcaag agaagctaca acaacaccaa ccaggaagat ctgctggtgc tgtgggggat 2220
   ccaccaccct aacgatgccg ccgagcagac aaggctgtac cagaacccaa ccacctacat 2280
   ctccgtgggg acaagcacac tgaaccagag actggtgcca aaaatcgcca tcagatccaa 2340
   agtgaacggg cagagcggaa gaatggagtt cttctggaca atcctgaaac ccaacgatgc 2400
   catcaacttc gagagcaacg gaaacttcat cgccccagaa tacgcctaca aaatcgtgaa 2460
   gaaaggggac agcgccatca tgaaaagcga actggaatac ggcaactgca acaccaagtg 2520
   ccagacccca atgggggcca tcaacagcag catgccattc cacaacatcc accctctgac 2580
   catcggggaa tgccccaaat acgtgaaaag caacagactg gtgctggcca ccgggctgag 2640
   aaacagccct cagagagaga ccagaggact gtttggagcc atcgccggct ttatcgaggg 2700
   aggatggcag ggaatggtgg atggctggta cggataccac cacagcaacg agcaggggag 2760
   cggatacgcc gccgacaaag aatccaccca gaaggccatc gacggcgtga ccaacaaagt 2820
   gaacagcatc atcgacaaaa tgaacaccca gtttgaggcc gtgggaaggg agtttaacaa 2880
   cctggaaagg agaatcgaga acctgaacaa gaagatggag gacggattcc tggatgtgtg 2940
   gacctacaac gccgaactgc tggtgctgat ggaaaacgag agaaccctgg actttcacga 3000
   cagcaacgtg aagaacctgt acgacaaagt gaggctgcag ctgagggata acgccaagga 3060
   gctgggcaac ggctgcttcg agttctacca caaatgcgat aacgaatgca tggaaagcat 3120
   cagaaacgga acctacaact acccccagta cagcgaagaa gccagactga aaagagaaga 3180
   aatctccgga gtgaaactgg aatccatcgg aacctaccag atcctgagca tctacagcac 3240
   agtggcctcc tccctggccc tggccatcat gatggccgga ctgagcctgt ggatgtgctc 3300
   caacggaagc ctgcagtgca gaatctgcat ctgactcgag tttttattga ctagttaatc 3360
   ataagataaa taatatacag cattgtaacc atcgtcatcc gttatacggg gaataatatt 3420
   accatacagt attattaaat tttcttacga agaatataga tcggtattta tcgttagttt 3480
   attttacatt tattaattaa acatgtctac tattacctgt tatggaaatg acaaatttag 3540
   ttatataatt tatgataaaa ttaagataat aataatgaaa tcaaataatt atgtaaatgc 3600
   tactagatta tgtgaattac gaggaagaaa gtttacgaac tggaaaaaat taagtgaatc 3660
   taaaatatta gtcgataatg taaaaaaaat aaatgataaa actaaccagt taaaaacgga 3720
   tatgattata tacgttaagg atattgatca taaaggaaga gatacttgcg gttactatgt 3780
   acaccaagat ctggtatctt ctatatcaaa ttggatatct ccgttattcg ccgttaaggt 3840
   aaataaaatt attaactatt atatatgtaa tgaatatgat atacgactta gcgaaatgga 3900
   atctgatatg acagaagtaa tagatgtagt tgataaatta gtaggaggat acaatgatga 3960
   aatagcagaa ataatatatt tgtttaataa atttatagaa aaatatattg ctaacatatc 4020
   gttatcaact gaattatcta gtatattaaa taattttata aattttaata aaaaatacaa 4080
   taacgacata aaagatatta aatctttaat tcttgatctg aaaaacacat ctataaaact 4140
   agataaaaag ttattcgata aagataataa tgaatcgaac gatgaaaaat tggaaacaga 4200
   agttgataag ctaatttttt tcatctaaat agtattattt tattgaagta cgaagtttta 4260
   cgttagataa ataataaagg tcgattttta ttttgttaaa tatcaaatat gtcattatct 4320
   gataaagata caaaaacaca cggtgattat caaccatcta acgaacagat attacaaaaa 4380
   atacgtcgga ctatggaaaa cgaagctgat agcctcaata gaagaagcat taaagaaatt 4440
   gttgtagatg ttatgaagaa ttgggatcat cctctcaacg aagaaataga taaagttcta 4500
   aactggaaaa atgatacatt aaacgattta gatcatctaa atacagatga taatattaag 4560
   gaaatcatac aatgtctgat tagagaattt gcgtttaaaa agatcaattc tattatgtat 4620
   agttatgcta tggtaaaact caattcagat aacgaaacat tgaaagataa aattaaggat 4680
   tattttatag aaactattct taaagacaaa cgtggttata aacaaaagcc attaccc 4737
<210> 15
   <211> 1692
   <212> DNA
   <213> Artificial Sequence
<220>
   <223> Description of Artificial Sequence: Synthetic construct
<400> 15
   atggagaaaa tcgtgctgct gctggccatc gtgagcctgg tgaaaagcga tcagatctgc 60
   atcggctacc acgccaacaa cagcacagag caagtggaca caatcatgga aaagaacgtg 120
   accgtgacac acgcccagga catcctggaa aagacacaca acgggaagct gtgcgatctg 180
   gatggagtga agcctctgat cctgagagat tgcagcgtgg ccggatggct gctggggaac 240
   ccaatgtgcg acgaattcat caacgtgccc gaatggagct acatcgtgga gaaggccaac 300
   ccagccaacg acctgtgcta cccagggaac ctgaacgact acgaagaact gaaacacctg 360
   ctgagcagaa tcaaccactt tgagaaaatc cagatcatcc ccaaaagcag ctggtccgat 420
   cacgaagcca gcagcggagt gagcagcgcc tgcccatacc agggaaagtc cagctttttt 480
   agaaacgtgg tgtggctgat caaaaagaac agcgcctacc caacaatcaa gagaagctac 540
   aacaacacca accaggaaga tctgctggtg ctgtggggga tccaccaccc taacgatgcc 600
   gccgagcaga caaggctgta ccagaaccca accacctaca tctccgtggg gacaagcaca 660
   ctgaaccaga gactggtgcc aaaaatcgcc atcagatcca aagtgaacgg gcagagcgga 720
   agaatggagt tcttctggac aatcctgaaa cccaacgatg ccatcaactt cgagagcaac 780
   ggaaacttca tcgccccaga atacgcctac aaaatcgtga agaaagggga cagcgccatc 840
   atgaaaagcg aactggaata cggcaactgc aacaccaagt gccagacccc aatgggggcc 900
   atcaacagca gcatgccatt ccacaacatc caccctctga ccatcgggga atgccccaaa 960
   tacgtgaaaa gcaacagact ggtgctggcc accgggctga gaaacagccc tcagagagag 1020
   accagaggac tgtttggagc catcgccggc tttatcgagg gaggatggca gggaatggtg 1080
   gatggctggt acggatacca ccacagcaac gagcagggga gcggatacgc cgccgacaaa 1140
   gaatccaccc agaaggccat cgacggcgtg accaacaaag tgaacagcat catcgacaaa 1200
   atgaacaccc agtttgaggc cgtgggaagg gagtttaaca acctggaaag gagaatcgag 1260
   aacctgaaca agaagatgga ggacggattc ctggatgtgt ggacctacaa cgccgaactg 1320
   ctggtgctga tggaaaacga gagaaccctg gactttcacg acagcaacgt gaagaacctg 1380
   tacgacaaag tgaggctgca gctgagggat aacgccaagg agctgggcaa cggctgcttc 1440
   gagttctacc acaaatgcga taacgaatgc atggaaagca tcagaaacgg aacctacaac 1500
   tacccccagt acagcgaaga agccagactg aaaagagaag aaatctccgg agtgaaactg 1560
   gaatccatcg gaacctacca gatcctgagc atctacagca cagtggcctc ctccctggcc 1620
   ctggccatca tgatggccgg actgagcctg tggatgtgct ccaacggaag cctgcagtgc 1680
   agaatctgca tc 1692
<210> 16
   <211> 564
   <212> PRT
   <213> Artificial Sequence
<220>
   <223> Description of Artificial Sequence: Synthetic construct
<400> 16
   Met Glu Lys Ile Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asp Leu Asp Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Ile Asn Val Pro Glu Trp Ser Tyr Ile Val
   85 90 95
   Glu Lys Ala Asn Pro Ala Asn Asp Leu Cys Tyr Pro Gly Asn Leu Asn
   100 105 110
   Asp Tyr Glu Glu Leu Lys His Leu Leu Ser Arg Ile Asn His Phe Glu
   115 120 125
   Lys Ile Gln Ile Ile Pro Lys Ser Ser Trp Ser Asp His Glu Ala Ser
   130 135 140
   Ser Gly Val Ser Ser Ala Cys Pro Tyr Gln Gly Lys Ser Ser Phe Phe
   145 150 155 160
   Arg Asn Val Val Trp Leu Ile Lys Lys Asn Ser Ala Tyr Pro Thr Ile
   165 170 175
   Lys Arg Ser Tyr Asn Asn Thr Asn Gln Glu Asp Leu Leu Val Leu Trp
   180 185 190
   Gly Ile His His Pro Asn Asp Ala Ala Glu Gln Thr Arg Leu Tyr Gln
   195 200 205
   Asn Pro Thr Thr Tyr Ile Ser Val Gly Thr Ser Thr Leu Asn Gln Arg
   210 215 220
   Leu Val Pro Lys Ile Ala Ile Arg Ser Lys Val Asn Gly Gln Ser Gly
   225 230 235 240
   Arg Met Glu Phe Phe Trp Thr Ile Leu Lys Pro Asn Asp Ala Ile Asn
   245 250 255
   Phe Glu Ser Asn Gly Asn Phe Ile Ala Pro Glu Tyr Ala Tyr Lys Ile
   260 265 270
   Val Lys Lys Gly Asp Ser Ala Ile Met Lys Ser Glu Leu Glu Tyr Gly
   275 280 285
   Asn Cys Asn Thr Lys Cys Gln Thr Pro Met Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Arg Glu Thr Arg Gly Leu Phe Gly Ala Ile Ala Gly Phe Ile
   340 345 350
   Glu Gly Gly Trp Gln Gly Met Val Asp Gly Trp Tyr Gly Tyr His His
   355 360 365
   Ser Asn Glu Gln Gly Ser Gly Tyr Ala Ala Asp Lys Glu Ser Thr Gln
   370 375 380
   Lys Ala Ile Asp Gly Val Thr Asn Lys Val Asn Ser Ile Ile Asp Lys
   385 390 395 400
   Met Asn Thr Gln Phe Glu Ala Val Gly Arg Glu Phe Asn Asn Leu Glu
   405 410 415
   Arg Arg Ile Glu Asn Leu Asn Lys Lys Met Glu Asp Gly Phe Leu Asp
   420 425 430
   Val Trp Thr Tyr Asn Ala Glu Leu Leu Val Leu Met Glu Asn Glu Arg
   435 440 445
   Thr Leu Asp Phe His Asp Ser Asn Val Lys Asn Leu Tyr Asp Lys Val
   450 455 460
   Arg Leu Gln Leu Arg Asp Asn Ala Lys Glu Leu Gly Asn Gly Cys Phe
   465 470 475 480
   Glu Phe Tyr His Lys Cys Asp Asn Glu Cys Met Glu Ser Ile Arg Asn
   485 490 495
   Gly Thr Tyr Asn Tyr Pro Gln Tyr Ser Glu Glu Ala Arg Leu Lys Arg
   500 505 510
   Glu Glu Ile Ser Gly Val Lys Leu Glu Ser Ile Gly Thr Tyr Gln Ile
   515 520 525
   Leu Ser Ile Tyr Ser Thr Val Ala Ser Ser Leu Ala Leu Ala Ile Met
   530 535 540
   Met Ala Gly Leu Ser Leu Trp Met Cys Ser Asn Gly Ser Leu Gln Cys
   545 550 555 560
   Arg Ile Cys Ile
<210> 17
   <211> 564
   <212> PRT
   <213> Artificial Sequence
<220>
   <223> Description of Artificial Sequence: Synthetic construct
<400> 17
   Met Glu Lys Ile Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asp Leu Asp Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Ile Asn Val Pro Glu Trp Ser Tyr Ile Val
   85 90 95
   Glu Lys Ala Asn Pro Ala Asn Asp Leu Cys Tyr Pro Gly Asn Leu Asn
   100 105 110
   Asp Tyr Glu Glu Leu Lys His Leu Leu Ser Arg Ile Asn His Phe Glu
   115 120 125
   Lys Ile Gln Ile Ile Pro Lys Ser Ser Trp Ser Asp His Glu Ala Ser
   130 135 140
   Ser Gly Val Ser Ser Ala Cys Pro Tyr Gln Gly Lys Ser Ser Phe Phe
   145 150 155 160
   Arg Asn Val Val Trp Leu Ile Lys Lys Asn Ser Ala Tyr Pro Thr Ile
   165 170 175
   Lys Arg Ser Tyr Asn Asn Thr Asn Gln Glu Asp Leu Leu Val Leu Trp
   180 185 190
   Gly Ile His His Pro Asn Asp Ala Ala Glu Gln Thr Arg Leu Tyr Gln
   195 200 205
   Asn Pro Thr Thr Tyr Ile Ser Val Gly Thr Ser Thr Leu Asn Gln Arg
   210 215 220
   Leu Val Pro Lys Ile Ala Ile Arg Ser Lys Val Asn Gly Gln Ser Gly
   225 230 235 240
   Arg Met Glu Phe Phe Trp Thr Ile Leu Lys Pro Asn Asp Ala Ile Asn
   245 250 255
   Phe Glu Ser Asn Gly Asn Phe Ile Ala Pro Glu Tyr Ala Tyr Lys Ile
   260 265 270
   Val Lys Lys Gly Asp Ser Ala Ile Met Lys Ser Glu Leu Glu Tyr Gly
   275 280 285
   Asn Cys Asn Thr Lys Cys Gln Thr Pro Met Gly Ala Ile Asn Ser Ser
   290 295 300
   Met Pro Phe His Asn Ile His Pro Leu Thr Ile Gly Glu Cys Pro Lys
   305 310 315 320
   Tyr Val Lys Ser Asn Arg Leu Val Leu Ala Thr Gly Leu Arg Asn Ser
   325 330 335
   Pro Gln Arg Glu Thr Arg Gly Leu Phe Gly Ala Ile Ala Gly Phe Ile
   340 345 350
   Glu Gly Gly Trp Gln Gly Met Val Asp Gly Trp Tyr Gly Tyr His His
   355 360 365
   Ser Asn Glu Gln Gly Ser Gly Tyr Ala Ala Asp Lys Glu Ser Thr Gln
   370 375 380
   Lys Ala Ile Asp Gly Val Thr Asn Lys Val Asn Ser Ile Ile Asp Lys
   385                 390                 395                 400
   Met Asn Thr Gln Phe Glu Ala Val Gly Arg Glu Phe Asn Asn Leu Glu
                   405                 410                 415
   Arg Arg Ile Glu Asn Leu Asn Lys Lys Met Glu Asp Gly Phe Leu Asp
               420                 425                 430
   Val Trp Thr Tyr Asn Ala Glu Leu Leu Val Leu Met Glu Asn Glu Arg
           435                 440                 445
   Thr Leu Asp Phe His Asp Ser Asn Val Lys Asn Leu Tyr Asp Lys Val
       450                 455                 460
   Arg Leu Gln Leu Arg Asp Asn Ala Lys Glu Leu Gly Asn Gly Cys Phe
   465                 470                 475                 480
   Glu Phe Tyr His Lys Cys Asp Asn Glu Cys Met Glu Ser Ile Arg Asn
                   485                 490                 495
   Gly Thr Tyr Asn Tyr Pro Gln Tyr Ser Glu Glu Ala Arg Leu Lys Arg
               500                 505                 510
   Glu Glu Ile Ser Gly Val Lys Leu Glu Ser Ile Gly Thr Tyr Gln Ile
           515                 520                 525
   Leu Ser Ile Tyr Ser Thr Val Ala Ser Ser Leu Ala Leu Ala Ile Met
       530                 535                 540
   Met Ala Gly Leu Ser Leu Trp Met Cys Ser Asn Gly Ser Leu Gln Cys
   545                 550                 555                 560
   Arg Ile Cys Ile
<210> 18
   <211> 3966
   <212> DNA
   <213> Artificial Sequence
<220>
   <221> CDS
   <222> (1800)..(3491)
<220>
   <223> Description of Artificial Sequence: Synthetic construct
<400> 18
   ggaaacagct atgaccatga ttacgaattg cggccgcaat tctgaatgtt aaatgttata 60
   ctttggatga agctataaat atgcattgga aaaataatcc atttaaagaa aggattcaaa 120
   tactacaaaa cctaagcgat aatatgttaa ctaagcttat tcttaacgac gctttaaata 180
   tacacaaata aacataattt ttgtataacc taacaaataa ctaaaacata aaaataataa 240
   aaggaaatgt aatatcgtaa ttattttact caggaatggg gttaaatatt tatatcacgt 300
   gtatatctat actgttatcg tatactcttt acaattacta ttacgaatat gcaagagata 360
   ataagattac gtatttaaga gaatcttgtc atgataattg ggtacgacat agtgataaat 420
   gctatttcgc atcgttacat aaagtcagtt ggaaagatgg atttgacaga tgtaacttaa 480
   taggtgcaaa aatgttaaat aacagcattc tatcggaaga taggatacca gttatattat 540
   acaaaaatca ctggttggat aaaacagatt ctgcaatatt cgtaaaagat gaagattact 600
   gcgaatttgt aaactatgac aataaaaagc catttatctc aacgacatcg tgtaattctt 660
   ccatgtttta tgtatgtgtt tcagatatta tgagattact ataaactttt tgtatactta 720
   tattccgtaa actatattaa tcatgaagaa aatgaaaaag tatagaagct gttcacgagc 780
   ggttgttgaa aacaacaaaa ttatacattc aagatggctt acatatacgt ctgtgaggct 840
   atcatggata atgacaatgc atctctaaat aggtttttgg acaatggatt cgaccctaac 900
   acggaatatg gtactctaca atctcctctt gaaatggctg taatgttcaa gaataccgag 960
   gctataaaaa tcttgatgag gtatggagct aaacctgtag ttactgaatg cacaacttct 1020
   tgtctgcatg atgcggtgtt gagagacgac tacaaaatag tgaaagatct gttgaagaat 1080
   aactatgtaa acaatgttct ttacagcgga ggctttactc ctttgtgttt ggcagcttac 1140
   cttaacaaag ttaatttggt taaacttcta ttggctcatt cggcggatgt agatatttca 1200
   aacacggatc ggttaactcc tctacatata gccgtatcaa ataaaaattt aacaatggtt 1260
   aaacttctat tgaacaaagg tgctgatact gacttgctgg ataacatggg acgtactcct 1320
   ttaatgatcg ctgtacaatc tggaaatatt gaaatatgta gcacactact taaaaaaaat 1380
   aaaatgtcca gaactgggaa aaattgatct tgccagctgt aattcatggt agaaaagaag 1440
   tgctcaggct acttttcaac aaaggagcag atgtaaacta catctttgaa agaaatggaa 1500
   aatcatatac tgttttggaa ttgattaaag aaagttactc tgagacacaa aagaggtagc 1560
   tgaagtggta ctctcaaagg tacgtgacta attagctata aaaaggatcc gggttaatta 1620
   attagtcatc aggcagggcg agaacgagac tatctgctcg ttaattaatt agagcttctt 1680
   tattctatac ttaaaaagtg aaaataaata caaaggttct tgagggttgt gttaaattga 1740
   aagcgagaaa taatcataaa ttatttcatt atcgcgatat ccgttaagtt tgtatcgta 1799
   atg gag aaa atc gtg ctg ctg ctg gcc atc gtg agc ctg gtg aaa agc 1847
   Met Glu Lys Ile Val Leu Leu Leu Ala Ile Val Ser Leu Val Lys Ser
   1 5 10 15
   gat cag atc tgc atc ggc tac cac gcc aac aac agc aca gag caa gtg 1895
   Asp Gln Ile Cys Ile Gly Tyr His Ala Asn Asn Ser Thr Glu Gln Val
   20 25 30
   gac aca atc atg gaa aag aac gtg acc gtg aca cac gcc cag gac atc 1943
   Asp Thr Ile Met Glu Lys Asn Val Thr Val Thr His Ala Gln Asp Ile
   35 40 45
   ctg gaa aag aca cac aac ggg aag ctg tgc gat ctg gat gga gtg aag 1991
   Leu Glu Lys Thr His Asn Gly Lys Leu Cys Asp Leu Asp Gly Val Lys
   50 55 60
   cct ctg atc ctg aga gat tgc agc gtg gcc gga tgg ctg ctg ggg aac 2039
   Pro Leu Ile Leu Arg Asp Cys Ser Val Ala Gly Trp Leu Leu Gly Asn
   65 70 75 80
   cca atg tgc gac gaa ttc atc aac gtg ccc gaa tgg agc tac atc gtg     2087
   Pro Met Cys Asp Glu Phe Ile Asn Val Pro Glu Trp Ser Tyr Ile Val
                   85                  90                  95
   gag aag gcc aac cca gcc aac gac ctg tgc tac cca ggg aac ctg aac     2135
   Glu Lys Ala Asn Pro Ala Asn Asp Leu Cys Tyr Pro Gly Asn Leu Asn
               100                 105                 110
   gac tac gaa gaa ctg aaa cac ctg ctg agc aga atc aac cac ttt gag     2183
   Asp Tyr Glu Glu Leu Lys His Leu Leu Ser Arg Ile Asn His Phe Glu
           115                 120                 125
   aaa atc cag atc atc ccc aaa agc agc tgg tcc gat cac gaa gcc agc     2231
   Lys Ile Gln Ile Ile Pro Lys Ser Ser Trp Ser Asp His Glu Ala Ser
       130                 135                 140
   agc gga gtg agc agc gcc tgc cca tac cag gga aag tcc agc ttt ttt     2279
   Ser Gly Val Ser Ser Ala Cys Pro Tyr Gln Gly Lys Ser Ser Phe Phe
   145                 150                 155                 160
   aga aac gtg gtg tgg ctg atc aaa aag aac agc gcc tac cca aca atc     2327
   Arg Asn Val Val Trp Leu Ile Lys Lys Asn Ser Ala Tyr Pro Thr Ile
                   165                 170                 175
   aag aga agc tac aac aac acc aac cag gaa gat ctg ctg gtg ctg tgg     2375
   Lys Arg Ser Tyr Asn Asn Thr Asn Gln Glu Asp Leu Leu Val Leu Trp
               180                 185                 190
   ggg atc cac cac cct aac gat gcc gcc gag cag aca agg ctg tac cag     2423
   Gly Ile His His Pro Asn Asp Ala Ala Glu Gln Thr Arg Leu Tyr Gln
           195                 200                 205
   aac cca acc acc tac atc tcc gtg ggg aca agc aca ctg aac cag aga     2471
   Asn Pro Thr Thr Tyr Ile Ser Val Gly Thr Ser Thr Leu Asn Gln Arg
       210                 215                 220
   ctg gtg cca aaa atc gcc atc aga tcc aaa gtg aac ggg cag agc gga     2519
   Leu Val Pro Lys Ile Ala Ile Arg Ser Lys Val Asn Gly Gln Ser Gly
   225                 230                 235                 240
   aga atg gag ttc ttc tgg aca atc ctg aaa ccc aac gat gcc atc aac     2567
   Arg Met Glu Phe Phe Trp Thr Ile Leu Lys Pro Asn Asp Ala Ile Asn
                   245                 250                 255
   ttc gag agc aac gga aac ttc atc gcc cca gaa tac gcc tac aaa atc     2615
   Phe Glu Ser Asn Gly Asn Phe Ile Ala Pro Glu Tyr Ala Tyr Lys Ile
               260                 265                 270
   gtg aag aaa ggg gac agc gcc atc atg aaa agc gaa ctg gaa tac ggc     2663
   Val Lys Lys Gly Asp Ser Ala Ile Met Lys Ser Glu Leu Glu Tyr Gly
           275                 280                 285
   aac tgc aac acc aag tgc cag acc cca atg ggg gcc atc aac agc agc     2711
   Asn Cys Asn Thr Lys Cys Gln Thr Pro Met Gly Ala Ile Asn Ser Ser
       290                 295                 300
   atg cca ttc cac aac atc cac cct ctg acc atc ggg gaa tgc ccc aaa     2759
   Met Pro Phe His Asn Ile His Pro Leu Thr Ile Gly Glu Cys Pro Lys
   305                 310                 315                 320
   tac gtg aaa agc aac aga ctg gtg ctg gcc acc ggg ctg aga aac agc     2807
   Tyr Val Lys Ser Asn Arg Leu Val Leu Ala Thr Gly Leu Arg Asn Ser
                   325                 330                 335
   cct cag aga gag acc aga gga ctg ttt gga gcc atc gcc ggc ttt atc    2855
   Pro Gln Arg Glu Thr Arg Gly Leu Phe Gly Ala Ile Ala Gly Phe Ile
               340                 345                 350
   gag gga gga tgg cag gga atg gtg gat ggc tgg tac gga tac cac cac    2903
   Glu Gly Gly Trp Gln Gly Met Val Asp Gly Trp Tyr Gly Tyr His His
           355                 360                 365
   agc aac gag cag ggg agc gga tac gcc gcc gac aaa gaa tcc acc cag    2951
   Ser Asn Glu Gln Gly Ser Gly Tyr Ala Ala Asp Lys Glu Ser Thr Gln
       370                 375                 380
   aag gcc atc gac ggc gtg acc aac aaa gtg aac agc atc atc gac aaa    2999
   Lys Ala Ile Asp Gly Val Thr Asn Lys Val Asn Ser Ile Ile Asp Lys
   385                 390                 395                 400
   atg aac acc cag ttt gag gcc gtg gga agg gag ttt aac aac ctg gaa    3047
   Met Asn Thr Gln Phe Glu Ala Val Gly Arg Glu Phe Asn Asn Leu Glu
                   405                 410                 415
   agg aga atc gag aac ctg aac aag aag atg gag gac gga ttc ctg gat    3095
   Arg Arg Ile Glu Asn Leu Asn Lys Lys Met Glu Asp Gly Phe Leu Asp
               420                 425                 430
   gtg tgg acc tac aac gcc gaa ctg ctg gtg ctg atg gaa aac gag aga    3143
   Val Trp Thr Tyr Asn Ala Glu Leu Leu Val Leu Met Glu Asn Glu Arg
           435                 440                 445
   acc ctg gac ttt cac gac agc aac gtg aag aac ctg tac gac aaa gtg    3191
   Thr Leu Asp Phe His Asp Ser Asn Val Lys Asn Leu Tyr Asp Lys Val
       450                 455                 460
   agg ctg cag ctg agg gat aac gcc aag gag ctg ggc aac ggc tgc ttc    3239
   Arg Leu Gln Leu Arg Asp Asn Ala Lys Glu Leu Gly Asn Gly Cys Phe
   465                 470                 475                 480
   gag ttc tac cac aaa tgc gat aac gaa tgc atg gaa agc atc aga aac    3287
   Glu Phe Tyr His Lys Cys Asp Asn Glu Cys Met Glu Ser Ile Arg Asn
                   485                 490                 495
   gga acc tac aac tac ccc cag tac agc gaa gaa gcc aga ctg aaa aga    3335
   Gly Thr Tyr Asn Tyr Pro Gln Tyr Ser Glu Glu Ala Arg Leu Lys Arg
               500                 505                 510
   gaa gaa atc tcc gga gtg aaa ctg gaa tcc atc gga acc tac cag atc    3383
   Glu Glu Ile Ser Gly Val Lys Leu Glu Ser Ile Gly Thr Tyr Gln Ile
           515                 520                 525
   ctg agc atc tac agc aca gtg gcc tcc tcc ctg gcc ctg gcc atc atg    3431
   Leu Ser Ile Tyr Ser Thr Val Ala Ser Ser Leu Ala Leu Ala Ile Met
       530                 535                 540
   atg gcc gga ctg agc ctg tgg atg tgc tcc aac gga agc ctg cag tgc    3479
   Met Ala Gly Leu Ser Leu Trp Met Cys Ser Asn Gly Ser Leu Gln Cys
   545                 550                 555                 560
   aga atc tgc atc tgactcgagt ttttattgac tagttaatca cggccgctta        3531
   Arg Ile Cys Ile
   taaagatcta aaatgcataa tttctaaata atgaaaaaaa gtacatcatg agcaacgcgt 3591
   tagtatattt tacaatggag attaacgctc tataccgttc tatgtttatt gattcagatg 3651
   atgttttaga aaagaaagtt attgaatatg aaaactttaa tgaagatgaa gatgacgacg 3711
   atgattattg ttgtaaatct gttttagatg aagaagatga cgcgctaaag tatactatgg 3771
   ttacaaagta taagtctata ctactaatgg cgacttgtgc aagaaggtat agtatagtga 3831
   aaatgttgtt agattatgat tatgaaaaac caaataaatc agatccatat ctaaaggtat 3891
   ctcctttgca cataatttca tctattccta gtttagaata cctgcagcca agcttggcac 3951
   tggccgtcgt tttac 3966
<210> 19
   <211> 6551
   <212> DNA
   <213> Artificial Sequence
<220>
   <223> Description of Artificial Sequence: Synthetic construct
<400> 19
   gcgcccaata cgcaaaccgc ctctccccgc gcgttggccg attcattaat gcagctggca 60
   cgacaggttt cccgactgga aagcgggcag tgagcgcaac gcaattaatg tgagttagct 120
   cactcattag gcaccccagg ctttacactt tatgcttccg gctcgtatgt tgtgtggaat 180
   tgtgagcgga taacaatttc acacaggaaa cagctatgac catgattacg aattgcggcc 240
   gcaattctga atgttaaatg ttatactttg gatgaagcta taaatatgca ttggaaaaat 300
   aatccattta aagaaaggat tcaaatacta caaaacctaa gcgataatat gttaactaag 360
   cttattctta acgacgcttt aaatatacac aaataaacat aatttttgta taacctaaca 420
   aataactaaa acataaaaat aataaaagga aatgtaatat cgtaattatt ttactcagga 480
   atggggttaa atatttatat cacgtgtata tctatactgt tatcgtatac tctttacaat 540
   tactattacg aatatgcaag agataataag attacgtatt taagagaatc ttgtcatgat 600
   aattgggtac gacatagtga taaatgctat ttcgcatcgt tacataaagt cagttggaaa 660
   gatggatttg acagatgtaa cttaataggt gcaaaaatgt taaataacag cattctatcg 720
   gaagatagga taccagttat attatacaaa aatcactggt tggataaaac agattctgca 780
   atattcgtaa aagatgaaga ttactgcgaa tttgtaaact atgacaataa aaagccattt 840
   atctcaacga catcgtgtaa ttcttccatg ttttatgtat gtgtttcaga tattatgaga 900
   ttactataaa ctttttgtat acttatattc cgtaaactat attaatcatg aagaaaatga 960
   aaaagtatag aagctgttca cgagcggttg ttgaaaacaa caaaattata cattcaagat 1020
   ggcttacata tacgtctgtg aggctatcat ggataatgac aatgcatctc taaataggtt 1080
   tttggacaat ggattcgacc ctaacacgga atatggtact ctacaatctc ctcttgaaat 1140
   ggctgtaatg ttcaagaata ccgaggctat aaaaatcttg atgaggtatg gagctaaacc 1200
   tgtagttact gaatgcacaa cttcttgtct gcatgatgcg gtgttgagag acgactacaa 1260
   aatagtgaaa gatctgttga agaataacta tgtaaacaat gttctttaca gcggaggctt 1320
   tactcctttg tgtttggcag cttaccttaa caaagttaat ttggttaaac ttctattggc 1380
   tcattcggcg gatgtagata tttcaaacac ggatcggtta actcctctac atatagccgt 1440
   atcaaataaa aatttaacaa tggttaaact tctattgaac aaaggtgctg atactgactt 1500
   gctggataac atgggacgta ctcctttaat gatcgctgta caatctggaa atattgaaat 1560
   atgtagcaca ctacttaaaa aaaataaaat gtccagaact gggaaaaatt gatcttgcca 1620
   gctgtaattc atggtagaaa agaagtgctc aggctacttt tcaacaaagg agcagatgta 1680
   aactacatct ttgaaagaaa tggaaaatca tatactgttt tggaattgat taaagaaagt 1740
   tactctgaga cacaaaagag gtagctgaag tggtactctc aaaggtacgt gactaattag 1800
   ctataaaaag gatccgggtt aattaattag tcatcaggca gggcgagaac gagactatct 1860
   gctcgttaat taattagagc ttctttattc tatacttaaa aagtgaaaat aaatacaaag 1920
   gttcttgagg gttgtgttaa attgaaagcg agaaataatc ataaattatt tcattatcgc 1980
   gatatccgtt aagtttgtat cgtaatggag aaaatcgtgc tgctgctggc catcgtgagc 2040
   ctggtgaaaa gcgatcagat ctgcatcggc taccacgcca acaacagcac agagcaagtg 2100
   gacacaatca tggaaaagaa cgtgaccgtg acacacgccc aggacatcct ggaaaagaca 2160
   cacaacggga agctgtgcga tctggatgga gtgaagcctc tgatcctgag agattgcagc 2220
   gtggccggat ggctgctggg gaacccaatg tgcgacgaat tcatcaacgt gcccgaatgg 2280
   agctacatcg tggagaaggc caacccagcc aacgacctgt gctacccagg gaacctgaac 2340
   gactacgaag aactgaaaca cctgctgagc agaatcaacc actttgagaa aatccagatc 2400
   atccccaaaa gcagctggtc cgatcacgaa gccagcagcg gagtgagcag cgcctgccca 2460
   taccagggaa agtccagctt ttttagaaac gtggtgtggc tgatcaaaaa gaacagcgcc 2520
   tacccaacaa tcaagagaag ctacaacaac accaaccagg aagatctgct ggtgctgtgg 2580
   gggatccacc accctaacga tgccgccgag cagacaaggc tgtaccagaa cccaaccacc 2640
   tacatctccg tggggacaag cacactgaac cagagactgg tgccaaaaat cgccatcaga 2700
   tccaaagtga acgggcagag cggaagaatg gagttcttct ggacaatcct gaaacccaac 2760
   gatgccatca acttcgagag caacggaaac ttcatcgccc cagaatacgc ctacaaaatc 2820
   gtgaagaaag gggacagcgc catcatgaaa agcgaactgg aatacggcaa ctgcaacacc 2880
   aagtgccaga ccccaatggg ggccatcaac agcagcatgc cattccacaa catccaccct 2940
   ctgaccatcg gggaatgccc caaatacgtg aaaagcaaca gactggtgct ggccaccggg 3000
   ctgagaaaca gccctcagag agagaccaga ggactgtttg gagccatcgc cggctttatc 3060
   gagggaggat ggcagggaat ggtggatggc tggtacggat accaccacag caacgagcag 3120
   gggagcggat acgccgccga caaagaatcc acccagaagg ccatcgacgg cgtgaccaac 3180
   aaagtgaaca gcatcatcga caaaatgaac acccagtttg aggccgtggg aagggagttt 3240
   aacaacctgg aaaggagaat cgagaacctg aacaagaaga tggaggacgg attcctggat 3300
   gtgtggacct acaacgccga actgctggtg ctgatggaaa acgagagaac cctggacttt 3360
   cacgacagca acgtgaagaa cctgtacgac aaagtgaggc tgcagctgag ggataacgcc 3420
   aaggagctgg gcaacggctg cttcgagttc taccacaaat gcgataacga atgcatggaa 3480
   agcatcagaa acggaaccta caactacccc cagtacagcg aagaagccag actgaaaaga 3540
   gaagaaatct ccggagtgaa actggaatcc atcggaacct accagatcct gagcatctac 3600
   agcacagtgg cctcctccct ggccctggcc atcatgatgg ccggactgag cctgtggatg 3660
   tgctccaacg gaagcctgca gtgcagaatc tgcatctgac tcgagttttt attgactagt 3720
   taatcacggc cgcttataaa gatctaaaat gcataatttc taaataatga aaaaaagtac 3780
   atcatgagca acgcgttagt atattttaca atggagatta acgctctata ccgttctatg 3840
   tttattgatt cagatgatgt tttagaaaag aaagttattg aatatgaaaa ctttaatgaa 3900
   gatgaagatg acgacgatga ttattgttgt aaatctgttt tagatgaaga agatgacgcg 3960
   ctaaagtata ctatggttac aaagtataag tctatactac taatggcgac ttgtgcaaga 4020
   aggtatagta tagtgaaaat gttgttagat tatgattatg aaaaaccaaa taaatcagat 4080
   ccatatctaa aggtatctcc tttgcacata atttcatcta ttcctagttt agaatacctg 4140
   cagccaagct tggcactggc cgtcgtttta caacgtcgtg actgggaaaa ccctggcgtt 4200
   acccaactta atcgccttgc agcacatccc cctttcgcca gctggcgtaa tagcgaagag 4260
   gcccgcaccg atcgcccttc ccaacagttg cgcagcctga atggcgaatg gcgcctgatg 4320
   cggtattttc tccttacgca tctgtgcggt atttcacacc gcatatggtg cactctcagt 4380
   acaatctgct ctgatgccgc atagttaagc cagccccgac acccgccaac acccgctgac 4440
   gcgccctgac gggcttgtct gctcccggca tccgcttaca gacaagctgt gaccgtctcc 4500
   gggagctgca tgtgtcagag gttttcaccg tcatcaccga aacgcgcgag acgaaagggc 4560
   ctcgtgatac gcctattttt ataggttaat gtcatgataa taatggtttc ttagacgtca 4620
   ggtggcactt ttcggggaaa tgtgcgcgga acccctattt gtttattttt ctaaatacat 4680
   tcaaatatgt atccgctcat gagacaataa ccctgataaa tgcttcaata atattgaaaa 4740
   aggaagagta tgagtattca acatttccgt gtcgccctta ttcccttttt tgcggcattt 4800
   tgccttcctg tttttgctca cccagaaacg ctggtgaaag taaaagatgc tgaagatcag 4860
   ttgggtgcac gagtgggtta catcgaactg gatctcaaca gcggtaagat ccttgagagt 4920
   tttcgccccg aagaacgttt tccaatgatg agcactttta aagttctgct atgtggcgcg 4980
   gtattatccc gtattgacgc cgggcaagag caactcggtc gccgcataca ctattctcag 5040
   aatgacttgg ttgagtactc accagtcaca gaaaagcatc ttacggatgg catgacagta 5100
   agagaattat gcagtgctgc cataaccatg agtgataaca ctgcggccaa cttacttctg 5160
   acaacgatcg gaggaccgaa ggagctaacc gcttttttgc acaacatggg ggatcatgta 5220
   actcgccttg atcgttggga accggagctg aatgaagcca taccaaacga cgagcgtgac 5280
   accacgatgc ctgtagcaat ggcaacaacg ttgcgcaaac tattaactgg cgaactactt 5340
   actctagctt cccggcaaca attaatagac tggatggagg cggataaagt tgcaggacca 5400
   cttctgcgct cggcccttcc ggctggctgg tttattgctg ataaatctgg agccggtgag 5460
   cgtgggtctc gcggtatcat tgcagcactg gggccagatg gtaagccctc ccgtatcgta 5520
   gttatctaca cgacggggag tcaggcaact atggatgaac gaaatagaca gatcgctgag 5580
   ataggtgcct cactgattaa gcattggtaa ctgtcagacc aagtttactc atatatactt 5640
   tagattgatt taaaacttca tttttaattt aaaaggatct aggtgaagat cctttttgat 5700
   aatctcatga ccaaaatccc ttaacgtgag ttttcgttcc actgagcgtc agaccccgta 5760
   gaaaagatca aaggatcttc ttgagatcct ttttttctgc gcgtaatctg ctgcttgcaa 5820
   acaaaaaaac caccgctacc agcggtggtt tgtttgccgg atcaagagct accaactctt 5880
   tttccgaagg taactggctt cagcagagcg cagataccaa atactgtcct tctagtgtag 5940
   ccgtagttag gccaccactt caagaactct gtagcaccgc ctacatacct cgctctgcta 6000
   atcctgttac cagtggctgc tgccagtggc gataagtcgt gtcttaccgg gttggactca 6060
   agacgatagt taccggataa ggcgcagcgg tcgggctgaa cggggggttc gtgcacacag 6120
   cccagcttgg agcgaacgac ctacaccgaa ctgagatacc tacagcgtga gctatgagaa 6180
   agcgccacgc ttcccgaagg gagaaaggcg gacaggtatc cggtaagcgg cagggtcgga 6240
   acaggagagc gcacgaggga gcttccaggg ggaaacgcct ggtatcttta tagtcctgtc 6300
   gggtttcgcc acctctgact tgagcgtcga tttttgtgat gctcgtcagg ggggcggagc 6360
   ctatggaaaa acgccagcaa cgcggccttt ttacggttcc tggccttttg ctggcctttt 6420
   gctcacatgt tctttcctgc gttatcccct gattctgtgg ataaccgtat taccgccttt 6480
   gagtgagctg ataccgctcg ccgcagccga acgaccgagc gcagcgagtc agtgagcgag 6540
   gaagcggaag a 6551
<210> 20
   <211> 25
   <212> DNA
   <213> Artificial Sequence
<220>
   <223> Description of Artificial Sequence: Synthetic primer
<400> 20
   gaatctgtta gttagttact tggat 25
<210> 21
   <211> 25
   <212> DNA
   <213> Artificial Sequence
<220>
   <223> Description of Artificial Sequence: Synthetic primer
<400> 21
   tgattatagc tattatcaca gactc 25
<210> 22
   <211> 3890
   <212> DNA
   <213> Artificial Sequence
<220>
   <223> Description of Artificial Sequence: Synthetic construct
<400> 22
   tgaatgttaa atgttatact ttggatgaag ctataaatat gcattggaaa aataatccat 60
   ttaaagaaag gattcaaata ctacaaaacc taagcgataa tatgttaact aagcttattc 120
   ttaacgacgc tttaaatata cacaaataaa cataattttt gtataaccta acaaataact 180
   aaaacataaa aataataaaa ggaaatgtaa tatcgtaatt attttactca ggaatggggt 240
   taaatattta tatcacgtgt atatctatac tgttatcgta tactctttac aattactatt 300
   acgaatatgc aagagataat aagattacgt atttaagaga atcttgtcat gataattggg 360
   tacgacatag tgataaatgc tatttcgcat cgttacataa agtcagttgg aaagatggat 420
   ttgacagatg taacttaata ggtgcaaaaa tgttaaataa cagcattcta tcggaagata 480
   ggataccagt tatattatac aaaaatcact ggttggataa aacagattct gcaatattcg 540
   taaaagatga agattactgc gaatttgtaa actatgacaa taaaaagcca tttatctcaa 600
   cgacatcgtg taattcttcc atgttttatg tatgtgtttc agatattatg agattactat 660
   aaactttttg tatacttata ttccgtaaac tatattaatc atgaagaaaa tgaaaaagta 720
   tagaagctgt tcacgagcgg ttgttgaaaa caacaaaatt atacattcaa gatggcttac 780
   atatacgtct gtgaggctat catggataat gacaatgcat ctctaaatag gtttttggac 840
   aatggattcg accctaacac ggaatatggt actctacaat ctcctcttga aatggctgta 900
   atgttcaaga ataccgaggc tataaaaatc ttgatgaggt atggagctaa acctgtagtt 960
   actgaatgca caacttcttg tctgcatgat gcggtgttga gagacgacta caaaatagtg 1020
   aaagatctgt tgaagaataa ctatgtaaac aatgttcttt acagcggagg ctttactcct 1080
   ttgtgtttgg cagcttacct taacaaagtt aatttggtta aacttctatt ggctcattcg 1140
   gcggatgtag atatttcaaa cacggatcgg ttaactcctc tacatatagc cgtatcaaat 1200
   aaaaatttaa caatggttaa acttctattg aacaaaggtg ctgatactga cttgctggat 1260
   aacatgggac gtactccttt aatgatcgct gtacaatctg gaaatattga aatatgtagc 1320
   acactactta aaaaaaataa aatgtccaga actgggaaaa attgatcttg ccagctgtaa 1380
   ttcatggtag aaaagaagtg ctcaggctac ttttcaacaa aggagcagat gtaaactaca 1440
   tctttgaaag aaatggaaaa tcatatactg ttttggaatt gattaaagaa agttactctg 1500
   agacacaaaa gaggtagctg aagtggtact ctcaaaggta cgtgactaat tagctataaa 1560
   aaggatccgg gttaattaat tagtcatcag gcagggcgag aacgagacta tctgctcgtt 1620
   aattaattag agcttcttta ttctatactt aaaaagtgaa aataaataca aaggttcttg 1680
   agggttgtgt taaattgaaa gcgagaaata atcataaatt atttcattat cgcgatatcc 1740
   gttaagtttg tatcgtaatg gagaaaatcg tgctgctgct ggccatcgtg agcctggtga 1800
   aaagcgatca gatctgcatc ggctaccacg ccaacaacag cacagagcaa gtggacacaa 1860
   tcatggaaaa gaacgtgacc gtgacacacg cccaggacat cctggaaaag acacacaacg 1920
   ggaagctgtg cgatctggat ggagtgaagc ctctgatcct gagagattgc agcgtggccg 1980
   gatggctgct ggggaaccca atgtgcgacg aattcatcaa cgtgcccgaa tggagctaca 2040
   tcgtggagaa ggccaaccca gccaacgacc tgtgctaccc agggaacctg aacgactacg 2100
   aagaactgaa acacctgctg agcagaatca accactttga gaaaatccag atcatcccca 2160
   aaagcagctg gtccgatcac gaagccagca gcggagtgag cagcgcctgc ccataccagg 2220
   gaaagtccag cttttttaga aacgtggtgt ggctgatcaa aaagaacagc gcctacccaa 2280
   caatcaagag aagctacaac aacaccaacc aggaagatct gctggtgctg tgggggatcc 2340
   accaccctaa cgatgccgcc gagcagacaa ggctgtacca gaacccaacc acctacatct 2400
   ccgtggggac aagcacactg aaccagagac tggtgccaaa aatcgccatc agatccaaag 2460
   tgaacgggca gagcggaaga atggagttct tctggacaat cctgaaaccc aacgatgcca 2520
   tcaacttcga gagcaacgga aacttcatcg ccccagaata cgcctacaaa atcgtgaaga 2580
   aaggggacag cgccatcatg aaaagcgaac tggaatacgg caactgcaac accaagtgcc 2640
   agaccccaat gggggccatc aacagcagca tgccattcca caacatccac cctctgacca 2700
   tcggggaatg ccccaaatac gtgaaaagca acagactggt gctggccacc gggctgagaa 2760
   acagccctca gagagagacc agaggactgt ttggagccat cgccggcttt atcgagggag 2820
   gatggcaggg aatggtggat ggctggtacg gataccacca cagcaacgag caggggagcg 2880
   gatacgccgc cgacaaagaa tccacccaga aggccatcga cggcgtgacc aacaaagtga 2940
   acagcatcat cgacaaaatg aacacccagt ttgaggccgt gggaagggag tttaacaacc 3000
   tggaaaggag aatcgagaac ctgaacaaga agatggagga cggattcctg gatgtgtgga 3060
   cctacaacgc cgaactgctg gtgctgatgg aaaacgagag aaccctggac tttcacgaca 3120
   gcaacgtgaa gaacctgtac gacaaagtga ggctgcagct gagggataac gccaaggagc 3180
   tgggcaacgg ctgcttcgag ttctaccaca aatgcgataa cgaatgcatg gaaagcatca 3240
   gaaacggaac ctacaactac ccccagtaca gcgaagaagc cagactgaaa agagaagaaa 3300
   tctccggagt gaaactggaa tccatcggaa cctaccagat cctgagcatc tacagcacag 3360
   tggcctcctc cctggccctg gccatcatga tggccggact gagcctgtgg atgtgctcca 3420
   acggaagcct gcagtgcaga atctgcatct gactcgagtt tttattgact agttaatcac 3480
   ggccgcttat aaagatctaa aatgcataat ttctaaataa tgaaaaaaag tacatcatga 3540
   gcaacgcgtt agtatatttt acaatggaga ttaacgctct ataccgttct atgtttattg 3600
   attcagatga tgttttagaa aagaaagtta ttgaatatga aaactttaat gaagatgaag 3660
   atgacgacga tgattattgt tgtaaatctg ttttagatga agaagatgac gcgctaaagt 3720
   atactatggt tacaaagtat aagtctatac tactaatggc gacttgtgca agaaggtata 3780
   gtatagtgaa aatgttgtta gattatgatt atgaaaaacc aaataaatca gatccatatc 3840
   taaaggtatc tcctttgcac ataatttcat ctattcctag tttagaatac            3890
<210> 23
   <211> 564
   <212> PRT
   <213> Artificial Sequence
<220>
   <223> Description of Artificial Sequence: Synthetic construct
<400> 23
   Met Glu Lys Ile Val Leu Leu Leu Ala Ile Val Ser Leu Val Lys Ser
    1               5                  10                  15
   Asp Gln Ile Cys Ile Gly Tyr His Ala Asn Asn Ser Thr Glu Gln Val
               20                  25                  30
   Asp Thr Ile Met Glu Lys Asn Val Thr Val Thr His Ala Gln Asp Ile
           35                  40                  45
   Leu Glu Lys Thr His Asn Gly Lys Leu Cys Asp Leu Asp Gly Val Lys
       50                  55                  60
   Pro Leu Ile Leu Arg Asp Cys Ser Val Ala Gly Trp Leu Leu Gly Asn
    65                 70                  75                  80
   Pro Met Cys Asp Glu Phe Ile Asn Val Pro Glu Trp Ser Tyr Ile Val
                   85                  90                  95
   Glu Lys Ala Asn Pro Ala Asn Asp Leu Cys Tyr Pro Gly Asn Leu Asn
               100                 105                 110
   Asp Tyr Glu Glu Leu Lys His Leu Leu Ser Arg Ile Asn His Phe Glu
           115                 120                 125
   Lys Ile Gln Ile Ile Pro Lys Ser Ser Trp Ser Asp His Glu Ala Ser
       130                 135                 140
   Ser Gly Val Ser Ser Ala Cys Pro Tyr Gln Gly Lys Ser Ser Phe Phe
   145                 150                 155                 160
   Arg Asn Val Val Trp Leu Ile Lys Lys Asn Ser Ala Tyr Pro Thr Ile
                   165                 170                 175
   Lys Arg Ser Tyr Asn Asn Thr Asn Gln Glu Asp Leu Leu Val Leu Trp
               180                 185                 190
   Gly Ile His His Pro Asn Asp Ala Ala Glu Gln Thr Arg Leu Tyr Gln
           195                 200                 205
   Asn Pro Thr Thr Tyr Ile Ser Val Gly Thr Ser Thr Leu Asn Gln Arg
       210                 215                 220
   Leu Val Pro Lys Ile Ala Ile Arg Ser Lys Val Asn Gly Gln Ser Gly
   225                 230                 235                 240
   Arg Met Glu Phe Phe Trp Thr Ile Leu Lys Pro Asn Asp Ala Ile Asn
                   245                 250                 255
   Phe Glu Ser Asn Gly Asn Phe Ile Ala Pro Glu Tyr Ala Tyr Lys Ile
               260                 265                 270
   Val Lys Lys Gly Asp Ser Ala Ile Met Lys Ser Glu Leu Glu Tyr Gly
           275                 280                 285
   Asn Cys Asn Thr Lys Cys Gln Thr Pro Met Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Arg Glu Thr Arg Gly Leu Phe Gly Ala Ile Ala Gly Phe Ile
               340                 345                 350
   Glu Gly Gly Trp Gln Gly Met Val Asp Gly Trp Tyr Gly Tyr His His
           355                 360                 365
   Ser Asn Glu Gln Gly Ser Gly Tyr Ala Ala Asp Lys Glu Ser Thr Gln
       370                 375                 380
   Lys Ala Ile Asp Gly Val Thr Asn Lys Val Asn Ser Ile Ile Asp Lys
   385                 390                 395                 400
   Met Asn Thr Gln Phe Glu Ala Val Gly Arg Glu Phe Asn Asn Leu Glu
                   405                 410                 415
   Arg Arg Ile Glu Asn Leu Asn Lys Lys Met Glu Asp Gly Phe Leu Asp
               420                 425                 430
   Val Trp Thr Tyr Asn Ala Glu Leu Leu Val Leu Met Glu Asn Glu Arg
           435                 440                 445
   Thr Leu Asp Phe His Asp Ser Asn Val Lys Asn Leu Tyr Asp Lys Val
       450                 455                 460
   Arg Leu Gln Leu Arg Asp Asn Ala Lys Glu Leu Gly Asn Gly Cys Phe
   465                 470                 475                 480
   Glu Phe Tyr His Lys Cys Asp Asn Glu Cys Met Glu Ser Ile Arg Asn
                   485                 490                 495
   Gly Thr Tyr Asn Tyr Pro Gln Tyr Ser Glu Glu Ala Arg Leu Lys Arg
               500                 505                 510
   Glu Glu Ile Ser Gly Val Lys Leu Glu Ser Ile Gly Thr Tyr Gln Ile
           515                 520                 525
   Leu Ser Ile Tyr Ser Thr Val Ala Ser Ser Leu Ala Leu Ala Ile Met
       530                 535                 540
   Met Ala Gly Leu Ser Leu Trp Met Cys Ser Asn Gly Ser Leu Gln Cys
   545                 550                 555                 560
   Arg Ile Cys Ile
<210> 24
   <211> 1692
   <212> DNA
   <213> Artificial Sequence
<220>
   <223> Description of Artificial Sequence: Synthetic construct
<400> 24
   atggagaaaa tcgtgctgct gctggccatc gtgagcctgg tgaaaagcga tcagatctgc 60
   atcggctacc acgccaacaa cagcacagag caagtggaca caatcatgga aaagaacgtg 120
   accgtgacac acgcccagga catcctggaa aagacacaca acgggaagct gtgcgatctg 180
   gatggagtga agcctctgat cctgagagat tgcagcgtgg ccggatggct gctggggaac 240
   ccaatgtgcg acgaattcat caacgtgccc gaatggagct acatcgtgga gaaggccaac 300
   ccagccaacg acctgtgcta cccagggaac ctgaacgact acgaagaact gaaacacctg 360
   ctgagcagaa tcaaccactt tgagaaaatc cagatcatcc ccaaaagcag ctggtccgat 420
   cacgaagcca gcagcggagt gagcagcgcc tgcccatacc agggaaagtc cagctttttt 480
   agaaacgtgg tgtggctgat caaaaagaac agcgcctacc caacaatcaa gagaagctac 540
   aacaacacca accaggaaga tctgctggtg ctgtggggga tccaccaccc taacgatgcc 600
   gccgagcaga caaggctgta ccagaaccca accacctaca tctccgtggg gacaagcaca 660
   ctgaaccaga gactggtgcc aaaaatcgcc atcagatcca aagtgaacgg gcagagcgga 720
   agaatggagt tcttctggac aatcctgaaa cccaacgatg ccatcaactt cgagagcaac 780
   ggaaacttca tcgccccaga atacgcctac aaaatcgtga agaaagggga cagcgccatc 840
   atgaaaagcg aactggaata cggcaactgc aacaccaagt gccagacccc aatgggggcc 900
   atcaacagca gcatgccatt ccacaacatc caccctctga ccatcgggga atgccccaaa 960
   tacgtgaaaa gcaacagact ggtgctggcc accgggctga gaaacagccc tcagagagag 1020
   accagaggac tgtttggagc catcgccggc tttatcgagg gaggatggca gggaatggtg 1080
   gatggctggt acggatacca ccacagcaac gagcagggga gcggatacgc cgccgacaaa 1140
   gaatccaccc agaaggccat cgacggcgtg accaacaaag tgaacagcat catcgacaaa 1200
   atgaacaccc agtttgaggc cgtgggaagg gagtttaaca acctggaaag gagaatcgag 1260
   aacctgaaca agaagatgga ggacggattc ctggatgtgt ggacctacaa cgccgaactg 1320
   ctggtgctga tggaaaacga gagaaccctg gactttcacg acagcaacgt gaagaacctg 1380
   tacgacaaag tgaggctgca gctgagggat aacgccaagg agctgggcaa cggctgcttc 1440
   gagttctacc acaaatgcga taacgaatgc atggaaagca tcagaaacgg aacctacaac 1500
   tacccccagt acagcgaaga agccagactg aaaagagaag aaatctccgg agtgaaactg 1560
   gaatccatcg gaacctacca gatcctgagc atctacagca cagtggcctc ctccctggcc 1620
   ctggccatca tgatggccgg actgagcctg tggatgtgct ccaacggaag cctgcagtgc 1680
   agaatctgca tc                                                     1692
<210> 25
   <211> 568
   <212> PRT
   <213> Influenza A virus
<400> 25
   Met Glu Lys Ile Val Leu Leu Leu Ala Ile Val Ser Leu Val Lys Ser
   1 5 10 15
   Asp Gln Ile Cys Ile Gly Tyr His Ala Asn Asn Ser Thr Glu Gln Val
   20 25 30
   Asp Thr Ile Met Glu Lys Asn Val Thr Val Thr His Ala Gln Asp Ile
   35 40 45
   Leu Glu Lys Thr His Asn Gly Lys Leu Cys Asp Leu Asp Gly Val Lys
   50 55 60
   Pro Leu Ile Leu Arg Asp Cys Ser Val Ala Gly Trp Leu Leu Gly Asn
   65 70 75 80
   Pro Met Cys Asp Glu Phe Ile Asn Val Pro Glu Trp Ser Tyr Ile Val
   85 90 95
   Glu Lys Ala Asn Pro Ala Asn Asp Leu Cys Tyr Pro Gly Asn Leu Asn
   100 105 110
   Asp Tyr Glu Glu Leu Lys His Leu Leu Ser Arg Ile Asn His Phe Glu
   115 120 125
   Lys Ile Gln Ile Ile Pro Lys Ser Ser Trp Ser Asp His Glu Ala Ser
   130 135 140
   Ser Gly Val Ser Ser Ala Cys Pro Tyr Gln Gly Lys Ser Ser Phe Phe
   145 150 155 160
   Arg Asn Val Val Trp Leu Ile Lys Lys Asn Ser Ala Tyr Pro Thr Ile
   165 170 175
   Lys Arg Ser Tyr Asn Asn Thr Asn Gln Glu Asp Leu Leu Val Leu Trp
   180 185 190
   Gly Ile His His Pro Asn Asp Ala Ala Glu Gln Thr Arg Leu Tyr Gln
   195 200 205
   Asn Pro Thr Thr Tyr Ile Ser Val Gly Thr Ser Thr Leu Asn Gln Arg
   210 215 220
   Leu Val Pro Lys Ile Ala Ile Arg Ser Lys Val Asn Gly Gln Ser Gly
   225 230 235 240
   Arg Met Glu Phe Phe Trp Thr Ile Leu Lys Pro Asn Asp Ala Ile Asn
   245 250 255
   Phe Glu Ser Asn Gly Asn Phe Ile Ala Pro Glu Tyr Ala Tyr Lys Ile
   260 265 270
   Val Lys Lys Gly Asp Ser Ala Ile Met Lys Ser Glu Leu Glu Tyr Gly
                   275                 280                 285
   Asn Cys Asn Thr Lys Cys Gln Thr Pro Met Gly Ala Ile Asn Ser Ser
       290                 295                 300
   Met Pro Phe His Asn Ile His Pro Leu Thr Ile Gly Glu Cys Pro Lys
   305                 310                 315                 320
   Tyr Val Lys Ser Asn Arg Leu Val Leu Ala Thr Gly Leu Arg Asn Ser
                   325                 330                 335
   Pro Gln Arg Glu Arg Arg Arg Lys Lys Arg Gly Leu Phe Gly Ala Ile
               340                 345                 350
   Ala Gly Phe Ile Glu Gly Gly Trp Gln Gly Met Val Asp Gly Trp Tyr
           355                 360                 365
   Gly Tyr His His Ser Asn Glu Gln Gly Ser Gly Tyr Ala Ala Asp Lys
       370                 375                 380
   Glu Ser Thr Gln Lys Ala Ile Asp Gly Val Thr Asn Lys Val Asn Ser
   385                 390                 395                 400
   Ile Ile Asp Lys Met Asn Thr Gln Phe Glu Ala Val Gly Arg Glu Phe
                   405                 410                 415
   Asn Asn Leu Glu Arg Arg Ile Glu Asn Leu Asn Lys Lys Met Glu Asp
               420                 425                 430
   Gly Phe Leu Asp Val Trp Thr Tyr Asn Ala Glu Leu Leu Val Leu Met
           435                 440                 445
   Glu Asn Glu Arg Thr Leu Asp Phe His Asp Ser Asn Val Lys Asn Leu
       450                 455                 460
   Tyr Asp Lys Val Arg Leu Gln Leu Arg Asp Asn Ala Lys Glu Leu Gly
   465                 470                 475                 480
   Asn Gly Cys Phe Glu Phe Tyr His Lys Cys Asp Asn Glu Cys Met Glu
                   485                 490                 495
   Ser Ile Arg Asn Gly Thr Tyr Asn Tyr Pro Gln Tyr Ser Glu Glu Ala
               500                 505                 510
   Arg Leu Lys Arg Glu Glu Ile Ser Gly Val Lys Leu Glu Ser Ile Gly
           515                 520                 525
   Thr Tyr Gln Ile Leu Ser Ile Tyr Ser Thr Val Ala Ser Ser Leu Ala
       530                 535                 540
   Leu Ala Ile Met Met Ala Gly Leu Ser Leu Trp Met Cys Ser Asn Gly
   545                 550                 555                 560
   Ser Leu Gln Cys Arg Ile Cys Ile
                   565

## Claims

1. An immunological composition comprising an avipox expression vector, wherein said expression vector comprises a polynucleotide encoding an influenza antigen;wherein the avipox expression vector is an ALVAC vector and wherein the influenza antigen is hemagglutinin H5.

2. The immunogenic composition according to claim 1, further comprising one or more of a pharmaceutically or veterinarily acceptable carrier, excipient, vehicle, or adjuvant.

3. The immunological composition according to claim 1 or 2 for use in protecting Felidae against avian influenza.

4. The immunological composition according to claim 3, wherein either prior or subsequent to administration of said immunological composition, a second immunological composition comprising either an avipox expression vector, wherein said vector comprises a polynucleotide encoding an influenza antigen, or an inactivated influenza immunological composition is administered.

5. The immunological composition of claim 3, wherein the Felidae is a cat.

6. The immunological composition according to any one of claims 3 to 5 wherein said composition is formulated for intramuscular administration, subcutaneous administration, oral administration or administration by needleless apparatus.

7. Use of the immunological composition according to claim 1 or 2 for the manufacture of a medicament for protecting Felidae against avian influenza.

8. The use according to claim 7, wherein either prior or subsequent to administration of said immunological composition, a second immunological composition comprising either an avipox expression vector, wherein said vector comprises a polynucleotide encoding an influenza antigen, or an inactivated influenza immunological composition is administered.

9. The use according to claim 7 or 8, wherein the Felidae is a cat.

10. The use according to any one of claims 7 to 9 wherein said composition is formulated for intramuscular administration, subcutaneous administration, oral administration or administration by needleless apparatus.

11. A kit comprising a recombinant influenza poxvirus immunological composition according to claim 1 or 2 and instructions for the use as defined in any one of claims 3 to 10.

## Patentansprüche

1. Immunologische Zusammensetzung, umfassend einen Avipox-Expressionsvektor, wobei der Expressionsvektor ein Polynucleotid umfasst, das ein Influenzaantigen codiert; wobei der Avipox-Expressionsvektor ein ALVAC-Vektor ist und wobei das Influenzaantigen Hemagglutinin H5 ist.

2. Immunogene Zusammensetzung gemäß Anspruch 1, ferner umfassend einen oder mehrere pharmazeutisch oder veterinärmedizinisch verträgliche Träger, Exzipient/en, Vehikel oder Adjuvantien.

3. Immunologische Zusammensetzung gemäß Anspruch 1 oder 2 zur Verwendung zum Schutz der Felidae gegen Vogelgrippe.

4. Immunologische Zusammensetzung gemäß Anspruch 3, wobei entweder vor oder nach Verabreichung der immunologischen Zusammensetzung eine zweite immunologische Zusammensetzung verabreicht wird, umfassend entweder einen Avipox-Expressionsvektor, wobei der Vektor ein Polynucleotid umfasst, das ein Influenzaantigen codiert, oder eine inaktivierte Influenza-immunologische Zusammensetzung.

5. Immunologische Zusammensetzung gemäß Anspruch 3, wobei die Felidae eine Katze ist.

6. Immunologische Zusammensetzung gemäß einem der Ansprüche 3 bis 5, wobei die Zusammensetzung formuliert ist für intramuskuläre Verabreichung, subkutane Verabreichung, orale Verabreichung oder Verabreichung durch einen nadellosen Apparat.

7. Verwendung der immunologischen Zusammensetzung gemäß Anspruch 1 oder 2 für die Herstellung eines Medikaments zum Schutz der Felidae gegen Vogelgrippe.

8. Verwendung gemäß Anspruch 7, wobei entweder vor oder nach Verabreichung der immunologischen Zusammensetzung eine zweite immunologische Zusammensetzung verabreicht wird, umfassend entweder einen Avipox-Expressionsvektor, wobei der Vektor ein Polynucleotid umfasst, das ein Influenzaantigen codiert, oder eine inaktivierte Influenza-immunologische Zusammensetzung.

9. Verwendung gemäß Anspruch 7 oder 8, wobei die Felidae eine Katze ist.

10. Verwendung gemäß einem der Ansprüche 7 bis 9, wobei die Zusammensetzung formuliert ist für intramuskuläre Verabreichung, subkutane Verabreichung, orale Verabreichung oder Verabreichung durch einen nadellosen Apparat.

11. Kit, umfassend eine rekombinante Influenza-Poxvirus-immunologische Zusammensetzung gemäß Anspruch 1 oder 2 und Instruktionen zur Verwendung wie in einem der Ansprüche 3 bis 10 definiert.

## Revendications

1. Composition immunologique comprenant un vecteur d'expression d'avipox, dans laquelle ledit vecteur d'expression comprend un polynucléotide codant pour un antigène grippal ; dans laquelle le vecteur d'expression d'avipox est un vecteur ALVAC et dans laquelle l'antigène grippal est l'hémagglutinine H5.

2. Composition immunogène selon la revendication 1, comprenant en outre un ou plusieurs d'un support, d'un excipient, d'un véhicule ou d'un adjuvant acceptable d'un point de vue pharmaceutique ou vétérinaire.

3. Composition immunologique selon la revendication 1 ou la revendication 2 pour utilisation dans la protection des félidés contre la grippe aviaire.

4. Composition immunologique selon la revendication 3, dans laquelle soit avant soit après l'administration de ladite composition immunologique, une seconde composition immunologique comprenant soit un vecteur d'expression d'avipox, dans laquelle ledit vecteur comprend un polynucléotide codant pour un antigène grippal, soit une composition immunologique à base de virus grippal inactivé, est administrée.

5. Composition immunologique selon la revendication 3, dans laquelle le félidé est un chat.

6. Composition immunologique selon l'une quelconque des revendications 3 à 5, dans laquelle ladite composition est formulée pour une administration intramusculaire, une administration sous-cutanée, une administration orale ou une administration par un appareil sans aiguille.

7. Utilisation de la composition immunologique selon la revendication 1 ou la revendication 2 pour la fabrication d'un médicament destiné à la protection des félidés contre la grippe aviaire.

8. Utilisation selon la revendication 7, dans laquelle soit avant soit après l'administration de ladite composition immunologique, une seconde composition immunologique comprenant soit un vecteur d'expression d'avipox, dans laquelle ledit vecteur comprend un polynucléotide codant pour un antigène grippal, soit une composition immunologique à base de virus grippal inactivé, est administrée.

9. Utilisation selon la revendication 7 ou la revendication 8, dans laquelle le félidé est un chat.

10. Utilisation selon l'une quelconque des revendications 7 à 9, dans laquelle ladite composition est formulée pour une administration intramusculaire, une administration sous-cutanée, une administration orale ou une administration par un appareil sans aiguille.

11. Kit comprenant une composition immunologique antigrippale à base de poxvirus recombinant selon la revendication 1 ou la revendication 2 et des instructions pour l'utilisation telle que définie dans l'une quelconque des revendications 3 à 10.
